# EUROPEAN PATENT APPLICATION

(11) **EP 3 115 784 A1**
(43) Date of publication of application: **11.01.2017**
(21) Application number: 15176356.2
(22) Date of filing: 10.07.2015
(51) Int. Cl.: G01N 33/68

(54) **POLYPEPTIDE MARKER FOR ANALYSIS, DIAGNOSIS AND THERAPY OF EYE-RELATED DISEASES**

(71) Applicant: Koss, Michael Janusz, 69118 Heidelberg (DE)
(72) Inventor: Koss, Michael Janusz, 69118 Heidelberg (DE)
(74) Representative: Prüfer & Partner mbB Patentanwälte · Rechtsanwälte

(57) **Abstract**

The present invention belongs to the field of eye-related diseases and its analysis and/or diagnosis. The present invention thus provides a method for analyzing and/or diagnosing such diseases comprising the step of determining the amount of specific marker polypeptides.

The present invention further refers to the use of said polypeptides for analyzing, diagnosing, and/or treating eye-related diseases, to the use of a kit for this purpose, as well as to a DNA chip for this purpose comprising the polypeptide markers. Finally, the present invention refers to an in vitro method for identifying a test compound that is capable of increasing or decreasing the amount of the specific marker polypeptides.

SEQUENCE LISTING

<110>  Koss, Michael Janusz

<120>  Polypeptide Marker for Analysis, Diagnosis and Therapy of
       Eye-related Diseases

<130>  ZZ 1036-P31642EP00

<160>  7

<170>  PatentIn version 3.5

<210>  1
<211>  214
<212>  PRT
<213>  Homo sapiens

<400>  1

Met Arg Pro Lys Thr Gly Gln Val Gly Cys Glu Thr Pro Glu Glu Leu
1               5                   10                  15

Gly Pro Gly Pro Arg Gln Arg Trp Pro Leu Leu Leu Leu Gly Leu Ala
            20                  25                  30

Met Val Ala His Gly Leu Leu Arg Pro Met Val Ala Pro Gln Ser Gly
        35                  40                  45

Asp Pro Asp Pro Gly Ala Ser Val Gly Ser Ser Arg Ser Ser Leu Arg
    50                  55                  60

Ser Leu Trp Gly Arg Leu Leu Leu Gln Pro Ser Pro Gln Arg Ala Asp
65                  70                  75                  80

Pro Arg Cys Trp Pro Arg Gly Phe Trp Ser Glu Pro Gln Ser Leu Cys
                85                  90                  95

Tyr Val Phe Gly Thr Gly Thr Lys Val Thr Val Leu Gly Gln Pro Lys
            100                 105                 110

Ala Asn Pro Thr Val Thr Leu Phe Pro Pro Ser Ser Glu Glu Leu Gln
        115                 120                 125

Ala Asn Lys Ala Thr Leu Val Cys Leu Ile Ser Asp Phe Tyr Pro Gly
    130                 135                 140

Ala Val Thr Val Ala Trp Lys Ala Asp Gly Ser Pro Val Lys Ala Gly
145                 150                 155                 160

Val Glu Thr Thr Lys Pro Ser Lys Gln Ser Asn Asn Lys Tyr Ala Ala
                165                 170                 175

Ser Ser Tyr Leu Ser Leu Thr Pro Glu Gln Trp Lys Ser His Arg Ser
        180                 185                 190

Tyr Ser Cys Gln Val Thr His Glu Gly Ser Thr Val Glu Lys Thr Val
    195                 200                 205

Ala Pro Thr Glu Cys Ser
    210

<210>  2
<211>  332
<212>  PRT
<213>  Homo sapiens

<400>  2

Met Arg Leu Leu Ala Phe Leu Ser Leu Leu Ala Leu Val Leu Gln Glu
1               5                   10                  15

Thr Gly Thr Ala Ser Leu Pro Arg Lys Glu Arg Lys Arg Arg Glu Glu
            20                  25                  30

Gln Met Pro Arg Glu Gly Asp Ser Phe Glu Val Leu Pro Leu Arg Asn
        35                  40                  45

Asp Val Leu Asn Pro Asp Asn Tyr Gly Glu Val Ile Asp Leu Ser Asn
    50                  55                  60

Tyr Glu Glu Leu Thr Asp Tyr Gly Asp Gln Leu Pro Glu Val Lys Val
65                  70                  75                  80

Thr Ser Leu Ala Pro Ala Thr Ser Ile Ser Pro Ala Lys Ser Thr Thr
                85                  90                  95

Ala Pro Gly Thr Pro Ser Ser Asn Pro Thr Met Thr Arg Pro Thr Thr
            100                 105                 110

Ala Gly Leu Leu Leu Ser Ser Gln Pro Asn His Gly Leu Pro Thr Cys
        115                 120                 125

Leu Val Cys Val Cys Leu Gly Ser Ser Val Tyr Cys Asp Asp Ile Asp
    130                 135                 140

Leu Glu Asp Ile Pro Pro Leu Pro Arg Arg Thr Ala Tyr Leu Tyr Ala
145                 150                 155                 160

Arg Phe Asn Arg Ile Ser Arg Ile Arg Ala Glu Asp Phe Lys Gly Leu
                165                 170                 175

Thr Lys Leu Lys Arg Ile Asp Leu Ser Asn Asn Leu Ile Ser Ser Ile
        180                 185                 190

Asp Asn Asp Ala Phe Arg Leu Leu His Ala Leu Gln Asp Leu Ile Leu
        195                 200                 205

Pro Glu Asn Gln Leu Glu Ala Leu Pro Val Leu Pro Ser Gly Ile Glu
    210                 215                 220

Phe Leu Asp Val Arg Leu Asn Arg Leu Gln Ser Ser Gly Ile Gln Pro
225                 230                 235                 240

Ala Ala Phe Arg Ala Met Glu Lys Leu Gln Phe Leu Tyr Leu Ser Asp
                245                 250                 255

Asn Leu Leu Asp Ser Ile Pro Gly Pro Leu Pro Leu Ser Leu Arg Ser
            260                 265                 270

Val His Leu Gln Asn Asn Leu Ile Glu Thr Met Gln Arg Asp Val Phe
        275                 280                 285

Cys Asp Pro Glu Glu His Lys His Thr Arg Arg Gln Leu Glu Asp Ile
    290                 295                 300

Arg Leu Asp Gly Asn Pro Ile Asn Leu Ser Leu Phe Pro Ser Ala Tyr
305                 310                 315                 320

Phe Cys Leu Pro Arg Leu Pro Ile Gly Arg Phe Thr
                325                 330

<210>  3
<211>  478
<212>  PRT
<213>  Homo sapiens

<400>  3

Met Ala Pro Leu Arg Pro Leu Leu Ile Leu Ala Leu Leu Ala Trp Val
1               5                   10                  15

Ala Leu Ala Asp Gln Glu Ser Cys Lys Gly Arg Cys Thr Glu Gly Phe
            20                  25                  30

Asn Val Asp Lys Lys Cys Gln Cys Asp Glu Leu Cys Ser Tyr Tyr Gln
        35                  40                  45

Ser Cys Cys Thr Asp Tyr Thr Ala Glu Cys Lys Pro Gln Val Thr Arg
    50                  55                  60

Gly Asp Val Phe Thr Met Pro Glu Asp Glu Tyr Thr Val Tyr Asp Asp
65                  70                  75                  80

Gly Glu Glu Lys Asn Asn Ala Thr Val His Glu Gln Val Gly Gly Pro
                85                  90                  95

Ser Leu Thr Ser Asp Leu Gln Ala Gln Ser Lys Gly Asn Pro Glu Gln
            100                 105                 110

Thr Pro Val Leu Lys Pro Glu Glu Glu Ala Pro Ala Pro Glu Val Gly
        115                 120                 125

Ala Ser Lys Pro Glu Gly Ile Asp Ser Arg Pro Glu Thr Leu His Pro
    130                 135                 140

Gly Arg Pro Gln Pro Pro Ala Glu Glu Glu Leu Cys Ser Gly Lys Pro
145                 150                 155                 160

Phe Asp Ala Phe Thr Asp Leu Lys Asn Gly Ser Leu Phe Ala Phe Arg
                165                 170                 175

Gly Gln Tyr Cys Tyr Glu Leu Asp Glu Lys Ala Val Arg Pro Gly Tyr
            180                 185                 190

Pro Lys Leu Ile Arg Asp Val Trp Gly Ile Glu Gly Pro Ile Asp Ala
        195                 200                 205

Ala Phe Thr Arg Ile Asn Cys Gln Gly Lys Thr Tyr Leu Phe Lys Gly
    210                 215                 220

Ser Gln Tyr Trp Arg Phe Glu Asp Gly Val Leu Asp Pro Asp Tyr Pro
225                 230                 235                 240

Arg Asn Ile Ser Asp Gly Phe Asp Gly Ile Pro Asp Asn Val Asp Ala
                245                 250                 255

Ala Leu Ala Leu Pro Ala His Ser Tyr Ser Gly Arg Glu Arg Val Tyr
            260                 265                 270

Phe Phe Lys Gly Lys Gln Tyr Trp Glu Tyr Gln Phe Gln His Gln Pro
        275                 280                 285

Ser Gln Glu Glu Cys Glu Gly Ser Ser Leu Ser Ala Val Phe Glu His
    290                 295                 300

Phe Ala Met Met Gln Arg Asp Ser Trp Glu Asp Ile Phe Glu Leu Leu
305                 310                 315                 320

Phe Trp Gly Arg Thr Ser Ala Gly Thr Arg Gln Pro Gln Phe Ile Ser
                325                 330                 335

Arg Asp Trp His Gly Val Pro Gly Gln Val Asp Ala Ala Met Ala Gly
            340                 345                 350

Arg Ile Tyr Ile Ser Gly Met Ala Pro Arg Pro Ser Leu Ala Lys Lys
        355                 360                 365

Gln Arg Phe Arg His Arg Asn Arg Lys Gly Tyr Arg Ser Gln Arg Gly
    370                 375                 380

His Ser Arg Gly Arg Asn Gln Asn Ser Arg Arg Pro Ser Arg Ala Thr
385                 390                 395                 400

Trp Leu Ser Leu Phe Ser Ser Glu Glu Ser Asn Leu Gly Ala Asn Asn
                405                 410                 415

Tyr Asp Asp Tyr Arg Met Asp Trp Leu Val Pro Ala Thr Cys Glu Pro
            420                 425                 430

Ile Gln Ser Val Phe Phe Phe Ser Gly Asp Lys Tyr Tyr Arg Val Asn
        435                 440                 445

Leu Arg Thr Arg Arg Val Asp Thr Val Asp Pro Pro Tyr Pro Arg Ser
    450                 455                 460

Ile Ala Gln Tyr Trp Leu Gly Cys Pro Ala Pro Gly His Leu
465                 470                 475

<210>  4
<211>  449
<212>  PRT
<213>  Homo sapiens

<400>  4

Met Met Lys Thr Leu Leu Leu Phe Val Gly Leu Leu Leu Thr Trp Glu
1               5                   10                  15

Ser Gly Gln Val Leu Gly Asp Gln Thr Val Ser Asp Asn Glu Leu Gln
            20                  25                  30

Glu Met Ser Asn Gln Gly Ser Lys Tyr Val Asn Lys Glu Ile Gln Asn
        35                  40                  45

Ala Val Asn Gly Val Lys Gln Ile Lys Thr Leu Ile Glu Lys Thr Asn
    50                  55                  60

Glu Glu Arg Lys Thr Leu Leu Ser Asn Leu Glu Glu Ala Lys Lys Lys
65                  70                  75                  80

Lys Glu Asp Ala Leu Asn Glu Thr Arg Glu Ser Glu Thr Lys Leu Lys
                85                  90                  95

Glu Leu Pro Gly Val Cys Asn Glu Thr Met Met Ala Leu Trp Glu Glu
            100                 105                 110

Cys Lys Pro Cys Leu Lys Gln Thr Cys Met Lys Phe Tyr Ala Arg Val
        115                 120                 125

Cys Arg Ser Gly Ser Gly Leu Val Gly Arg Gln Leu Glu Glu Phe Leu
    130                 135                 140

Asn Gln Ser Ser Pro Phe Tyr Phe Trp Met Asn Gly Asp Arg Ile Asp
145                 150                 155                 160

Ser Leu Leu Glu Asn Asp Arg Gln Gln Thr His Met Leu Asp Val Met
                165                 170                 175

Gln Asp His Phe Ser Arg Ala Ser Ser Ile Ile Asp Glu Leu Phe Gln
            180                 185                 190

Asp Arg Phe Phe Thr Arg Glu Pro Gln Asp Thr Tyr His Tyr Leu Pro
        195                 200                 205

Phe Ser Leu Pro His Arg Arg Pro His Phe Phe Phe Pro Lys Ser Arg
    210                 215                 220

Ile Val Arg Ser Leu Met Pro Phe Ser Pro Tyr Glu Pro Leu Asn Phe
225                 230                 235                 240

His Ala Met Phe Gln Pro Phe Leu Glu Met Ile His Glu Ala Gln Gln
                245                 250                 255

Ala Met Asp Ile His Phe His Ser Pro Ala Phe Gln His Pro Pro Thr
            260                 265                 270

Glu Phe Ile Arg Glu Gly Asp Asp Asp Arg Thr Val Cys Arg Glu Ile
        275                 280                 285

Arg His Asn Ser Thr Gly Cys Leu Arg Met Lys Asp Gln Cys Asp Lys
    290                 295                 300

Cys Arg Glu Ile Leu Ser Val Asp Cys Ser Thr Asn Asn Pro Ser Gln

305                 310                 315                 320

Ala Lys Leu Arg Arg Glu Leu Asp Glu Ser Leu Gln Val Ala Glu Arg
                325                 330                 335

Leu Thr Arg Lys Tyr Asn Glu Leu Leu Lys Ser Tyr Gln Trp Lys Met
            340                 345                 350

Leu Asn Thr Ser Ser Leu Leu Glu Gln Leu Asn Glu Gln Phe Asn Trp
        355                 360                 365

Val Ser Arg Leu Ala Asn Leu Thr Gln Gly Glu Asp Gln Tyr Tyr Leu
    370                 375                 380

Arg Val Thr Thr Val Ala Ser His Thr Ser Asp Ser Asp Val Pro Ser
385                 390                 395                 400

Gly Val Thr Glu Val Val Val Lys Leu Phe Asp Ser Asp Pro Ile Thr
                405                 410                 415

Val Thr Val Pro Val Glu Val Ser Arg Lys Asn Pro Lys Phe Met Glu
            420                 425                 430

Thr Val Ala Glu Lys Ala Leu Gln Glu Tyr Arg Lys Lys His Arg Glu
        435                 440                 445

Glu

<210>  5
<211>  1662
<212>  PRT
<213>  Homo sapiens

<400>  5

Met Gly Pro Thr Ser Gly Pro Ser Leu Leu Leu Leu Leu Leu Thr His
1               5                   10                  15

Leu Pro Leu Ala Leu Gly Ser Pro Met Tyr Ser Ile Ile Thr Pro Asn
            20                  25                  30

Ile Leu Arg Leu Glu Ser Glu Glu Thr Met Val Leu Glu Ala His Asp
        35                  40                  45

Ala Gln Gly Asp Val Pro Val Thr Val Thr His Asp Phe Pro Gly Lys
    50                  55                  60

Lys Leu Val Leu Ser Ser Glu Lys Thr Val Leu Thr Pro Ala Thr Asn

65                  70                  75                  80

His Met Gly Asn Val Thr Phe Thr Ile Pro Ala Asn Arg Glu Phe Lys
                85                  90                  95

Ser Glu Lys Gly Arg Asn Lys Phe Val Thr Val Gln Ala Thr Phe Gly
            100                 105                 110

Thr Gln Val Val Glu Lys Val Val Leu Val Ser Leu Gln Ser Gly Tyr
        115                 120                 125

Leu Phe Ile Gln Thr Asp Lys Thr Ile Tyr Thr Pro Gly Ser Thr Val
    130                 135                 140

Leu Tyr Arg Ile Phe Thr Val Asn His Lys Leu Leu Pro Val Gly Arg
145                 150                 155                 160

Thr Val Met Val Asn Ile Glu Asn Pro Glu Gly Ile Pro Val Lys Gln
                165                 170                 175

Asp Ser Leu Ser Ser Gln Asn Gln Leu Gly Val Leu Pro Leu Ser Trp
            180                 185                 190

Asp Ile Pro Glu Leu Val Asn Met Gly Gln Trp Lys Ile Arg Ala Tyr
        195                 200                 205

Tyr Glu Asn Ser Pro Gln Gln Val Phe Ser Thr Glu Phe Glu Val Lys
    210                 215                 220

Glu Tyr Val Leu Pro Ser Phe Glu Val Ile Val Glu Pro Thr Glu Lys
225                 230                 235                 240

Phe Tyr Tyr Ile Tyr Asn Glu Lys Gly Leu Glu Val Thr Ile Thr Ala
                245                 250                 255

Arg Phe Leu Tyr Gly Lys Lys Val Glu Gly Thr Ala Phe Val Ile Phe
            260                 265                 270

Gly Ile Gln Asp Gly Glu Gln Arg Ile Ser Leu Pro Glu Ser Leu Lys
        275                 280                 285

Arg Ile Pro Ile Glu Asp Gly Ser Gly Glu Val Val Leu Ser Arg Lys
    290                 295                 300

Val Leu Leu Asp Gly Val Gln Asn Pro Arg Ala Glu Asp Leu Val Gly
305                 310                 315                 320

Lys Ser Leu Tyr Val Ser Ala Thr Val Ile Leu His Ser Gly Ser Asp
                325                 330                 335

Met Val Gln Ala Glu Arg Ser Gly Ile Pro Ile Val Thr Ser Pro Tyr
            340                 345                 350

Gln Ile His Phe Thr Lys Thr Pro Lys Tyr Phe Lys Pro Gly Met Pro
        355                 360                 365

Phe Asp Leu Met Val Phe Val Thr Asn Pro Asp Gly Ser Pro Ala Tyr
    370                 375                 380

Arg Val Pro Val Ala Val Gln Gly Glu Asp Thr Val Gln Ser Leu Thr
385                 390                 395                 400

Gln Gly Asp Gly Val Ala Lys Leu Ser Ile Asn Thr His Pro Ser Gln
                405                 410                 415

Lys Pro Leu Ser Ile Thr Val Arg Thr Lys Lys Gln Glu Leu Ser Glu
            420                 425                 430

Ala Glu Gln Ala Thr Arg Thr Met Gln Ala Leu Pro Tyr Ser Thr Val
        435                 440                 445

Gly Asn Ser Asn Asn Tyr Leu His Leu Ser Val Leu Arg Thr Glu Leu
    450                 455                 460

Arg Pro Gly Glu Thr Leu Asn Val Asn Phe Leu Leu Arg Met Asp Arg
465                 470                 475                 480

Ala His Glu Ala Lys Ile Arg Tyr Tyr Thr Tyr Leu Ile Met Asn Lys
                485                 490                 495

Gly Arg Leu Leu Lys Ala Gly Arg Gln Val Arg Glu Pro Gly Gln Asp
            500                 505                 510

Leu Val Val Leu Pro Leu Ser Ile Thr Thr Asp Phe Ile Pro Ser Phe
        515                 520                 525

Arg Leu Val Ala Tyr Tyr Thr Leu Ile Gly Ala Ser Gly Gln Arg Glu
    530                 535                 540

Val Val Ala Asp Ser Val Trp Val Asp Val Lys Asp Ser Cys Val Gly
545                 550                 555                 560

Ser Leu Val Val Lys Ser Gly Gln Ser Glu Asp Arg Gln Pro Val Pro
                565                 570                 575

Gly Gln Gln Met Thr Leu Lys Ile Glu Gly Asp His Gly Ala Arg Val
            580                 585                 590

Val Leu Val Ala Val Asp Lys Gly Val Phe Val Leu Asn Lys Lys Asn
        595                 600                 605

Lys Leu Thr Gln Ser Lys Ile Trp Asp Val Val Glu Lys Ala Asp Ile
    610                 615                 620

Gly Cys Thr Pro Gly Ser Gly Lys Asp Tyr Ala Gly Val Phe Ser Asp
625                 630                 635                 640

Ala Gly Leu Thr Phe Thr Ser Ser Ser Gly Gln Gln Thr Ala Gln Arg
                645                 650                 655

Ala Glu Leu Gln Cys Pro Gln Pro Ala Ala Arg Arg Arg Arg Ser Val
            660                 665                 670

Gln Leu Thr Glu Lys Arg Met Asp Lys Val Gly Lys Tyr Pro Lys Glu
        675                 680                 685

Leu Arg Lys Cys Cys Glu Asp Gly Met Arg Glu Asn Pro Met Arg Phe
    690                 695                 700

Ser Cys Gln Arg Arg Thr Arg Phe Ile Ser Leu Gly Glu Ala Cys Lys
705                 710                 715                 720

Lys Val Phe Leu Asp Cys Cys Asn Tyr Ile Thr Glu Leu Arg Arg Gln
                725                 730                 735

His Ala Arg Ala Ser His Leu Gly Leu Ala Arg Ser Asn Leu Asp Glu
            740                 745                 750

Asp Ile Ile Ala Glu Glu Asn Ile Val Ser Arg Ser Glu Phe Pro Glu
        755                 760                 765

Ser Trp Leu Trp Asn Val Glu Asp Leu Lys Glu Pro Pro Lys Asn Gly
    770                 775                 780

Ile Ser Thr Lys Leu Met Asn Ile Phe Leu Lys Asp Ser Ile Thr Thr
785                 790                 795                 800

Trp Glu Ile Leu Ala Val Ser Met Ser Asp Lys Lys Gly Ile Cys Val
                805                 810                 815

Ala Asp Pro Phe Glu Val Thr Val Met Gln Asp Phe Phe Ile Asp Leu
            820                 825                 830

Arg Leu Pro Tyr Ser Val Val Arg Asn Glu Gln Val Glu Ile Arg Ala
        835                 840                 845

Val Leu Tyr Asn Tyr Arg Gln Asn Gln Glu Leu Lys Val Arg Val Glu
    850                 855                 860

Leu Leu His Asn Pro Ala Phe Cys Ser Leu Ala Thr Thr Lys Arg Arg
865                 870                 875                 880

His Gln Gln Thr Val Thr Ile Pro Pro Lys Ser Ser Leu Ser Val Pro
                885                 890                 895

Tyr Val Ile Val Pro Leu Lys Thr Gly Leu Gln Glu Val Glu Val Lys
            900                 905                 910

Ala Ala Val Tyr His His Phe Ile Ser Asp Gly Val Arg Lys Ser Leu
        915                 920                 925

Lys Val Val Pro Glu Gly Ile Arg Met Asn Lys Thr Val Ala Val Arg
    930                 935                 940

Thr Leu Asp Pro Glu Arg Leu Gly Arg Glu Gly Val Gln Lys Glu Asp
945                 950                 955                 960

Ile Pro Pro Ala Asp Leu Ser Asp Gln Val Pro Asp Thr Glu Ser Glu
                965                 970                 975

Thr Arg Ile Leu Leu Gln Gly Thr Pro Val Ala Gln Met Thr Glu Asp
            980                 985                 990

Ala Val Asp Ala Glu Arg Leu Lys His Leu Ile Val Thr Pro Ser Gly
        995                 1000                1005

Cys Gly Glu Gln Asn Met Ile Gly Met Thr Pro Thr Val Ile Ala
    1010                1015                1020

Val His Tyr Leu Asp Glu Thr Glu Gln Trp Glu Lys Phe Gly Leu
    1025                1030                1035

Glu Lys Arg Gln Gly Ala Leu Glu Leu Ile Lys Lys Gly Tyr Thr
    1040                1045                1050

Gln Gln Leu Ala Phe Arg Gln Pro Ser Ser Ala Phe Ala Ala Phe
    1055                1060                1065

Val Lys Arg Ala Pro Ser Thr Trp Leu Thr Ala Tyr Val Val Lys

1070                1075                1080

Val Phe Ser Leu Ala Val Asn Leu Ile Ala Ile Asp Ser Gln Val
    1085                1090                1095

Leu Cys Gly Ala Val Lys Trp Leu Ile Leu Glu Lys Gln Lys Pro
    1100                1105                1110

Asp Gly Val Phe Gln Glu Asp Ala Pro Val Ile His Gln Glu Met
    1115                1120                1125

Ile Gly Gly Leu Arg Asn Asn Asn Glu Lys Asp Met Ala Leu Thr
    1130                1135                1140

Ala Phe Val Leu Ile Ser Leu Gln Glu Ala Lys Asp Ile Cys Glu
    1145                1150                1155

Glu Gln Val Asn Ser Leu Pro Gly Ser Ile Thr Lys Ala Gly Asp
    1160                1165                1170

Phe Leu Glu Ala Asn Tyr Met Asn Leu Gln Arg Ser Tyr Thr Val
    1175                1180                1185

Ala Ile Ala Gly Tyr Ala Leu Ala Gln Met Gly Arg Leu Lys Gly
    1190                1195                1200

Pro Leu Leu Asn Lys Phe Leu Thr Thr Ala Lys Asp Lys Asn Arg
    1205                1210                1215

Trp Glu Asp Pro Gly Lys Gln Leu Tyr Asn Val Glu Ala Thr Ser
    1220                1225                1230

Tyr Ala Leu Leu Ala Leu Leu Gln Leu Lys Asp Phe Asp Phe Val
    1235                1240                1245

Pro Pro Val Val Arg Trp Leu Asn Glu Gln Arg Tyr Tyr Gly Gly
    1250                1255                1260

Gly Tyr Gly Ser Thr Gln Ala Thr Phe Met Val Phe Gln Ala Leu
    1265                1270                1275

Ala Gln Tyr Gln Lys Asp Ala Pro Asp His Gln Glu Leu Asn Leu
    1280                1285                1290

Asp Val Ser Leu Gln Leu Pro Ser Arg Ser Ser Lys Ile Thr His
    1295                1300                1305

Arg Ile His Trp Glu Ser Ala Ser Leu Leu Arg Ser Glu Glu Thr
    1310                1315                1320

Lys Glu Asn Glu Gly Phe Thr Val Thr Ala Glu Gly Lys Gly Gln
    1325                1330                1335

Gly Thr Leu Ser Val Val Thr Met Tyr His Ala Lys Ala Lys Asp
    1340                1345                1350

Gln Leu Thr Cys Asn Lys Phe Asp Leu Lys Val Thr Ile Lys Pro
    1355                1360                1365

Ala Pro Glu Thr Glu Lys Arg Pro Gln Asp Ala Lys Asn Thr Met
    1370                1375                1380

Ile Leu Glu Ile Cys Thr Arg Tyr Arg Gly Asp Gln Asp Ala Thr
    1385                1390                1395

Met Ser Ile Leu Asp Ile Ser Met Met Thr Gly Phe Ala Pro Asp
    1400                1405                1410

Thr Asp Asp Leu Lys Gln Leu Ala Asn Gly Val Asp Arg Tyr Ile
    1415                1420                1425

Ser Lys Tyr Glu Leu Asp Lys Ala Phe Ser Asp Arg Asn Thr Leu
    1430                1435                1440

Ile Ile Tyr Leu Asp Lys Val Ser His Ser Glu Asp Asp Cys Leu
    1445                1450                1455

Ala Phe Lys Val His Gln Tyr Phe Asn Val Glu Leu Ile Gln Pro
    1460                1465                1470

Gly Ala Val Lys Val Tyr Ala Tyr Tyr Asn Leu Glu Glu Ser Cys
    1475                1480                1485

Thr Arg Phe Tyr His Pro Glu Lys Glu Asp Gly Lys Leu Asn Lys
    1490                1495                1500

Leu Cys Arg Asp Glu Leu Cys Arg Cys Ala Glu Glu Asn Cys Phe
    1505                1510                1515

Ile Gln Lys Ser Asp Asp Lys Val Thr Leu Glu Glu Arg Leu Asp
    1520                1525                1530

Lys Ala Cys Glu Pro Gly Val Asp Tyr Val Tyr Lys Thr Arg Leu
    1535                1540                1545

Val Lys Val Gln Leu Ser Asn Asp Phe Asp Glu Tyr Ile Met Ala
    1550                1555                1560

Ile Glu Gln Thr Ile Lys Ser Gly Ser Asp Glu Val Gln Val Gly
    1565                1570                1575

Gln Gln Arg Thr Phe Ile Ser Pro Ile Lys Cys Arg Glu Ala Leu
    1580                1585                1590

Lys Leu Glu Glu Lys Lys His Tyr Leu Met Trp Gly Leu Ser Ser
    1595                1600                1605

Asp Phe Trp Gly Glu Lys Pro Asn Leu Ser Tyr Ile Ile Gly Lys
    1610                1615                1620

Asp Thr Trp Val Glu His Trp Pro Glu Glu Asp Glu Cys Gln Asp
    1625                1630                1635

Glu Glu Asn Gln Lys Gln Cys Gln Asp Leu Gly Ala Phe Thr Glu
    1640                1645                1650

Ser Met Val Val Phe Gly Cys Pro Asn
    1655                1660

<210>  6
<211>  418
<212>  PRT
<213>  Homo sapiens

<400>  6

<210>  7
<211>  190
<212>  PRT
<213>  Homo sapiens

<400>  7

Met Ala Thr His His Thr Leu Trp Met Gly Leu Ala Leu Leu Gly Val
1               5                   10                  15

Leu Gly Asp Leu Gln Ala Ala Pro Glu Ala Gln Val Ser Val Gln Pro
            20                  25                  30

Asn Phe Gln Gln Asp Lys Phe Leu Gly Arg Trp Phe Ser Ala Gly Leu
        35                  40                  45

Ala Ser Asn Ser Ser Trp Leu Arg Glu Lys Lys Ala Ala Leu Ser Met
    50                  55                  60

Cys Lys Ser Val Val Ala Pro Ala Thr Asp Gly Gly Leu Asn Leu Thr
65                  70                  75                  80

Ser Thr Phe Leu Arg Lys Asn Gln Cys Glu Thr Arg Thr Met Leu Leu
                85                  90                  95

Gln Pro Ala Gly Ser Leu Gly Ser Tyr Ser Tyr Arg Ser Pro His Trp
            100                 105                 110

Gly Ser Thr Tyr Ser Val Ser Val Val Glu Thr Asp Tyr Asp Gln Tyr
        115                 120                 125

Ala Leu Leu Tyr Ser Gln Gly Ser Lys Gly Pro Gly Glu Asp Phe Arg
    130                 135                 140

Met Ala Thr Leu Tyr Ser Arg Thr Gln Thr Pro Arg Ala Glu Leu Lys
145                 150                 155                 160

Glu Lys Phe Thr Ala Phe Cys Lys Ala Gln Gly Phe Thr Glu Asp Thr
                165                 170                 175

Ile Val Phe Leu Pro Gln Thr Asp Lys Cys Met Thr Glu Gln
            180                 185                 190

## Description

### Field of the invention

The present invention belongs to the field of eye-related diseases and relates to a method for analyzing, diagnosing, and/or treating said diseases.

### Description of the background art

There are eye-related diseases, either affecting the physiology and/or the eyes themselves/directly, or as an indication of other optionally systemic diseases where the eye is affected in conjunction. In particular, direct eye diseases are selected from the group consisting of retino-choroidal diseases, retinal vascular diseases, diabetic retinopathy, retinal detachment, and macular edemas, may be a common cause of vision loss, in particular in older patients. Examples of such eye-related diseases are for instance Age-Related Macular Degeneration (AMD) and Retinal vein occlusion (RVO). AMD and RVO are used as exemplary eye-related diseases.

Age-Related Macular Degeneration (AMD) is a complex, multifactorial disease and the major cause of central vision loss among the elderly in western, industrialized countries. Neovascular or wet AMD (nAMD) with choroidal neovascularization (CNV) is considered more aggressive, due to the rapid disease progression without therapy, than late dry AMD with geographic atrophy. It is estimated, that in Germany (representing the other western countries) the incidence of AMD, due to the ageing population, will almost double by the year 2030.

Intensive experimental and clinical research in recent years expanded our knowledge on the pathophysiology of nAMD. Today dysregulated complement system activation, oxidative stress and inflammatory processes are widely accepted as cofactors to proangiogenic activity mediated by VEGF. Nevertheless, there is still a lack of understanding of many pathophysiological components, especially on a molecular level.

The introduction of anti-VEGF agents marked a breakthrough in the therapy of nAMD. Nevertheless, a consistent dosing scheme is missing, the benefit of anti-VEGF agents for all patients is not predictable and long term anti-VEGF treatment may have severe side effects on ocular tissues. Therefore, research to evaluate potential future biomarkers or possible new targets of therapy is warranted and may overcome these obstacles.

Retinal vein occlusion (RVO) is the second most common cause of vision loss in older patients due to retinal vascular disease after diabetic retinopathy. Despite major achievements in diagnosis and treatment perspectives based on spectral OCT, there is still limited understanding of the pathophysiology of RVO. A number of cytokines including vascular endothelial growth factor (VEGF) and interleukin-6 (IL-6) have been shown to be associated to RVO. However these cytokines display high interindividual variations and their concentrations often do not correspond to the associated clinical RVO phenotype. Even in non-diseased eyes, reproducible vitreous protein profiling is quite complicated as the aging vitreous incorporates different stages of liquefaction and syneresis, which is aggravated after treatment with intravitreal injections. Therefore, many aspects of the molecular mechanism of RVO remain poorly described.

Proteomics is a promising approach allowing the analysis of the total protein content of a sample and in combination with robust statistical analyses can lead to the identification of proteins associated to specific diseases in ophthalmology. At the same time, proteome analysis is one of the most advanced exploratory techniques for the discovery of new protein biomarkers of clinical significance. A number of studies have already employed proteome analysis for the study of age-related macular degeneration. Most of them used aqueous humor aspirates, were performed *ex-vivo* or used animal samples.

Koss et al. ("Proteomics of Vitreous Humor of Patients with Exudative Age-Related Macular Degeneration", PLOS ONE, May 2014, Vol. 9, Issue 5) describes the proteomics vitreous humor of patients with exudative age-related macular degeneration. In this context, a general list of proteins/polypeptides that are present in vitreous humor of humans suffering from age-related macular degeneration (AMD) could be set up.

Following from the above, there is a need for providing marker that, for instance, allow for analyzing, diagnosing, and/or treating eye-related diseases: Such markers may not only be indicative of the presence of eye-related diseases, but they may also serve as therapeutic targets for the treatment of such diseases.

### Summary of the invention

The present invention provides the following aspects, subject-matters and preferred embodiments, which respectively taken alone or in combination, contribute to solving the object of the present invention.
(1) Method for analyzing and/or diagnosing an eye-related disease,
   the method comprising determining in vitro the amount of one or more marker(s) selected from the group consisting of the proteins/polypeptides Immunoglobulin lambda-like polypeptide 5, Opticin, Vitronectin, Clusterin, Complement C3, pigment-epithelium-derived factor, and prostaglandin-H2 D-isomerase, or a variant thereof, and determining whether or not a respective eye-related disease on the basis of the amount of the marker(s) is present, and wherein the respective variant exhibits % identity of 90% or higher to the amino acid sequence of the respective wildtype polypeptide.
   In a preferred embodiment according to item (1), one or more of only these polypeptides listed in item (1) are determined.
   Within the meaning of the present invention it has surprisingly been found that by in vitro determining the amount of one or more of the polypeptides listed in item (1), preferably by determining the amount of one or more of only the polypeptides listed in item (1) it can reliably and reproducibly be determined whether a test subject suffers from an eye-related disease as disclosed elsewhere herein.
(2) Method for analyzing and/or diagnosing an eye-related disease according to item (1), wherein the eye-related disease is selected from the group consisting of retino-choroidal diseases, retinal vascular diseases, diabetic retinopathy, retinal detachment, and macular edemas.
(3) Method for analyzing and/or diagnosing an eye-related disease according to item (1), wherein the eye-related disease is selected from the group consisting of diabetes mellitus, rheumatism, vascular diseases, cardiac diseases, stroke, anemia, and leukemia.
(4) Method for analyzing and/or diagnosing according to any of items (1) to (3), wherein the one or more marker(s) or variant thereof is (are) present in a test sample derived from a test subject.
(5) Method according to any of the preceding items, wherein
   Immunoglobulin lambda-like polypeptide 5 is a polypeptide comprising, preferably consisting of, the sequence shown in SEQ ID NO: 1;
   Opticin is a polypeptide comprising, preferably consisting of, the sequence shown in SEQ ID NO: 2;
   Vitronectin is a polypeptide comprising, preferably consisting of, the sequence shown in SEQ ID NO: 3;
   Clusterin is a polypeptide comprising, preferably consisting of, the sequence shown in SEQ ID NO: 4;
   Complement C3 is a polypeptide comprising, preferably consisting of, the sequence shown in SEQ ID NO: 5;
   Pigment-epithelium-derived factor is a polypeptide comprising, preferably consisting of, the sequence shown in SEQ ID NO: 6; and
   Prostaglandin-H2 D-isomerase is a polypeptide comprising, preferably consisting of, the sequence shown in SEQ ID NO: 7.
(6) The method according to any of items (1) to (5), wherein when the amount of the respective marker for analyzing and/or diagnosing the respective disease selected from the group as defined in any of items (1) to (3) in a test subject is statistically significantly different from that of a normal individual, the test subject is determined to have the respective disease.
(7) The method according to item (6), wherein a P-Value of α<5.00E-02, being analyzed by using the Mann-Whitney test, is considered statistically significant.
(8) The method according to any of items (1) to (7), wherein the test subject is determined to have any of the disease defined in any of items (1) to (3), if the amount of Immunoglobulin lambda-like polypeptide 5 or a variant thereof is significantly higher; the amount of Opticin or a variant thereof is significantly lower; the amount of Vitronectin or a variant thereof is significantly higher; the amount of Clusterin or a variant thereof is significantly higher; the amount of Complement C3 or a variant thereof is significantly higher; the amount of pigment-epithelium-derived factor or a variant thereof is significantly higher; and/or the amount of prostaglandin-H2 D-isomerase or a variant thereof is significantly higher; respectively compared to the amount of the respective wildtype polypeptide.
(9) The method according to any of the preceding items, wherein
   retino-choroidal diseases comprise age-related macular degeneration (AMD), in particular neovascular AMD (nAMD);
   retinal vascular diseases comprise retinal vein occlusion (RVO) such as central retinal vein occlusion, hemi-central retinal vein occlusion, and brach retinal vein occlusion;
   diabetic retinopathy comprises diabetic macular edema, retinal thrombosis including macular edema, and
   further macular edemas comprising inflammatory and postoperative macular edemas.
(10) The method according to any of the preceding items, wherein the amount of one or more of the marker(s) selected from the group consisting of the polypeptides Immunoglobulin lambda-like polypeptide 5, Opticin, Vitronectin, Clusterin, and Complement C3, or a respective variant thereof, is determined in vitro for determining whether or not the test subject has retinal vascular diseases, preferably RVO.
   In particular, within the meaning of the present invention it has been surprisingly found that by in vitro determining the amount of one or more of the polypeptides listed in item (10), it can be determined whether a test subject suffers from (has) a retinal vascular disease such as RVO.
(11) The method according to item (10), wherein the amount of Immunoglobulin lambda-like polypeptide 5 and Complement C3, or a variant thereof, is determined in vitro for determining whether or not the test subject has retinal vascular diseases, preferably RVO.
(12) The method according to item (10), wherein the respective amount of all the marker polypeptides Immunoglobulin lambda-like polypeptide 5, Opticin, Vitronectin, Clusterin, and Complement C3, or a respective variant thereof, is determined in vitro for determining whether or not the test subject has RVO.
(13) The method according to any of the preceding items (1) to (9) wherein the amount of one or more of the marker(s) selected from the group consisting of the polypeptides Opticin, Clusterin, pigment-epithelium-derived factor, and prostaglandin-H2 D-isomerase, or a respective variant thereof, is determined in vitro for determining whether or not a test subject has diabetic retinopathy comprising diabetic macular edema, retinal thrombosis including macular edema, further macular edemas comprising inflammatory and postoperative macular edemas, and/or retino-choroidal diseases comprising age-related macular degeneration (AMD), in particular neovascular AMD (nAMD).
(14) The method according to item (13), wherein the respective amount of all the marker polypeptides Opticin, Clusterin, pigment-epithelium-derived factor, and prostaglandin-H2 D-isomerase, or a respective variant thereof, preferably the amount of the marker polypeptides Opticin, Clusterin, and prostaglandin-H2 D-isomerase, or a respective variant thereof, is determined in vitro for determining whether or not the test subject has diabetic macular edema, retinal thrombosis including macular edema, further macular edemas comprising inflammatory and postoperative macular edemas, and/or age-related macular degeneration (AMD), preferably neovascular AMD (nAMD).
(15) The method according to any of the preceding items, wherein the amount of the marker(s) is (are) determined comprising the use of, or by applying, capillary electrophoresis coupled to mass spectrometry (CE-MS analysis).
(16) Use of one or more marker polypeptide(s) selected from the group consisting of the polypeptides Immunoglobulin lambda-like polypeptide 5, Opticin, Vitronectin, Clusterin, Complement C3, pigment-epithelium-derived factor, and prostaglandin-H2 D-isomerase, or a variant thereof, for analyzing and/or diagnosing eye-related diseases, wherein the respective variant exhibits % identity of 90% or higher to the amino acid sequence of the respective wildtype polypeptide.
(17) The use according to item (16), wherein the eye-related diseases is selected from the group as defined in item (2) or (3).
(18) The use according to item (17), wherein retino-choroidal diseases comprise age-related macular degeneration (AMD), in particular neovascular AMD (nAMD);
   retinal vascular diseases comprise retinal vein occlusion (RVO) such as central retinal vein occlusion, hemi-central retinal vein occlusion, and brach retinal vein occlusion;
   diabetic retinopathy comprises diabetic macular edema, retinal thrombosis including macular edema, and
   further macular edemas comprising inflammatory and postoperative macular edemas.
(19) Method for treating an eye-related disease, the method comprising the step of determining in vitro the amount of one or more marker(s) selected from the group as defined in claim 1, determining whether or not a respective eye-related disease on the basis of the determined amount of the marker(s) is present in a test subject, and subjecting this test subject to an adequate treatment.
   In case of a too high amount (i.e. an amount that results in disease signs in the test subject, which is generally the case if the amount of the respective polypeptide is significantly higher than the amount of the corresponding wildtype polypeptide), the test person can be subjected to an adequate treatment. Such an adequate treatment is directed at compensating the overexpression. An adequate treatment is thus for instance the administration of suitable antibodies inhibiting the function of the overexpressed polypeptide, of siRNA, and/or of antagonists of the function of the respective polypeptide.
   In case of a too low amount (i.e. an amount that results in disease signs in the test subject, which is generally the case if the amount of the respective polypeptide is significantly lower than the amount of the corresponding wildtype polypeptide), the test person can be subjected to an adequate treatment. Such an adequate treatment is directed at compensating the downregulation, i.e. the too low amount. An adequate treatment is thus for instance the administration of the polypeptide itself which is present in too low amounts, or the introduction of an expression vector that comprises a nucleotide sequence encoding and expressing the affected polypeptide. It is also possible to administer other compounds than the polypeptide, and that are able to compensate the too low amount of the affected polypeptide.
(20) Method for treating an eye-related disease according to item (19), wherein the eye-related disease is selected from the group as defined in any of items (2), (3), (13), and (14), and/or wherein the marker(s) is (are) selected from the group as defined in any of items (5), (11), (12), and (13).
(21) Method for treating an eye-related disease according to items (19) or (20), wherein the test subject is determined to have any of the disease defined in item (18), if the amount of Immunoglobulin lambda-like polypeptide 5 or a variant thereof is significantly higher; the amount of Opticin or a variant thereof is significantly lower; the amount of Vitronectin or a variant thereof is significantly higher; the amount of Clusterin or a variant thereof is significantly higher; the amount of Complement C3 or a variant thereof is significantly higher; the amount of pigment-epithelium-derived factor or a variant thereof is significantly higher; and/or the amount of prostaglandin-H2 D-isomerase or a variant thereof is significantly higher.
(22) Use of a kit for analyzing, diagnosing, and/or treating eye-related diseases selected from the group consisting of retino-choroidal diseases, retinal vascular diseases, diabetic retinopathy, retinal detachment, and macular edemas, wherein the kit comprises one or more antibodie(s) that is (are) specifically directed against one or more marker(s) selected from the group of polypeptide(s) or variant(s) thereof as defined in item (1) or (2).
(23) Use of a kit according to item (22), wherein the kit comprises one or more antibodie(s) that is (are) directed against the marker(s) as defined in any of items (10), (11), or (12), and/or wherein the disease is as defined in any of items (10), (11), or (12).
(24) Use of a kit according to item (22), wherein the kit comprises one or more antibodie(s) that is (are) directed against the marker(s) as defined in item (13) or (14), and/or wherein the disease is as defined in item (13) or (14).
(25) A DNA chip for analyzing and/or diagnosing diseases selected from the group consisting of retino-choroidal diseases, retinal vascular diseases, diabetic retinopathy, retinal detachment, and macular edemas, comprising one or more nucleic acid sequences selected from the group of nucleotide sequences that respectively encode the polypeptide(s) as defined in item (1) or (2).
(26) The DNA chip according to item (25), comprising one or more nucleic acid sequence(s) selected from the group of nucleotide sequences that respectively encode the polypeptide(s) as defined in any of items (10), (11), or (12), and/or wherein the disease is as defined in any of items (10), (11), or (12).
(27) The DNA chip according to item (25), comprising one or more nucleic acid sequence(s) selected from the group of nucleotide sequences that respectively encode the polypeptide(s) as defined in item (13) or (14), and/or wherein the disease is as defined in item (13) or (14).
(28) The use of a kit according to any of items (22) to (24), or the DNA chip according to any of items (25) to (27), in a method as defined in any of items (1) to (21).
(29) In vitro method for identifying a test compound that is capable of increasing or decreasing the amount of one or more of the polypeptide(s) as defined in item (1) in a test sample, comprising the steps of
   (a) carrying out a method as defined in any of items (1) to (15) by using a test sample, and based on the result determining whether an eye-related disease is present;
   (b) if it is determined that an eye-related disease is present, contacting the test compound with the same or a corresponding test sample;
   (c) determining whether the test compound has increased or decreased the amount of the polypeptide(s).

With regard to the terms and expressions (e.g. "test sample", "eye-related disease", "test sample", "polypeptide") in item (29), reference is made to the description elsewhere herein.

Within the meaning of the present invention, a test compound can be any compound that is able to modulate (e.g. change) the amount of the respective marker polypeptide that is determined as being present in a different amount when compared to the wildtype situation. Examples of such a compound are listed elsewhere herein, in particular in the context of the disclosure referring to the term "treating" or "adequate treatment", for instance inhibitors or activators of the function of the respective, affected polypeptide, antibodies, siRNA, or compounds in general that compensate the overexpression/downregulationof the affected polypeptide.

"Modulating" or "changing" means leading to an increase or decrease of the affected, respective polypeptide. Whether the application of the test compound resulted in an increase or decrease of the respective, assessed polypeptide can be determined by any suitable method that is known to a person skilled in the art. Such methods are listed elsewhere herein and comprise for instance western blotting, ELISA, or CE-MS analysis.

After having determined a suitable test compound that is capable of modulating the amount (and, thus, the function) of a certain polypeptide, this compound can be used in the treatment of a non-human subject suffering from an eye-related disease as disclosed elsewhere herein. Based on the determined amount of the respective polypeptide marker analysed, it can be determined whether or not an eye-related disease is present. The too high or too low amount of a respective analysed marker is indicative of the presence of an eye-related disease. With this regard, reference is made to items (19) to (21) above. In particular, an eye-related disease is present if the amount of Immunoglobulin lambda-like polypeptide 5 or a variant thereof is significantly higher; the amount of Opticin or a variant thereof is significantly lower; the amount of Vitronectin or a variant thereof is significantly higher; the amount of Clusterin or a variant thereof is significantly higher; the amount of Complement C3 or a variant thereof is significantly higher; the amount of pigment-epithelium-derived factor or a variant thereof is significantly higher; and/or the amount of prostaglandin-H2 D-isomerase or a variant thereof is significantly higher.

### Detailed description of the invention

The present invention is now described in more detail by preferred embodiments and examples, which are however presented for illustrative purpose only and shall not be understood as limiting the scope of the present invention in any way.

Within the meaning of the present invention it has been found that the specific polypeptides disclosed herein can be used as marker for analyzing, diagnosing, and/or treating eye-related diseases, such as diseases as disclosed herein. The present invention further provides a method for analyzing, diagnosing, and/or treating eye-related diseases. For this purpose it can be sufficient to determine the presence (and if present, the amount) of at least one of said polypeptides. Determining the presence (and amount) however of multiple (e.g. two or more, three or more, four or more, or five or more), the precision of the obtained result can be improved.

It is not only possible, based on the respective assessed presence or absence of the respective polypeptide, and the amount thereof, to analyze the health status of the eye (and the test subject, respectively), but also to adapt a suitable treatment. Such a treatment can for instance be the treatment of the excessive (or too less) amount of the respective polypeptide, that is differing from the normal amount that can be determined in a healthy test subject under the same conditions. For instance a possible suitable treatment can be the administration of neutralizing antibodies that specifically bind to the respective upregulated (compared to a healthy test subject) polypeptide, thus inhibiting its function (action), or providing a test person exhibiting downregulated expression of marker polypeptides with a balancing amount of said downregulated marker polypeptide(s).

Within the meaning of the present invention, the term "diagnosing" refers to the recognition (detection) of a disease (such as an eye-related disease as defined herein) by carrying out respective steps (i.e. taking suitable measures), such as examining a test subject.

Within the meaning of the present invention, the term "overexpression" of a certain protein such as a polypeptide defines that the expression thereof is at levels greater than normal in a wildtype cell. For instance, in the present invention the marker polypeptides can be overexpressed. The statistical significance of such an overexpression is explained elsewhere herein. As a result, the amount of the respective protein is higher, compared to the amount of said protein in wildtype status.

The other way round, the expression that the amount of a protein is lower means that it is lower compared to the amount of said protein in wildtype status.

The terms "higher" and "lower" are always to be understood in relation to the amount of the respective protein (e.g. polypeptide) in wildtype status.

Within the meaning of the present invention, the term "treating", or "adequate treatment", respectively, refers to taking specific measures against a disease a test subject may suffer from. For instance, if a test subject suffers from an eye-related disease that is (also) caused by overexpression of a certain polypeptide, such a test subject could be treated by taking suitable measures that compensate (or balance) this overexpression. Such suitable measures could for instance be means that (down)regulate the expression of the affected polypeptide on the genetic level (nucleic acid level), such as the introduction of modifications (e.g. mutations) into the nucleic acid sequence that encodes the affected polypeptide. Further, suitable measures can also be means that interfere with the respective RNA (ribonucleic acid), e.g. siRNA. Suitable measures can also be means that affect the overexpressed polypeptide itself, e.g. the use of suitable antagonists (e.g. antibodies) that specifically bind said overexpressed polypeptide, and thus inhibit its action (function). Further, it is also possible that the overexpression of a certain polypeptide is caused by the affected function of a possible antagonist or precursor of the overexpressed polypeptide, which antagonist would, in a wildtype-state, keep the amount of the polypeptide on a wildtype level. It is also possible that the interaction between the polypeptide on the one hand and a respective precursor, or a respective antagonist of the polypeptide, or a cell, on the other hand, results in overexpression of said polypeptide.

All the measures taken in regard the overexpression of a certain polypeptide aim at and finally are desired to result in a down regulation of the expression of the affected polypeptide to a level that is found in healthy individuals.

If a test subject suffers from a too low amount of a certain polypeptide, e.g. caused by a downregulation of the respective polypeptide (e.g. a protective polypeptide), likewise suitable measures can be taken in order to adjust the level (amount) of the affected polynucleotide to a level (amount) that is present in healthy individuals (wildtype level/wildtype amount). Suitable measures can for instance be the introduction of an expression vector that comprises a nucleotide sequence encoding and expressing the affected polypeptide. Further suitable measures can be the administration of the polypeptide itself (or its precursors) to the test subject, e.g. systemically or locally. It can also be possible to compensate the too low amount of the affected polypeptide by providing the test subject with precursors of the downregulated polypeptide, or with other compounds that are able to compensate the too low amount of the affected polypeptide.

Within the meaning of the present invention, the term "wildtype" denotes that a certain matter, e.g. a polypeptide, such as a marker polypeptide as defined herein, is present in its natural occurring state, and thus exerts its natural function and/or activity.

Within the meaning of the present invention, the term "analyzing" refers to studying or assessing (determining) the state or condition of (a certain part of) a subject, such as a test subject. For instance, the condition of an eye can be analyzed, e.g. in terms of determining the amount of the marker polypeptides of the present invention.

Within the meaning of the present invention, the term "eye-related diseases" denotes diseases that are in connection with the eyes, as initially mentioned either directly or indirectly. Primarily, the term denotes retino-choroidal diseases, choroidal diseases, retinal vascular diseases, diabetic retinopathy, retinal detachment, and macular edemas. Macular edemas comprise inflammatory and postoperative macular edemas. Optionally, the selected marker(s) defined above may be used in the analysis, diagnosis and/or therapy of diseases which are potentially associated with eye diseases, i.e. meaning "eye-related diseases" in the present invention more generally, such as diabetes mellitus; rheumatism; vascular diseases; cardiac diseases; stroke; anemia; and leukemia.

In the present invention, the principle underlying the present invention is exemplified and thus disclosed in connection with the eye-disease "Age-related Macular Degeneration" (AMD) and retinal vein occlusion (RVO) as model eye-related diseases. The polypeptide markers that are identified in the present invention are not limited to these two model eye-diseases only, but can likewise be used as marker polypetides for analyzing, diagnosing, and/or treating further eye-related diseases disclosed herein. In particular, with this "proof of concept", the defined markers are indicative for other eye diseases, but alternatively and optionally for the aforementioned eye-related diseases indirectly but generally.

In the present invention, the "marker for analyzing, diagnosing, and/or treating eye-related diseases" is a polypeptide marker (also referred to herein as "polypeptide" or "marker") intended for analyzing, diagnosing, and/or treating eye-related diseases and refers to a polypeptide that serves as an indicator (marker) showing that the test subject has the respective eye-related disease. The respective polypeptide marker(s) for analyzing, diagnosing, and/or treating eye-related diseases are the polypeptides Immunoglobulin lambda-like polypeptide 5, Opticin, Vitronectin, Clusterin, Complement C3, pigment-epithelium-derived factor, and prostaglandin-H2 D-isomerase, or a variant thereof.

In the present specification, the term "variant" of the respective polypeptide marker means a variant comprising, or consisting of, an amino acid sequence that exhibits % identity of approximately 90% or higher, more preferably approximately 95% or higher, approximately 97% or higher, approximately 98% or higher, or approximately 99% or higher, to the amino acid sequence or its partial sequence of the respective polypeptide, preferably the respective polypeptide shown in SEQ ID NO: 1 to 6. The "% identity" can be determined with or without a gap introduction using a BLAST- or FASTA-based protein search system (Karlin, S. et al., 1993, Proceedings of the National Academic Sciences U.S.A., Vol. 90, p. 5873-5877; Altschul, S.F. et al., 1990, Journal of Molecular Biology, Vol. 215, p. 403-410; and Pearson, W.R. et al., 1988, Proceedings of the National Academic Sciences U.S.A., Vol. 85, p. 2444-2448).

In addition to the above-defined property with regard to sequence identity, the variant within the meaning of the present invention still exhibits its natural function and activity. This can be assessed by any suitable test that is known to a skilled person, for instance by applying fluorescence dyes.

The term "variant" also comprises isomers of the respective polypeptides, and splice variants of the respective polypeptides.

In the present specification, the "test subject" refers to a specimen subjected to the detection of eye-related disease affecting the individual and corresponds to a vertebrate, preferably a mammal, particularly preferably a human.

The step of determining the amount of the respective marker polypeptide is the step of measuring in vitro the amount of the respective marker polypetide or variant thereof, which typically is present in the vitreous humor derived from a test subject.

Determining the amount (or mass) of the respective polypeptide marker in a test sample such as in ocular fluids like e.g. vitreous humor, aqueous humor or tear film, can be carried out by any method that is known to a person skilled in the art. The respective amount can for instance be determined by suitable analytical chemistry techniques, such as capillary electrophoresis coupled to mass spectrometry (CE-MS), preferably as it is described in Theodorescu et al. (Theodorescu D, Wittke S, Ross MM, Walden M, Conaway M, Just I, et al. Discovery and validation of new protein biomarkers for urothelial cancer: a prospective analysis. Lancet Oncol. 2006;7:230-40), and/or by liquid chromatography coupled to tandem mass spectrometry (LC-MS/MS) according to standard protocols known to a person skilled in the art. Examples of possible methods for determining the amount of the respective marker polypeptide are for instance gas chromatography-mass spectrometry liquid chromatography-mass spectrometry, ion mobility spectrometry-mass spectrometry protein chips, reverse-phased protein microarrays. Further examples are quantitative detection methods like western blot, Enzyme-linked immunosorbent Assay (ELISA), or cytometric bead assay.

It is also possible to detect (quantitatively and qualitatively) the presence and/or amount of the respective polypeptide markers by using antibodies that specifically bind to said polypeptides. These antibodies can for instance be present in a kit, which, in turn, can be used in determining whether, and if so, in which amount, the respective polypeptide marker is present in the sample to be tested (test sample).

Within the meaning of the present invention, such a test sample preferably is an eye-related test sample (e.g. derived from the eye), comprising ocular fluids, which can be human or non-human. Examples of such ocular fluids are vitreous humor, aqueous humor, tear film, suprachoroidal fluid, or subretinal fluid. The test sample can further comprise ocular tissues such as coronal neovascularization (CNV), choroid tissue, sub-intra membranes, and epiretinal membranes.

Preparing (treating) the test sample, if this should be necessary, such that it can be subjected to a procedure for determining the amount of the respective polypeptide marker represents routine work that is known to a person skilled in the art.

In the present specification, the "amount of the marker" for analyzing, diagnosing, and/or treating diseases of the present invention refers to the quantity of the respective marker polypeptide present in a test sample (e.g. eye-related test sample such as vitreous humor) derived from a test subject. This quantity may be any of absolute and relative amounts. The absolute amount corresponds to the mass or volume of the marker. The relative amount refers to a relative value indicated by the measured value of the test subject-derived marker compared with a particular measured value. Examples thereof include concentration, fluorescence intensity, and absorbance. As disclosed elsewhere herein, suitable methods for determining the amount of a polypeptide or marker, respectively, are known to a person skilled in the art and represent routine methods.

The present invention also refers to the use of a kit comprising antibodies that are specifically directed against the polypeptide markers of the present invention. The generation of antibodies that are directed against a certain polypeptide is known to a person skilled in the art and can be carried out according to suitable protocols. One possibility is to immunize a suitable subject with the respective polypeptide marker. The immunized subject (e.g. non-human animal) then produces antibodies that are specifically directed against the polypeptide that has been used for immunization. The antibodies produced are present in the blood of the immunized subject and may be recovered by standard procedures that are well known to skilled persons. The antibodies can be monoclonal or polyclonal, depending on the generation process of the antibodies.

The antibody preferably is an antibody that binds specifically to its intended target polypeptide (the marker). The term "specific binding" within the meaning of the present invention denotes that the generated antibody exhibits specificity for the respective intended target polypeptide that may be present in the vitreous humor of a test subject. The generated antibody does not bind to the other polypeptide markers, preferably the antibody exhibits exclusive binding specificity for the intended target polynucleotide. The specificity of an antibody can be assessed according to any suitable protocol that is known to a person skilled in the art.

### 1. AMD

### 1.1 Abstract

Neovascular age-related macular degeneration (nAMD) has been described as an outbalanced inflammatory and proangiogenic retino-choroidal disease. Vitreous humor (VH) is the adjacent and attainable compartment which, due to the vicinity to the retina, might best represent changes of protein-based mediators of nAMD. The aim of this clinical-experimental study was to analyze the nAMD associated VH proteome of previously untreated patients and to correlate it to clinical diagnosis groups of nAMD. Capillary electrophoresis coupled online to mass spectrometry (CE-MS) as well as liquid chromatography coupled to tandem mass spectrometry (LC-MS/MS) were used to analyze VH of 108 nAMD patients and 24 controls with idiopathic floaters. A total of 101 different proteins with at least two unique peptides could be identified. Using a stringent statistical analysis with implementation of the closed test principle, we were able to identify four proteins that might be causatively linked to the pathogenesis of nAMD: Clusterin, opticin, pigment epithelium-derived factor and prostaglandin-H2 D-isomerase. Using independent samples, ROC-Area under the curve was determined proving the validity of the results: Clusterin 0.747, opticin 0.656, pigment epithelium-derived factor 0.514, prostaglandin-H2 D-isomerase 0.712. These identified proteins may serve as potential biomarkers or even targets of therapy for nAMD.

### 1.2 Significance

Within the meaning of the present invention, significant changes of the VH proteome in neovascular age-related macular degeneration (nAMD) were detected using one of the highest patient numbers in literature so far (108 nAMD, 24 controls). Four proteins with specific significance in the pathophysiology of nAMD were identified: Clusterin, opticin, pigment epithelium-derived factor and prostaglandin-H2 D-isomerase. On the basis of a stringent and exhaustive statistical analysis regarding clinical diagnosis groups, the confounding factor of expressed blood components into the vitreous cavity during nAMD could be excluded.

### 1.3 Introduction

Age-Related Macular Degeneration (AMD) is a complex, multifactorial disease and the major cause of central vision loss among the elderly in western, industrialized countries. Neovascular or wet AMD (nAMD) with choroidal neovascularization (CNV) is considered more aggressive, due to the rapid disease progression without therapy, than late dry AMD with geographic atrophy. It is estimated, that in Germany (representing the other western countries) the incidence of AMD, due to the ageing population, will almost double by the year 2030.

Intensive experimental and clinical research in recent years expanded our knowledge on the pathophysiology of nAMD. Today dysregulated complement system activation, oxidative stress and inflammatory processes are widely accepted as cofactors to proangiogenic activity mediated by VEGF. Nevertheless, there is still a lack of understanding of many pathophysiological components, especially on a molecular level.

The introduction of anti-VEGF agents marked a breakthrough in the therapy of nAMD. Nevertheless, a consistent dosing scheme is missing, the benefit of anti-VEGF agents for all patients is not predictable and long term anti-VEGF treatment may have severe side effects on ocular tissues. Therefore, research to evaluate potential future biomarkers or possible new targets of therapy is warranted and may overcome these obstacles.

Proteome analysis is one of the most advanced exploratory techniques for the discovery of new protein biomarkers of clinical significance. A number of studies have already employed proteome analysis for the study of age-related macular degeneration. Most of them used aqueous humor aspirates, were performed ex-vivo or used animal samples. Two publications, one a prior work of our team without consideration of clinical diagnosis groups, involved samples of VH, which might represent the changes in nAMD within the photoreceptor/retinal pigment epithelium/Bruch's membrane/choriocapillaris complex preferably due to the close anatomical proximity.

Within the meaning of the present invention, we directed our attention to proteins in the VH considering different clinical diagnosis groups of nAMD. The studied groups included choroidal neovascularization without signs of bleeding (CNVw/oB), with signs of bleeding (CNVwB) and hemorrhagic choroidal neovascularization (hCNV). To our best knowledge, this is the first VH-based proteomics study involving different nAMD types based on clinical presentation. The aim of the present study was to identify new biomarkers associated with the disease which may also serve as therapeutic targets for treatment of nAMD.

For the analysis of the VH protein content a combination of capillary electrophoresis coupled online to a time-of-flight mass spectrometer (CE-MS) and liquid chromatography coupled to tandem mass spectrometry (LC-MS/MS) was employed. CE-MS, due to its high reproducibility, is used for detection and semi-quantification of peptides, while LC-MS/MS was used for the determination of peptide amino acid sequences.

### 1.4 Material and Methods

### 1.4.1 Study Design

This was a retrospective, clinical-experimental study approved by the local institutional review board and adhering to the tenets of Declaration of Helsinki.

### 1.4.2 Patient Characterization

In this study a total of 132 undiluted vitreous body samples from previously untreated patients were analyzed: 108 from patients suffering from nAMD and 24 from patients with idiopathic floaters (controls). All Patients were treated at our clinic between April 2011 and October 2012. Exclusion criteria were previous intravitreal treatment with anti-VEGF, intraocular steroids, coagulation with photo laser, vitrectomy or previous intraocular operations, such as cataract operation at the included or not included eye in the last six months. Patients with systemic diabetes, nephropathy and uncontrolled hypertension were excluded as well as patients with compromising ocular conditions like diabetic retinopathy or uveitis. Furthermore, patients suffering complications like rubeosis iridis, vascularizations of the iridocorneal chamber, the papilla or in the retina perimeter were also considered ineligible. Personal data like age and gender were obtained for all participants as well as information regarding clinical subgroup of nAMD (CNVw/oB, CNVwB and hCNV), lens status and eye location. Sample analysis was conducted as described by Koss *et al.* (Koss MJ, Hoffmann J, Nguyen N, Pfister M, Mischak H, Mullen W, et al. Proteomics of vitreous humor of patients with exudative age-related macular degeneration. PLoS One. 2014;9:e96895.).

### 1.4.3 Sample Acquisition

nAMD patients were treated with a combination therapy containing of a core pars plana vitrectomy and intravitreal injections of triamcinolone (8 mg), bevacizumab (1.5 mg) and balanced salt solution (Koss MJ, Scholtz S, Haeussler-Sinangin Y, Singh P, Koch FH. Combined Intravitreal Pharmacosurgery in Patients with Occult Choroidal Neovascularization Secondary to Wet Age-Related Macular Degeneration. Ophthalmologica. 2009;224:72-8.). Samples were taken via single site core vitrectomy using Intrector technology (Koch FH, Koss MJ. Microincision vitrectomy procedure using Intrector technology. Arch Ophthalmol. 2011;129:1599-604.). 0.5 to 0.8 mL undiluted vitreous was aspirated before application of any drugs.

Patients with idiopathic floaters were served with the same surgical technique, substituting balanced salt solution. After collection all samples were immediately frozen and stored at-80°C until further use.

### 1.4.4 Tryptic Digestion

Samples were prepared as described in Koss et al., 2014 (Koss MJ, Hoffmann J, Nguyen N, Pfister M, Mischak H, Mullen W, et al. Proteomics of vitreous humor of patients with exudative age-related macular degeneration. PLoS One. 2014;9:e96895.). Briefly, ten µl of thawed sample was added to 90 µL 0.1% SDS, 20 mM DTT and 0.1 M Tris-HCl (pH=7.6). Subsequently the samples were sonicated at room temperature for 30 minutes followed by denaturation at 95°C for three minutes. In the next step the sample was incubated with 0.05 M iodacetamide, 8 M urea and 0.2 M Tris-HCl for 30 minutes at room temperature in the absence of light. Afterwards 50 mM ammonium bicarbonate buffer solution was added and the sample was applied to a NAP5 desalting column. Elution was performed using 50 M ammonium bicarbonate buffer solution following complete penetration of the sample into the column. Twenty µg sequencing grade modified trypsin (Promega, Mannheim, Germany) was added to 50 µl trypsin resuspension buffer (Promega, Mannheim, Germany). Two µl of this trypsin containing solution was added to the desalted sample. Tryptic digestion was completed overnight at 37°C. Subsequently, the sample was lyophilized. Hereafter, the sample was stored at 4°C and resuspended in distilled water shortly before analysis.

### 1.4.5 CE-MS Analysis

Capillary electrophoresis coupled to mass spectrometry (CE-MS) was performed as described by Theodorescu et al. (Theodorescu D, Wittke S, Ross MM, Walden M, Conaway M, Just I, et al. Discovery and validation of new protein biomarkers for urothelial cancer: a prospective analysis. Lancet Oncol. 2006;7:230-40). A P/ACE MDQ electrophoresis system (Beckman Coulter, Brea, CA) was coupled online to a micro-TOF MS (Bruker Daltonic, Leipzig, Germany). The applied sprayer (Agilent Technologies, Santa Clara, CA) interfacing the CE and MS was grounded and the interface potential was adjusted to -4.5 kV. Mass spectra were recorded for three seconds with signals at an m/z range of 350 to 3000. One fmol was the detection limit of the TOF-Analyzer.

### 1.4.6 Data Processing

CE-MS raw data was analyzed using MosaiquesVisu Version 2.1.0. (Mosaiques diagnostics GmbH, Hannover, Germany) (Neuhoff N, Kaiser T, Wittke S, Krebs R, Pitt A, Burchard A, et al. Mass spectrometry for the detection of differentially expressed proteins: a comparison of surface-enhanced laser desorption/ionization and capillary electrophoresis/mass spectrometry. Rapid Commun Mass Spectrom. 2004;18:149-56.). For mass deconvolution MosaiquesVisu uses conjugated mass detection and isotope identification. All signals with a signal-to-random noise ratio < 4 and charge = 1 were not taken into consideration.

For normalization of peptide CE-time and mass, 292 signals with a frequency > 35% could be determined and served as references. The signal intensities were ppm-normalized.

The normalized peptides defined by CE-time, mass and signal intensity were deposited, matched and annotated in a Microsoft SQL database. Peptides were considered identical when deviation of mass was < ±50 ppm for an 800 Da peptide. Mass deviation was gradually adjusted by an increase in size up to ±75 ppm for 15 kDa. Furthermore, peptides were only considered identical if the CE-migration time window did not exceed 2-5%, continuously increasing between 10 and 60 minutes.

### 1.4.7 MS/MS Sequencing

For MS/MS analysis nine randomly selected, lyophilized and trypsin-digested samples of vitreous humor were dissolved in 15 µl distilled water. Separation was carried out as described by Metzger et a/.[28] using a Dionex Ultimate 3000 Nano LC System (Dionex, Camberly, UK). After loading 5 µl onto a Dionex 0.1×20 mm 5 µm C18 nano trap column at a flowrate of 5 µl/min in 98% 0.1% formic acid and 2% acetonitrile, sample was eluted onto an Acclaim PepMap C18 nano column 75 µm×15 cm, 2 µm 100 A at a flow rate of 0.3 µl/min. The trap and nano flow column were maintained at 35°C. The samples were eluted with a gradient of solvent A: 98% 0.1% formic acid, 2% acetonitrile verses solvent B: 80% acetonitrile, 20% 0.1% formic acid starting at 1% B for 5 minutes rising to 20% B after 90 min and finally to 40% B after 120 min. The column was then washed and re-equilibrated prior to the next injection.

The Proxeon nano spray ESI source (Thermo Fisher Hemel UK) in positive ion mode was used for samples ionization. Ionization voltage was 2.6 kV, the capillary temperature was 200°C. The samples were analysed using an Orbitrap Velos FTMS (Thermo Finnigan, Bremen, Germany). The mass spectrometer was operated in MS/MS mode scanning from 380 to 2000 amu. The samples were analysed using both CID and HCD to obtain the maximum number of sequence identifications. The top 20 multiply charged ions were selected from each scan for MS/MS analysis using either CID or HCD at 40% collision energy. The resolution of ions in MS1 was 60,000 and 7,500 for mass fragmentation MS2. The searches were performed by the use of Proteome Discoverer (version 1.3, Thermo Fisher Scientific, Bremen, Germany) with the use of the SEQUEST algorithm and against the human, non-redundant IPI database (version 3.87, entry count: 91464). Trypsin was used as the enzyme while screening for proteins. Hydroxylated proline from collagen fragments and oxidation of methionine were accepted as variable modifications and carbamidomethylated cystein as fixed modification. A maximal mass deviation of 10 ppm for MS and 0.8 Da for MS/MS was accepted. The sequences were matched to the detected CE-MS data as described by Zürbig et al. (Zurbig P, Renfrow MB, Schiffer E, Novak J, Walden M, Wittke S, et al. Biomarker discovery by CE-MS enables sequence analysis via MS/MS with platform-independent separation. Electrophoresis. 2006;27:2111-25). Although in this matching procedure the LC retention time cannot be used, CE-MS data generate an additional parameter that can be used in this matching procedure, which is the charge of the peptides (at low pH, the condition in which peptides are analysed by CE-MS). Therefore, even when having identical or very close masses in the LC-MS/MS analysis, discrimination between peptides with similar masses can be performed on the basis of their charge. This is due to the fact that the number of basic and neutral polar amino acids of peptide sequences distinctly correlates with their CE-MS migration time/molecular weight coordinates. In nearly all cases this allows linking a unique LC-MS/MS peptide to a CE-MS peptide as shown previously. Protein identifications were accepted if they contained at least two identified peptides. The individual peptides were combined to protein. Relative abundance was calculated as the average of all normalized CE-MS peptide intensities for a given protein.

### 1.4.8 Statistical Analysis

Statistical analysis was performed using SPSS version 21.0. A P-value of α < 5.00E-02 was considered statistically significant.

### 1.4.9 Patient Characterization

Clinical and demographic characteristics of controls and each subgroup of nAMD (CNVw/oB, CNVwB, hCNV) were compared by using ANOVA and chi-square test where appropriate.

### 1.4.10 Proteomic Analysis

Candidate biomarkers were determined by examination of differences in signal intensity, as measured by CE-MS analysis, of the proteins between clinical subgroups of nAMD and control samples. Non-parametric tests were used as non-detection of signals below the signal-to-noise ratio in CE-MS produces data that is not normally distributed.

### 1.4.11 Selection of potential biomarker candidates

Procedure for statistical analysis regarding clinical diagnosis groups was based on the closed test principle [30]. Kruskal-Wallis test was carried out when comparing more than two independent groups. Comparison of two independent groups was done using Wilcoxon-Mann-Whitney test. Only proteins significant in the prior step were taken into account for following steps. Step 1: CNVw/oB vs. CNVwB vs. hCNV vs. controls (Kruskal-Wallis test); Step 2: CNVw/oB vs. CNVwB vs. controls, CNVw/oB vs. hCNV vs. controls, CNVwB vs. hCNV vs. controls, CNVw/oB vs. CNVwB vs. hCNV (Kruskal-Wallis test); Step 3: CNVw/oB vs controls, CNVwB vs. controls, hCNV vs. controls (Wilcoxon-Mann-Whitney test).

The Wilcoxon-Mann-Whitney test was used to analyze the differences between nAMD samples and controls to affirm the primary results of the closed testing procedure. Correction for multiple testing was performed as described by Benjamini and Hochberg (Benjamini Y, Hochberg Y. Controlling the false discovery rate: a practical and powerful approach to multiple testing. J Royal Stat Soc B (Methodological). 1995:125-33).

To investigate whether age difference is a confounding factor, Spearman's rank correlation coefficient was calculated as measure of statistical dependence between signal intensities of each potential biomarker candidate and age in each clinical diagnosis group of nAMD and control group.

### 1.4.12 Validation of biomarker candidates

For validation of biomarker candidates in nAMD, receiver operating characteristic (ROC) analysis was performed. Area under the curve (AUC) was determined.

### 1.5 Results and Discussion

### 1.5.1 Patient Characterization

A total of 132 samples of vitreous humor (nAMD: n=108; controls: n=24) were analyzed. Patients with nAMD were subdivided in following groups: CNVw/oB (n=54), CNVwB (n=33) and hCNV (n=21). Epidemiologic data and further information of all 132 participants are listed in **Table 1.**

**Table 1. Epidemiology**

| | **N**^{a} | **Age** in years (mean±SD) | **Sex** | | **Eye** | | **Lens** | |
|---|---|---|---|---|---|---|---|---|
| | | | Male | Female | Right | Left | Phakic | Pseudophakic |
| **nAMD*^{b}*** | 108 | 77.1±9.5 | 40 | 68 | 53 | 55 | 48 | 60 |
| ***CNVw*/*oB^{c}*** | 54 | 76±8.9 | 23 | 31 | 22 | 32 | 25 | 29 |
| ***CNVwB^{d}*** | 33 | 77.8±11.4 | 12 | 21 | 19 | 14 | 16 | 17 |
| ***hCNV^{e}*** | 21 | 79±7.6 | 5 | 16 | 12 | 9 | 7 | 14 |
| **Controls** | 24 | 62.7±11.7 | 11 | 13 | 18 | 6 | 12 | 12 |
| ^{a}N: Number of patients in each group, *^{b}*nAMD: Neovascular age-related macular degeneration, | | | | | | | | |
| *^{c}*CNVw/oB: Choroidal neovascularization without signs of bleeding, *^{d}*CNVwB: CNV with signs of bleeding, *^{e}*hCNV: hemorrhagic CNV | | | | | | | | |
| ANOVA was used for analysis of age; Chi-square test for analyses of sex and lens; P value of α<5.00E-02 was considered significant. Age P=3.88E-08; Sex P=4.07E-01; Lens P=6.63E-01 | | | | | | | | |

Differences between subgroups regarding age, sex and lens status were analyzed. A significant difference in patent age between controls and nAMD was observed (P=3.88E-08), which is discussed in a separate paragraph. No differences between the groups was observed based on gender (P=4.07E-01) and lens status (P=6.63E-01).

### 1.5.2 Proteomic Analysis

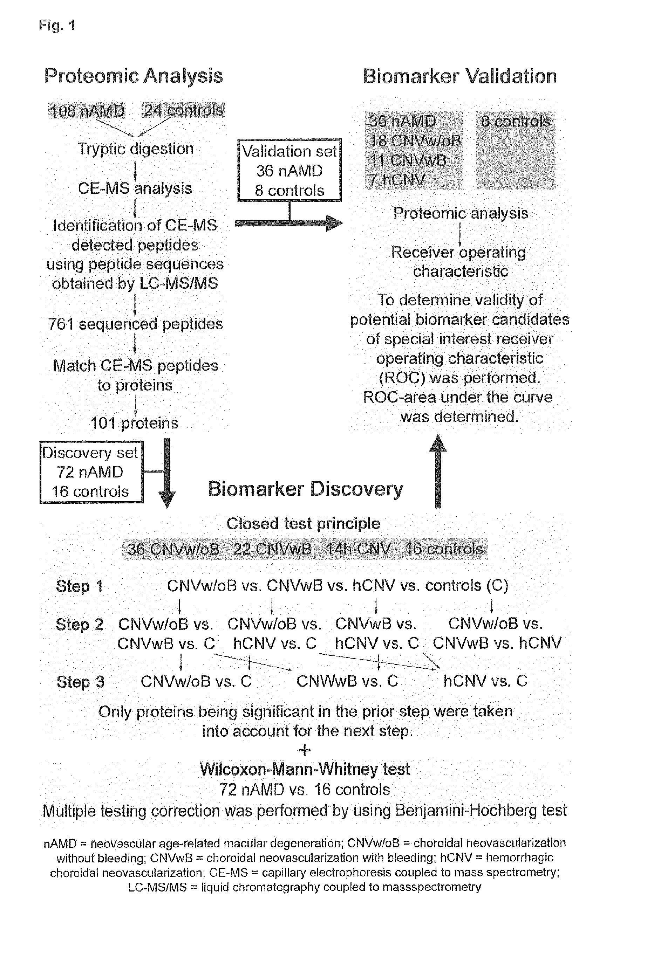

The study layout is depicted in **Figure1****.**

In total, 761 tryptic peptides detected by CE-MS could be identified using LC-MS/MS. The mass ranged between 800.4 and 3619.0 Da, CE migration time between 19.6 and 38.0 min. These 761 tryptic peptides corresponded to 101 different proteins.

For selection of potential biomarker candidates 36 CNVw/oB, 22 CNVwB, 14 hCNV and 16 control samples were used.

### 1.5.3 Selection of Potential Biomarker Candidates

Identification of potential biomarkers for nAMD in human vitreous is hampered by the presence of blood components. Therefore, it is difficult to determine whether a protein displaying differential abundance in the presence of bleeding has a functional pathophysiological role in nAMD. Hence, we performed the closed test principle on clinical subgroups of nAMD patients and controls, with the objective of filtering the influence of bleeding. As these different groups represent different degrees of bleeding (none (CNVw/oB), signs (CNVwB) of bleeding and massive bleeding (hCNV), proteins being significant in every comparison of each subgroup and controls should be independent from the presence of bleeding and are linked to the pathophysiology of nAMD. Proteins meeting these requirements were selected as potential biomarker candidates.

Using the closed test principle, seven proteins displayed a significantly different abundance in CNVw/oB samples compared to controls; nine and six proteins were different between CNVwB and hCNV compared to controls, respectively. Four proteins overlapped in these 3 comparisons and were therefore selected as candidate biomarkers: clusterin, opticin, pigment epithelium-derived factor (PEDF) and prostaglandin-H2 D-isomerase (PH2D). **Table** 2 shows results of the statistical analysis regarding clinical diagnosis groups:

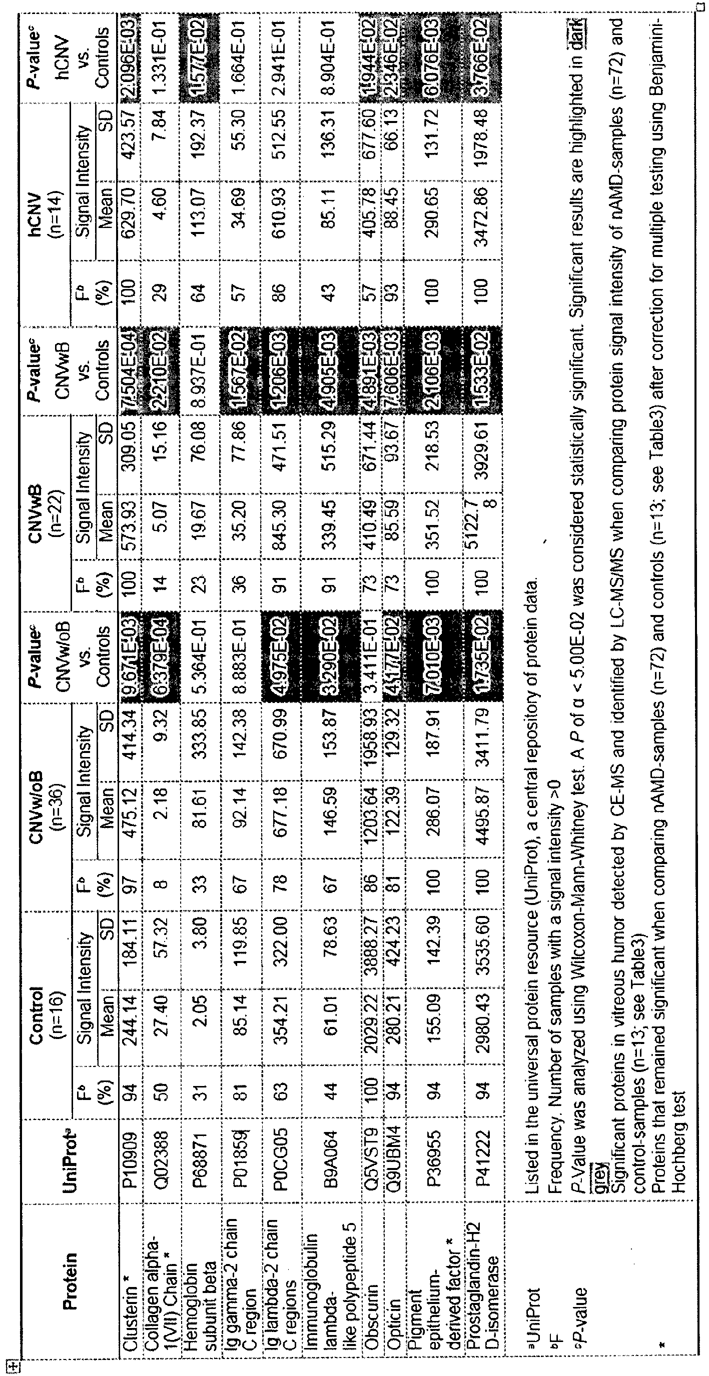

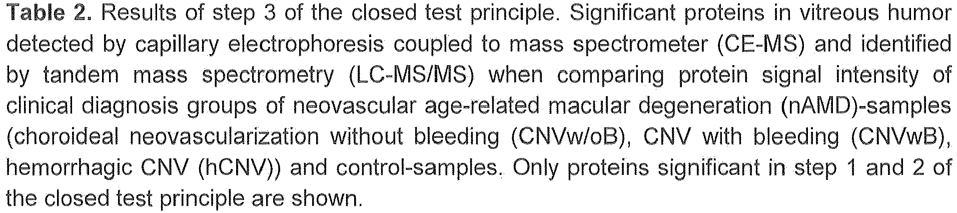

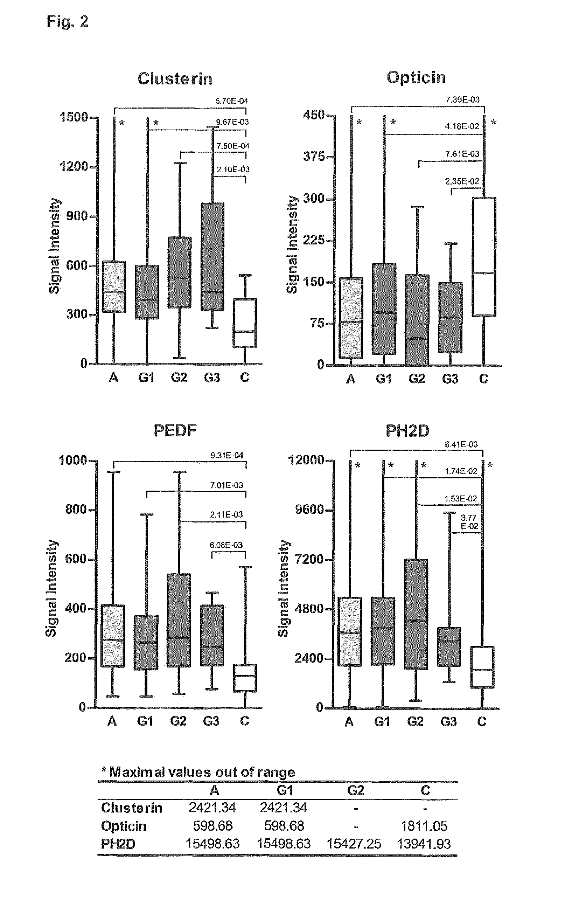

Distribution of signal intensities of the four biomarker candidates in the discovery cohort and corresponding p-values are shown in **Figure 2****.**

In the comparison CNVw/oB vs. CNVwB vs. hCNV none of these four protein intensities showed significant up- or down regulation (P=2.16E-01 to 5.43E-01), which is a confirmation of our hypothesis that these proteins are linked to the pathophysiology of nAMD and are independent from the degree of bleeding.

Furthermore, comparison of protein data between all, irrespective of the subgroups, and control samples was performed resulting in 13 significant proteins (P=5.70E-04 to 4.84E-02, **Table 3).**

**Table 3. Significant proteins in vitreous humor detected by capillary electrophoresis coupled to mass spectrometer (CE-MS) and identified by tandem mass spectrometry (LC-MS/MS) when comparing protein signal intensity of neovascular age-related macular degeneration (nAMD)-samples and controls.**

| **Protein** | | **UniProt*^{a}*** | **nAMD** (n=72) | | | | | | **Control** (n=16) | | | | ***P*-value*^{e}*** nAMD vs. Controls | **Benjamini-Hochberg** |
|---|---|---|---|---|---|---|---|---|---|---|---|---|---|---|
| | | | F*^{b}* (%) | IF*^{c}* | Coverage*^{d}* | Signal Intensity | | F*^{b}* (%) | IF*^{c}* | Coverage*^{d}* | Signal Intensity | | | |
| | | | | | | Mean | SD | | | | Mean | SD | | |
| Upregulation | | | | | | | | | | | | | | |
| Clusterin * | | P10909 | 99 | 12 | 0.33 | 535.37 | 387.02 | 94 | 11 | 0.28 | 244.14 | 184.11 | 5.70E-04 | 3.14E-02 |
| Pigment epithelium-derived factor * | | P36955 | 100 | 15 | 0.36 | 306.96 | 188.67 | 94 | 14 | 0.33 | 155.09 | 142.39 | 9.31E-04 | 3.14E-02 |
| Prostaglandin-H2 D-isomerase | | P41222 | 100 | 4 | 0.21 | 4488.51 | 3369.02 | 94 | 4 | 0.21 | 2980.43 | 3535.60 | 6.41E-03 | 1.49E-01 |
| Serotransferrin | | P02787 | 100 | 42 | 0.56 | 543.69 | 365.61 | 100 | 33 | 0.46 | 329.85 | 226.58 | 1.00E-02 | 1.69E-01 |
| Ig lambda-2 chain C regions | | P0CG05 | 83 | 3 | 0.42 | 715.67 | 586.45 | 63 | 2 | 0.27 | 354.21 | 322.00 | 1.17E-02 | 1.69E-01 |
| Immunoglobulin lambda-like polypeptidelide 5 | | B9A064 | 69 | 3 | 0.20 | 193.56 | 321.93 | 44 | 2 | 0.13 | 61.01 | 78.63 | 2.62E-02 | 2.82E-01 |
| Ig gamma-1 chain C region | | P01857 | 100 | 9 | 0.31 | 877.04 | 575.50 | 94 | 8 | 0.25 | 560.07 | 410.39 | 2.81E-02 | 2.82E-01 |
| Titin | | Q8WZ42 | 49 | 6 | < 0.01 | 19.41 | 49.47 | 19 | 4 | < 0.01 | 3.55 | 9.38 | 3.89E-02 | 3.27E-01 |
| Ig kappa chain C region | | P01834 | 88 | 5 | 0.80 | 190.52 | 258.58 | 63 | 3 | 0.63 | 89.31 | 105.87 | 4.84E-02 | 3.76E-01 |
| | | | | | | | | | | | | | | |
| Downregulation | | | | | | | | | | | | | | |
| Collagen alpha-1(VII) chain * | | Q02388 | 14 | 3 | 0.02 | 3.53 | 11.13 | 50 | 3 | 0.02 | 27.40 | 57.32 | 8.65E-04 | 3.14E-02 |
| Opticin | | Q9UBM4 | 81 | 5 | 0.16 | 104.55 | 109.38 | 94 | 5 | 0.18 | 280.21 | 424.23 | 7.39E-03 | 1.49E-01 |
| Alpha-crystallin A chain | | P02489 | 7 | 3 | 0.19 | 2.67 | 10.94 | 25 | 2 | 0.14 | 235.88 | 806.19 | 2.49E-02 | 2.82E-01 |
| Obscurin | | Q5VST9 | 76 | 2 | < 0.01 | 806.15 | 1506.42 | 100 | 2 | < 0.01 | 2029.22 | 3888.27 | 3.07E-02 | 2.82E-01 |
| | | | | | | | | | | | | | | |
| ^{a}UniProt | Listed in the universal protein resource (UniProt), a central repository of protein data. | | | | | | | | | | | | | |
| *^{b}*F | Frequency. Number of samples with a signal intensity >0 | | | | | | | | | | | | | |
| *^{c}*IF | Identified Peptides. Number of peptides observed by CE-MS analysis and identified by LC-MS/MS for each protein | | | | | | | | | | | | | |
| *^{d}*coverage | Percentage of peptide coverage of the protein sequence | | | | | | | | | | | | | |
| *^{e}P*-value | P-Value was analyzed using Wilcoxon-Mann-Whitney test. A P of α < 5.00E-02 was considered statistically significant | | | | | | | | | | | | | |
| * | Proteins that remained significant after performing multiple testing correction, analyzed using Benjamini-Hochberg test for false discovery rate. An adjusted P-Value of α<5.00E-02 was considered statistically significant. | | | | | | | | | | | | | |

**Table 3.** Significant proteins in vitreous humor detected by capillary electrophoresis coupled to mass spectrometer (CE-MS) and identified by tandem mass spectrometry (LC-MS/MS) when comparing protein signal intensity of neovascular age-related macular degeneration (nAMD)-samples and controls.

Three proteins, including clusterin and PEDF, remained significant after correction for multiple testing (see *; **Table 2** and **3).** The third protein, collagen alpha-1(VII) chain, showed no significance in the closed test principle and therefore was not considered as potential biomarker candidate. Opticin and PH2D were also significant, but barely missed the significance level after correction for multiple testing. Thus, our assumption that the four proteins being significant in the closed test principle may play a crucial role within the pathophysiology of nAMD is further affirmed.

Spearman's rank correlation coefficient of signal intensities of clusterin, opticin, PEDF, PH2D and age in the individual groups showed no statistical dependence (p=-0.499 to 0.572; P=1.80E-02 to 9.23E-01). The PEDF correlation to age showed significance in two comparisons, but in an opposite manner (positive in the control and negative in the hCNV group). We therefore concluded that for PEDF as well, no sufficient dependence on age exists. Given these results, age has no bearing on our findings. For coefficients and P values see Supplemental **Table S3.**

**Table S3:**

| | | | **Clusterin** | **Opticin** | **PEDF** | **PH2D** |
|---|---|---|---|---|---|---|
| | ***CNVw*/*oB*** | Spearman ρ | 0.251 | -0.146 | 0.107 | -0.118 |
| | | *P* value | 0.139 | 0.394 | 0.535 | 0.492 |
| | ***CNVwB*** | Spearman ρ | -0.300 | -0.063 | -0.499 | -0.141 |
| **Age** | | *P* value | 0.175 | 0.780 | 0.018 | 0.532 |
| | ***hCNV*** | Spearman ρ | -0.260 | 0.242 | -0.029 | 0.117 |
| | | *P* value | 0.370 | 0.404 | 0.923 | 0.691 |
| | ***control*** | Spearman ρ | 0.442 | -0.102 | 0.572 | -0.096 |
| | | P value | 0.087 | 0.707 | 0.021 | 0.724 |

Table S3. Spearman's rank correlation coefficients (Spearman p) and corresponding P-values regarding age and signal intensities of clusterin, opticin, pigment epithelium-derived factor (PEDF), prostaglandin-H2 D-isomerase (PH2D). Given for control group and each subgroup of age-related macular degeneration: Choroidal neovascularization without bleeding (CNVw/oB), with bleeding (CNVwB) and hemorrhagic choroidal neovascularization (hCNV).

### 1.5.4 Potential Biomarker Candidates

Clusterin is an extracellular chaperone encoded by a single nine-exon gene on chromosome 8 and consists of two subunits. From an evolutionary point of view clusterin has possibly a high biological significance, as the gene is highly conserved among species and expressed in nearly all human tissues representing a multifunctional role. In recent years clusterin has been proposed as a key marker in cancer research. Nevertheless, it was also identified in aqueous humor aspirates and in aqueous humor exosomes of patients with AMD and in retinal drusen. Interestingly, blood levels of clusterin are associated with atrophy of the entorhinal cortex and elevated levels may predict increased fibrillar amyloid-β burden in the temporal lobe in Alzheimer's disease, for which clusterin seems to be a marker of disease severity. In addition, antisense oligonucleotides to clusterin were shown to inhibit angiogenesis *in vitro* and induce apoptosis in human umbilical vein endothelial cells. Our findings of increased expression in nAMD patients indicate for the first time that clusterin in human vitreous is associated with this disease suggesting, alongside additional literature evidence, that this is a biochemical sign of a neurodegenerative disease.

Opticin is a member of the small leucin rich repeat protein family and is produced by the non-pigmented ciliary epithelium. Interestingly, as an extracellular matrix protein it is secreted into the vitreous cavity at an unusually high rate in adults. Using immunostaining, opticin was found to be located, alongside various ocular tissues, especially in basal and cortical vitreous and adjacent basement membranes, like the internal limiting membrane, suggesting a possible role in vitreoretinal adhesion. It was also demonstrated that opticin knock-out mice produce significantly more preretinal neovascularization than wild-type mice. The intravitreal application of excess opticin inhibited neovascularization in wild-type mice, but lack of opticin did not affect physiological vascular development. Furthermore, the opticin gene is located on chromosome 1 within an AMD susceptibility locus. Our findings, decreased expression in nAMD group suggest opticin is a factor in choroidal neovascularization, but additional information regarding the association of opticin and nAMD is still required.

Pigment epithelium-derived factor (PEDF) is a potent inhibitor of angiogenesis with protective effects on various neural cells, such as photoreceptors. PEDF is secreted by retinal pigment epithelium cells and photoreceptors into the interphotoreceptor matrix and can be found in rods, cones, cells of the ganglion cell layer and of the inner nuclear layer. Increased levels were described in aqueous humor aspirates of patients with CNV. A vascular endothelial growth factor (VEGF) dependent upregulation of PEDF in CNV was suggested in literature. Hence, we also observed PEDF upregulation in VH of patients with nAMD and we therefore consider upregulation of PEDF as a positive control validating our VH proteome-based screen in nAMD patients.

Prostaglandin H2 D-isomerase (PH2D) is an enzyme that converts prostaglandin H2 to prostaglandin D2. Prostaglandin D2 is elevated in inflammation and seems to be associated with pathological symptoms in allergies. Furthermore, it is involved in the regulation of sleep, the sensation of pain and the development of male reproductive organs. PH2D was shown to be secreted into the interphotoreceptor matrix by the retinal pigment epithelium in adult rats and is significantly increased in the vitreous body of patients with proliferative diabetic retinopathy compared to patients suffering from macular hole. Previously, our group found elevated PH2D levels in vitreous of nAMD patients compared to controls in a smaller scale study (Koss MJ, Hoffmann J, Nguyen N, Pfister M, Mischak H, Mullen W, et al. Proteomics of vitreous humor of patients with exudative age-related macular degeneration. PLoS One. 2014;9:e96895). We are now able to confirm this finding. PH2D seems to be constantly traceable in VH of nAMD patients and its role in CNV appears reasonable to be further investigated.

### 1.5.5 Gene Ontology (GO) and Pathway Analysis

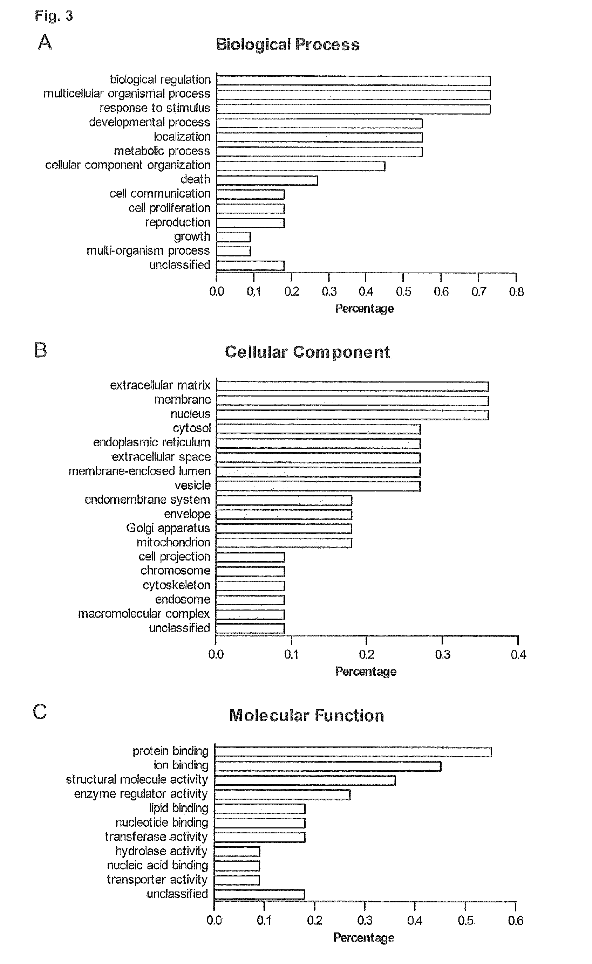

Thirteen proteins that were significant in comparison of nAMD and control samples (see **Table 3)** were analyzed via WebGestalt (WEB-based GEne SeT AnaLysis Toolkit; http://bioinfo.vaderbilt.edu/webgestalt/) (Zhang B, Kirov S, Snoddy J. WebGestalt: an integrated system for exploring gene sets in various biological contexts. Nucleic acids research. 2005;33:W741-8.). GO-Terms were allocated to proteins in three main categories. Major biological processes were assigned as biological regulation, multicellular organismal process and response to stimulus **(****Figure 3A****).**

Major cellular components were assigned as extracellular matrix, membrane and nucleus **(****Figure 3B****).** Major molecular functions were assigned as protein binding and ion binding as well as structural molecule activity **(****Figure 3C****).**

In order to reveal canonical pathways that are potentially involved in the pathogenesis of nAMD, we used pathway commons analysis tool via WebGestalt. Primary pathways associated with nAMD were assigned as responses to elevated platelet cytosolic Ca2+, platelet degranulation and platelet activation, signaling and aggregation. Therefore, based on this enrichment analysis we confirm dysregulated complement system activation as a widely accepted factor in the pathophysiology of nAMD.

### 1.5.6 Validation of Selected Potential Biomarker Candidates

Clusterin, opticin, PEDF and PH2D were listed significant in every subgroup analysis comparison **(Table 2).** When comparing all nAMD (irrespective of the subgrouping) and control samples, clusterin and opticin remained significant after correction for multiple testing (see *; **Table 2 and 3).** Therefore, these proteins were selected for validation of candidate biomarkers.

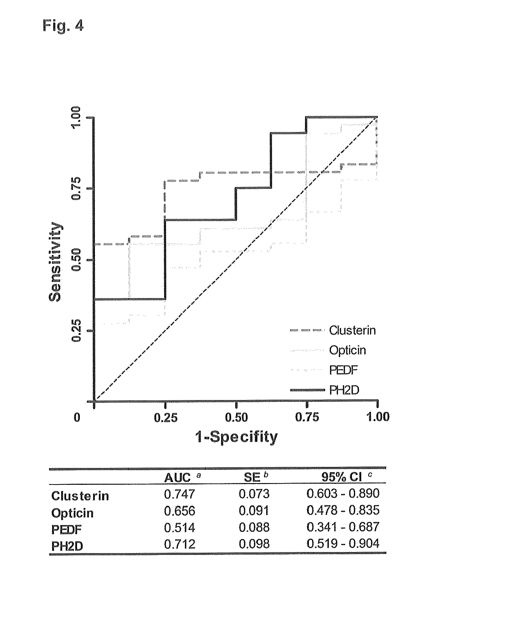

Receiver operating characteristic analysis was performed using randomly selected, independent samples to prove statistical significance. Eight control samples and 36 nAMD samples (18 CNVw/oB, eleven CNVwB, seven hCNV) not used for the biomarker discovery were used **(****Figure 1****).** Results of ROC analysis are shown in **Figure 4****.** Area under the curve was 0.747 for Clusterin, 0.656 for Opticin, 0.514 for PEDF and 0.712 for PH2D.

### 1.5.7 Advantages of the Study

This is the first proteomic study on nAMD not differentiating simply between cases and controls, but performing a clinical graduation of samples to satisfy the heterogeneity of this complex disease. Furthermore, we are able to present results based on one of the highest patient numbers in literature, with 132 participants in total (Proteomic analysis: 72 nAMD, 16 controls; ROC-analysis (validation): 36 nAMD, 8 controls). By using samples of VH in contrast to samples of aqueous humor we are able to show changes of the proteome in nAMD in more detail.

### 1.6 Conclusion

In this retrospective, clinical-experimental study we applied a reproducible proteomics detection method in a high number of undiluted VH samples of patients with nAMD. Our findings were thoroughly statistically evaluated and resulted in the selection of four potential biomarker candidates being associated with the pathophysiology of nAMD: Clusterin, opticin, PEDF and PH2D. Clusterin and PH2D, with AUC>0.7, were validated in an independent set of samples. Future studies are needed to validate the remaining two proteins, which could be helpful with regard to future diagnostic or therapeutic approaches in neovascular age-related macular degeneration.

### 2. RVO

### 2.1 Abstract

### 2.1.1 Purpose

To analyze the protein profile of human vitreous of untreated patients with retinal vein occlusion (RVO)

### 2.1.2 Methods

Sixty-eight vitreous humor (VH) samples (44 from patients with treatment naïve RVO, 24 controls with idiopathic floaters) were analyzed in this retrospective case series using capillary electrophoresis coupled to mass spectrometer and tandem mass spectrometry. To define potential candidate protein markers of RVO, proteomic analysis was performed on RVO patients (n=30) and compared with controls (n=16). To determine validity of potential biomarker candidates in RVO, receiver operating characteristic (ROC) was performed by using proteome data of independent RVO (n=14) and control samples (n=8).

### 2.1.3 Results

Ninety-six different proteins (755 tryptic peptides) could be identified. Seventeen proteins were found to be significant when comparing RVO and control samples (P=1.43E-05 to 4.48E-02). Five proteins (Clusterin, Complement C3, Ig lambda-like polypeptide 5 (IGLL5), Opticin and Vitronectin), remained significant after using correction for multiple testing. These five proteins were also detected significant when comparing subgroups of RVO (central RVO, hemi-central RVO, branch RVO) to controls. Using independent samples ROC-Area under the curve was determined proving the validity of the results: Clusterin 0.884, Complement C3 0.955, IGLL5 1.000, Opticin 0.741, Vitronectin 0.786.

### 2.1.4 Conclusion

The results of the study reveal that the proteomic composition of VH differed significantly between the patients with RVO and the controls. The proteins identified may serve as potential biomarkers for pathogenesis induced by RVO.

### 2.2 Introduction

Retinal vein occlusion (RVO) is the second most common cause of vision loss in older patients due to retinal vascular disease after diabetic retinopathy. Despite major achievements in diagnosis and treatment perspectives based on spectral OCT, there is still limited understanding of the pathophysiology of RVO. A number of cytokines including vascular endothelial growth factor (VEGF) and interleukin-6 (IL-6) have been shown to be associated to RVO. However these cytokines display high interindividual variations and their concentrations often do not correspond to the associated clinical RVO phenotype. Even in non-diseased eyes, reproducible vitreous protein profiling is quite complicated as the aging vitreous incorporates different stages of liquefaction and syneresis, which is aggravated after treatment with intravitreal injections. Therefore, many aspects of the molecular mechanism of RVO remain poorly described.

Proteomics is a promising approach allowing the analysis of the total protein content of a sample and in combination with robust statistical analyses can lead to the identification of proteins associated to specific diseases in ophthalmology. In the context of RVO the current proteomic data are incomplete. To the best of our knowledge, only one proteome study in RVO exists.

Therefore, to potentially improve upon the insight in the pathophysiology of RVO we performed high-resolution proteome analysis of vitreous humor (VH) of a high number of patients with RVO (n=44).

### 2.3 Materials and Methods

### 2.3.1 Study Design

This was a clinical-experimental study. The study was approved by the local ethics committee and adhered to the tenets of the Declaration of Helsinki. All participants signed an informed consent form.

### 2.3.2 Patient Characterization

In our retrospective case series we analyzed a total of 68 undiluted samples from previously untreated (any intravitreal drug application) patients for VH proteome identification and quantitative verification; 44 from RVO patients and 24 randomly chosen controls with idiopathic floaters. All patients consulted our Department of Ophthalmology between April and August 2012. Patients suffering neovascular complications such as rubeosis irids, vascularisations of iridocorneal chamber, neovascularisations of the papilla or in the retina perimeter were considered ineligible for this study as well as patients with vitreo-macular traction or patients previously treated with intravitreal anti-VEGF, intraocular steroids, coagulation with photo laser, vitrectomy or previous intraocular operations, such as cataract operation at the included or not included eye in the last six months. Patients with diabetic retinopathy, uveitis, glaucoma or other compromising ocular conditions were also excluded. For all participants personal data, such as gender and patient-age, were collected as well as information regarding type of RVO (central-RVO (CRVO), hemi-central RVO (H-CRVO), BRVO), lens status, averaged and central retinal thickness, posterior attachment of the vitreous body and presence of retinal cysts with spectral domain OCT (SD-OCT; Topcon 200, Tokyo, Japan).

### 2.3.3 Tryptic Digestion of Vitreous Humor

Sampling of VH, sample preparation and analysis was conducted as described in Koss *et al.* 2014 (Koss MJ, Hoffmann J, Nguyen N, et al. Proteomics of vitreous humor of patients with exudative age-related macular degeneration. PLoS One 2014;9(5):e96895.). In short: 10 µL of the thawed samples were diluted with 1:10 with 0.1 % SDS, 20 mM DTT, and 0.1 M Tris-HCl (pH=7.6). Samples were sonicated at room temperature for 30 minutes. Afterwards, the samples were denaturated at 95°C for 3 min and then were incubated for 30 min at room temperature in the absence of light with 0.05 M lodoacetamide. Eight M urea, 0.2 M Tris-HCl and 50 mM ammonium bicarbonate buffer solution was added and the samples were applied to NAP-5 columns equilibrated in 50 mM ammonium bicarbonate buffer solution. Trypsin solution was added to the desalted samples. Trypsin digestion was carried out overnight at a temperature of 37°C. Subsequently, the samples were lyophilized. Afterwards the samples were stored at 4°C and were resuspended with distilled water shortly before mass spectrometry analysis.

### 2.3.4 CE-MS Analysis

Capillary electrophoresis coupled to mass spectrometer (CE-MS) analysis was carried out as described by Theodorescu *et al.* 2006 (Theodorescu D, Wittke S, Ross MM, et al. Discovery and validation of new protein biomarkers for urothelial cancer: a prospective analysis. Lancet Oncol 2006;7(3):230-240.). A P/ACE MDQ capillary electrophoresis system (Beckman Coulter, Brea, CA) being linked online to a micro-TOF MS (Bruker Daltonik, Leipzig, Germany) was used. The sprayer (Agilent Technologies, Santa Clara, CA) interfacing the CE and MS was grounded. Interface potential was adjusted to -4.5 kV. Mass spectra were recorded for three seconds with signals at an m/z range between 350 and 3000. The detection limit of the TOF-Analyzer was 1 fmol (Theodorescu D, Wittke S, Ross MM, et al. Discovery and validation of new protein biomarkers for urothelial cancer: a prospective analysis. Lancet Oncol 2006;7(3):230-240.).

### 2.3.5 Data Processing Of CE-MS Analysis

Analysis of raw CE-MS data was carried out via MosaiquesVisu version 2.1.0 (mosaigues diagnostics GmbH, Hannover, Germany) which uses isotope identification and conjugated mass detection for mass deconvolution (Neuhoff N, Kaiser T, Wittke S, et al. Mass spectrometry for the detection of differentially expressed proteins: a comparison of surface-enhanced laser desorption/ionization and capillary electrophoresis/mass spectrometry. Rapid Commun Mass Spectrom 2004;18(2):149-156). Signals with a signal-to-random noise ratio >4 and charge >1 were used. Mosaiques-Visu employs a probabilistic clustering algorithm and uses both, isotopic distribution and conjugated masses for charge-state determination of peptides/proteins. The resulting peak list characterizes each polypeptide by its molecular mass, CE-migration time, and ion signal intensity (amplitude) value. Mass spectral ion peaks from the same molecule at different charge states were deconvoluted into a single mass. To minimize effects of biological and analytical variability between the different lots, a normalization of retention time, signal intensity and mass was performed. In total, 292 signals for mass and CE-time with a frequency ≥35% could be determined that served as reference signals for normalization of peptide CE-time using local and mass using linear regression. The signal intensities were relative normalized according to the whole signal intensity in individual analysis (ppm normalization).

All normalized peptides were deposited, matched, and annotated in a Microsoft SQL database, allowing further analysis and comparison of multiple samples (Siwy J, Mullen W, Golovko I, Franke J,Zurbig P. Human urinary peptide database for multiple disease biomarker discovery. Proteomics Clin Appl 2011;5(5-6):367-374). Peptides were considered identical when deviation of mass was <±50 ppm (parts per million) for an 800 Da peptide, respectively <±75 ppm for a 15 kDa peptide. Peptides were considered identical if the CE-migration time window did not exceed 2-5%, continuously increasing between 10 and 60 min.

### 2.3.6 Peptide Sequencing

Nine lyophilized, tryptic-digested randomly selected vitreous samples were dissolved in 15 µL distilled water for MS/MS analysis. Separation was carried out via Dionex Ultimate 3000 RSLS nano flow system (Dionex, Camberly UK) as described by Metzger *et al.* (Metzger J, Negm AA, Plentz RR, et al. Urine proteomic analysis differentiates cholangiocarcinoma from primary sclerosing cholangitis and other benign biliary disorders. Gut 2013;62(1):122-130.). After loading 5 µl onto a Dionex 0.1×20 mm 5 µm C18 nano trap column at a flowrate of 5 µl/min in 98% 0.1% formic acid and 2% acetonitrile, sample was eluted onto an Acclaim PepMap C18 nano column 75 µm×15 cm, 2 µm 100 Å at a flow rate of 0.3 µl/min. The trap and nano flow column were maintained at 35°C. The samples were eluted with a gradient of solvent A: 98% 0.1% formic acid, 2% acetonitrile verses solvent B: 80% acetonitrile, 20% 0.1% formic acid starting at 1% B for 5 minutes rising to 20% B after 90 min and finally to 40% B after 120 min. Subsequently, the column was washed and re-equilibrated prior to the next injection. The Proxeon nano spray ESI source (Thermo Fisher Hemel UK) in positive ion mode was used for samples ionisation. Ionization voltage was 2.6 kV, the capillary temperature was 200°C. The samples were analysed using an Orbitrap Velos FTMS (Thermo Finnigan, Bremen, Germany). The mass spectrometer was operated in MS/MS mode scanning from 380 to 2000 amu. The samples were analysed using both CID and HCD to obtain the maximum number of sequence identifications. The top 20 multiply charged ions were selected from each scan for MS/MS analysis using either CID or HCD at 40% collision energy. The resolution of ions in MS1 was 60,000 and 7,500 for mass fragmentation MS2. The MS data and the human, non-redundant database IPI were matched via SEQUEST software. Trypsin was used as the enzyme while screening for proteins. Hydroxylated proline from collagen fragments and oxidation of methionine were accepted as variable modifications and carbamidomethylated cysteine as fixed modification. A maximal mass deviation of 10 ppm for MS and 0.8 Da for MS/MS was accepted.

The sequences were matched to the detected CE-MS data according to Zurbig *et al.* (Zurbig P, Renfrow MB, Schiffer E, et al. Biomarker discovery by CE-MS enables sequence analysis via MS/MS with platform-independent separation. Electrophoresis 2006;27(11):2111-2125.). Charge of the peptides was used in this matching procedure instead of the LC retention time. On the basis of their charge, discrimination between peptides with similar masses can be performed even when having identical or very close masses in the liquid chromatography coupled with tandem mass spectrometry (LC-MS/MS) analysis. This is due to the fact that the number of basic and neutral polar amino acids of peptide sequences distinctly correlates with their CE-MS migration time/molecular weight coordinates. This enables linking a unique LC-MS/MS peptide to a CE-MS peptide in nearly all cases (Zurbig P, Renfrow MB, Schiffer E, et al. Biomarker discovery by CE-MS enables sequence analysis via MS/MS with platform-independent separation. Electrophoresis 2006;27(11):2111-2125.).

CE-MS peptides with sequencing information were combined for each protein. Proteins were accepted when being represented by a minimum of two peptides. Protein abundance was calculated as the average of all normalized CE-MS peptide intensities for the given protein.

### 2.3.7 Statistical Methods

SPSS version 21.0 was used for statistical analysis. A P of α<5.00E-02 was considered statistically significant.

### 2.3.8 Descriptive Statistics

For the descriptive data analysis, mean± standard deviation (SD), median values, and minimal and maximal values were calculated.

### 2.3.9 Patient Characterization

Clinical and demographic characteristics of control patients and each subgroup of RVO (CRVO, H-CRVO, BRVO) was compared by using the Kruskal Wallis test where appropriate.

### 2.3.10 Proteomic Analysis

Candidate RVO biomarkers were defined by examination of differences in signal intensity of the proteins between the RVO-patients and the controls. Mean CE-MS based protein signal intensity was used as a measure for relative abundance. Mann-Whitney test was used for analysis. Multiple hypotheses testing correction was performed by using the Benjamini-Hochberg test for false discovery rate (Benjamini Y,Hochberg Y. Controlling the false discovery rate: a practical and powerful approach to multiple testing. Journal of the Royal Statistical Society, Series B: Methodological 1995;57(289-300.).

### 2.3.11 Subgroup Analysis

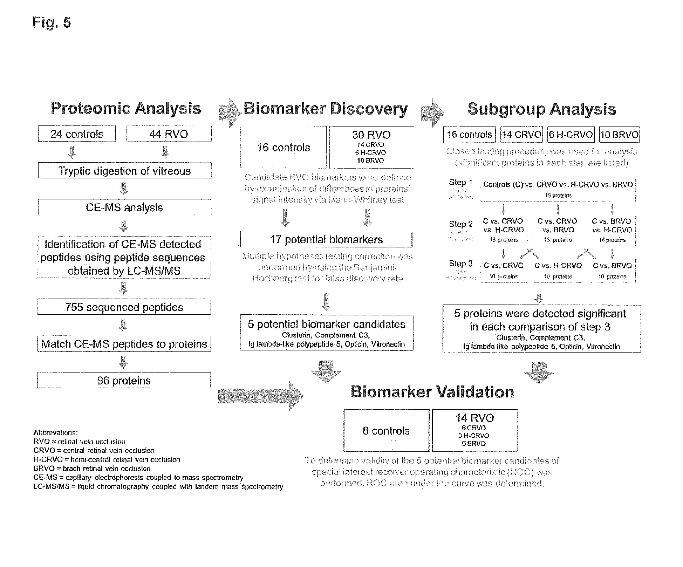

Patients with RVO were subdivided in following subgroups: CRVO, H-CRVO, BRVO. The closed testing procedure was used for subgroup analysis (Marcus R, Peritz E,Gabriel KR. On closed testing procedures with special reference to ordered analysis of variance. Biometrika 1976;63(655-660). For detailed information see **Figure 5****.**

### 2.3.12 Influence Of Past Time Period since Suffering RVO on Biomarker Discovery

To analyze the influence of past time period since suffering RVO we compared protein signal intensity between fresh (patients suffered RVO <8 weeks ago) and old RVO-samples (patients suffered RVO ≥8 weeks ago) *versus* controls. The Mann-Whitney test was used for analysis.

### 2.3.13 Verification of Selected Potential Biomarker Candidates

To determine validity of potential biomarker candidates in RVO, receiver operating characteristic (ROC) was performed by using data of randomly selected, independent control and RVO samples not being used in proteomic analysis and biomarker definitions. Area under the curve (AUC) was determined. To verify validity of the results of the ROC analysis, mean ± SD of AUC of 50 analyses using one third of all control samples and one third of all RVO-samples, was determined. The used samples were randomly selected for each of the 50 analyses.

### 2.4 Results

### 2.4.1 Patient Characterization

A total of 44 RVO samples and 24 controls with idiopathic floaters were analyzed. Patients with RVO were additionally subdivided in the following subgroups: CRVO (n=20), H-CRVO (n=9), BRVO (n=15). Epidemiologic data and further patient information such as fresh/old RVO, lens status, retinal thickness, attachment of the vitreous body, and presence of retinal cysts are listed in **Table 4.** No differences between subgroups regarding patient-age and average or center retinal thickness were observed (P=0.160 to 0.548).

**Table 4. Epidemiology**

| | **CRVO** | **Hemi-CRVO** | **BRVO** | **Control** |
|---|---|---|---|---|
| **N** | 20 | 9 | 15 | 24 |
| **Fresh/Old** | 14/6 | 7/2 | 11/4 | - |
| **Female/Male** | 8/12 | 4/5 | 10/5 | 13/11 |
| **Age in years (mean ± SD)** | 67.6 ± 14.3* | 69.2 ± 14.2* | 68.4 ± 12.2* | 62.7 ± 11.7* |
| **Phakic/Pseudophakic** | 16/4 | 8/1 | 13/2 | 12/12 |
| **Average Thickness in µm (mean ± SD)** | 376.3 ± 82.8* | 351.33 ± 44,2* | 351.5 ± 69.6* | - |
| **Center Thickness in µm (mean ± SD)** | 513.8 ± 196.9* | 443.4 ± 142.2* | 462.0 ± 149.9* | - |
| **Posterior Vitreous attached/ detached/unrecognizable** | 6/14/0 | 3/5/1 | 0/14/1 | - |
| **Cysts yes/no** | 15/5 | 8/1 | 13/2 | - |
| * Kruskal Wallis test was used for analyses; P-Value of α<5.00E-02 was considered significant. No significant difference was tested: Age P=3.71E-01; Average Thickness P=1.60E-01; Center Thickness P=5.48E-01 | | | | |

### 2.4.2 Proteomic Analysis

The study layout is depicted in **Figure 5****.** Samples were randomly subdivided for selection and verification of potential biomarker candidates. Two thirds of all samples were used as a discovery set and selection of potential biomarker candidates and subgroup analysis (16 controls, 14 CRVO, 6 H-CRVO, 10 BRVO); one third was used for the validation using ROC analysis (8 controls, 6 CRVO, 3 H-CRVO, 5 BRVO). The two mass spectrometry techniques employed in this study are used in a complementary fashion. CE-MS, due to its high reproducibility, is used for candidate peptide selection while LC-MS/MS is used for identification of the biomarker peptides. Using LC-MS/MS analysis, 755 of the tryptic peptides detected with CE-MS could be sequenced. The mass of sequenced peptides ranged between 800.4 and 3619.0 Da. Migration time ranged between 20.1 and 38.0 min. The 755 tryptic peptides corresponded to 96 different proteins in VH **(Table S4,** **Figure 5****).**

**Table S4. Proteins in vitreous humor detected by capillary electrophoresis coupled to mass spectrometer (CE-MS) and identified by tandem mass spectrometry (LC-MS/MS) when comparing protein signal intensity of retinal vein occlusion (RVO)-samples compared to control-samples.**

| **Protein** | **UniProt^{‡}** | **RVO (n=30)** | | | | | | **Control (n=16)** | | | | |
|---|---|---|---|---|---|---|---|---|---|---|---|---|
| | | **Protein identification** | | **Statistical analysis** | | | **Protein identification** | | **Statistical analysis** | | | ***P*-Value^{§}** |
| | | Peptides^{##} | Coverage^{†} | N^{#} | Mean intensity | SD | Peptides^{##} | Coverage^{†} | N^{#} | Mean intensity | SD | |
| Afamin | P43652 | 3 | 0.05 | 19 | 34.6 | 105.6 | 3 | 0.05 | 12 | 48.9 | 53.3 | 7.07E-02 |
| Alpha-1-acid glycoprotein 1 | P02763 | 7 | 0.34 | 27 | 144.3 | 129.1 | 8 | 0.41 | 14 | 75.8 | 50.2 | 7.19E-D2 |
| Alpha-1-acid glycoprotein 2 | P19652 | 3 | 0.15 | 26 | 86.7 | 69.2 | 4 | 0.16 | 12 | 68.6 | 63.6 | 4.59E-01 |
| Alpha-1-antitrypsin | P01009 | 23 | 0.57 | 30 | 287.6 | 146.1 | 23 | 0.62 | 16 | 272.4 | 101.1 | 8.00E.01 |
| Alpha-1B-glycoprotein | P04217 | 2 | 0.04 | 6 | 9.4 | 27.2 | 3 | 0.07 | 4 | 5.7 | 14.7 | 8.73E-01 |
| Alpha-2-HS-glycoprotein | P02765 | 3 | 0.13 | 11 | 5.7 | 10.2 | 3 | 0.10 | 8 | 10.3 | 16.5 | 3.02E-01 |
| Alpha-2-macroglobulin | P01023 | 17 | 0.01 | 30 | 97.7 | 294.9 | 13 | 0.10 | 15 | 23.6 | 16.2 | 3.93E-01 |
| Alpha-crystallin B chain | P02511 | 2 | 0.11 | 3 | 1.9 | 7.4 | 5 | 0.30 | 7 | 30.1 | 75.5 | 9.20E-03 |
| Amyloid-like protein 2 | Q06481 | 7 | 0.11 | 14 | 9.1 | 27.2 | 5 | 0.08 | 9 | 5.6 | 9.4 | 7.67E-01 |
| Angiotensinogen | P01019 | 2 | 0.05 | 10 | 15.0 | 33.7 | 2 | 0.05 | 8 | 49.8 | 83.5 | 1.29E-01 |
| Antithrombin-III | P01008 | 11 | 0.27 | 28 | 71.1 | 33.1 | 11 | 0.27 | 14 | 50.2 | 31.7 | 5.55E-02 |
| Apolipoprotein A-I | P02647 | 17 | 0.70 | 26 | 184.4 | 75.8 | 17 | 0.74 | 15 | 142.0 | 87.6 | 1.17E-01 |
| Apolipoprotein A-II | P02652 | 2 | 0.29 | 30 | 8.1 | 14.4 | 4 | 0.41 | 15 | 50.1 | 67.6 | 1.78E-02 |
| Apolipoprotein A-IV | P06727 | 19 | 0.56 | 10 | 47.0 | 35.6 | 16 | 0.51 | 10 | 147.4 | 352.9 | 8.36E-01 |
| Apolipoprotein E | P02649 | 13 | 0.41 | 30 | 123.1 | 147.8 | 12 | 0.37 | 16 | 112.1 | 116.1 | 6.78E-01 |
| Beta-2-glycoprotein 1 | P02749 | 2 | 0.07 | 4 | 0.5 | 1.3 | 1 | 0.03 | 4 | 4.6 | 12.8 | 2.09E.01 |
| Beta-crystallin B2 | P43320 | 6 | 0.42 | 11 | 18.3 | 35.3 | 7 | 0.40 | 5 | 12.0 | 39.7 | 6.16E-01 |
| Calmodulin-regulated spectrin-associated protein 3^{§§} | Q9P1Y5 | 1 | 0.01 | 2 | 42.7 | 165.7 | 2 | 0.02 | 3 | 1.6 | 4.9 | 2.67E-01 |
| Cartilage gtycoprotein-39^{§§} | Q9NY41 | 2 | 0.13 | 12 | 3.0 | 4.9 | 3 | 0.20 | 7 | 6.9 | 12.6 | 5.70E-01 |
| Cathepsin D | P07339 | 6 | 0.20 | 29 | 242.1 | 467.3 | 7 | 0.22 | 15 | 553.9 | 1198.1 | 7.38E-01 |
| Ceruloplasmin | P00450 | 15 | 0.22 | 30 | 207.4 | 180.6 | 17 | 0.21 | 15 | 157.1 | 187.6 | 9.68E-02 |
| Clusterin* | P10909 | 9 | 0.26 | 30 | 524.1 | 194.2 | 10 | 0.28 | 15 | 282.3 | 174.6 | 4.97E-04 |
| Collagen alpha-1(I) chain | P02452 | 4 | 0.04 | 14 | 7.7 | 10.2 | 4 | 0.02 | 4 | 5.1 | 11.3 | 2.18E-01 |
| Collagen alpha-1(II) chain | P02458 | 29 | 0.25 | 30 | 95.7 | 132.7 | 35 | 0.45 | 15 | 371.1 | 1026.1 | 2.99E-01 |
| Collagen alpha-1(III) chain | P02461 | 3 | 0.04 | 23 | 93.4 | 91.6 | 3 | 0.04 | 10 | 37.9 | 41.8 | 4.48E-02 |
| Collagen alpha-1(V) chain | P20908 | 5 | 0.04 | 22 | 35.2 | 36.8 | 6 | 0.04 | 13 | 64.7 | 142.9 | 7.10E-01 |
| Collagen alpha-1(VII) chain | Q02388 | 3 | 0.02 | 5 | 29.3 | 101.6 | 2 | 0.01 | 8 | 25.1 | 57.2 | 2.94E-02 |
| Collagen alpha-1(IX) chain | P20849 | 9 | 0.15 | 21 | 18.9 | 32.3 | 9 | 0.16 | 12 | 73.1 | 187.1 | 8.16E-01 |
| Collagen alpha-1(XI) chain | P12107 | 5 | 0.04 | 14 | 88.2 | 317.3 | 6 | 0.04 | 8 | 145.8 | 444.7 | 9.80E-01 |
| Collagen alpha-1(XII) chain | Q99715 | 2 | 0.02 | 14 | 13.3 | 19.2 | 2 | 0.02 | 5 | 6.1 | 14.5 | 2.06E-01 |
| Collagen alpha-1 (XXII) chain | Q8NFW1 | 6 | 0.06 | 18 | 24.0 | 35.6 | 6 | 0.06 | 9 | 14.0 | 22.0 | 4-88E-01 |
| Collagen alpha-1 (XXIV) chain^{§§} | Q17RW2 | 1 | 0.01 | 1 | 1.9 | 10.5 | 2 | 0.02 | 3 | 6.6 | 20.7 | 8-50E-02 |
| Collagen alpha-1 (XXVII) chain^{§§} | Q8IZC6 | 2 | 0.03 | 4 | 1.2 | 3.8 | 1 | 0.02 | 2 | 257.3 | 854.5 | 9-37E-01 |
| Collagen alpha-1(XXVIII) chain | Q2UY09 | 1 | 0.01 | 1 | 2.0 | 11.1 | 1 | 0.02 | 1 | 16.9 | 67.5 | 6.25E-01 |
| Collagen alpha-2(I) chain | P08123 | 4 | 0.07 | 18 | 15.1 | 15.8 | 4 | 0.07 | 6 | 5.1 | 8.1 | 4.32E-02 |
| Collagen alpha-2(IX) chain | Q14055 | 3 | 0.06 | 6 | 17.2 | 87.5 | 5 | 0.09 | 5 | 134.1 | 443.6 | 2.95E-01 |
| Collagen alpha-2(XI) chain | P13942 | 3 | 0.03 | 30 | 740.4 | 678.7 | 4 | 0.03 | 13 | 377.1 | 528.5 | 8.55E-03 |
| Collagen alpha-3(IX) chain | Q14050 | 8 | 0.15 | 18 | 64.6 | 104.3 | 8 | 0.17 | 11 | 155.8 | 156.3 | 8.41E-02 |
| Collagen alpha-4(IV) chain^{§§} | P53420 | 1 | 0.01 | 1 | 1.3 | 7.1 | 1 | 0.01 | 2 | 2.0 | 5.6 | 2.58E-01 |
| Collagen alpha-5(IV) chain | P29400 | 2 | 0.02 | 24 | 251.2 | 271.9 | 2 | 0.02 | 16 | 264.0 | 353.1 | 8.35E-01 |
| Complement C3* | P01024 | 43 | 0.29 | 30 | 582.3 | 134.3 | 41 | 0.27 | 16 | 390.2 | 194.5 | 1.85E-03 |
| Complement C4-A | P0C0L4 | 2 | 0.02 | 4 | 2.0 | 6.9 | 4 | 0.03 | 7 | 29.3 | 78.3 | 1.91E-02 |
| Complement component 4B preproprotein | P0C0L5 | 13 | 0.10 | 26 | 18.0 | 17.0 | 12 | 0.10 | 13 | 18.2 | 23.0 | 5.48E-01 |
| Complement component C9 | P02748 | 2 | 0.04 | 28 | 280.4 | 175.9 | 2 | 0.04 | 13 | 245.2 | 194.7 | 7.12E-01 |
| Complement factor B | P00751 | 2 | 0.03 | 13 | 18.1 | 60.4 | 4 | 0.06 | 11 | 44.9 | 61.4 | 1.23E-02 |
| Cystatin-C | P01034 | 4 | 0.30 | 25 | 48.4 | 90.8 | 3 | 0.23 | 12 | 39.9 | 38.3 | 5.78E-01 |
| Dermcidin | P81605 | 2 | 0.20 | 10 | 65.7 | 159.6 | 1 | 0.10 | 6 | 79.1 | 178.6 | 4.02E-01 |
| Dickkopf-related protein 3 | Q9UBP4 | 6 | 0.22 | 27 | 92.7 | 102.6 | 6 | 0.27 | 14 | 129.9 | 142.7 | 3.21E-01 |
| Fibrinogen alpha chain | P02671 | 4 | 0.08 | 16 | 8.9 | 11.2 | 4 | 0.08 | 6 | 12.0 | 31.1 | 3.70E-01 |
| Fibrinogen beta chain | P02675 | 3 | 0.06 | 18 | 27.3 | 38.3 | | 0.09 | 7 | 6.9 | 14.1 | 6.54E-02 |
| Fizzy-related protein homolog^{§§} | Q9UM11 | 2 | 0.05 | 12 | 5.8 | 9.9 | 1 | 0.01 | 6 | 2.7 | 4.4 | 4.96E-01 |
| Gelsolin | P06396 | 4 | 0.06 | 17 | 26.2 | 43.8 | 3 | 0.05 | 12 | 29.5 | 66.1 | 9.25E-01 |
| Glutathione peroxidase 3 | P22352 | 4 | 0.18 | 24 | 88.4 | 83.7 | 4 | 0.18 | 12 | 78.7 | 174.6 | 1.57E-01 |
| Haptoglobin | P00738 | 8 | 0.28 | 24 | 49.5 | 92.3 | 9 | 0.24 | 10 | 30.6 | 51.3 | 2.74E-01 |
| Hemoglobin subunit beta | P68871 | 4 | 0.36 | 14 | 94.8 | 389.5 | 3 | 0.34 | 7 | 38.6 | 136.9 | 3.65E-01 |
| Hemopexin | P02790 | 14 | 0.34 | 30 | 133.7 | 55.5 | 12 | 0.28 | 15 | 159.3 | 92.7 | 1.74E-01 |
| Heparin cofactor 2 | P05546 | 1 | 0.02 | 4 | 1.3 | 4.5 | 2 | 0.04 | 4 | 1.3 | 3.0 | 3.64E-01 |
| Histidine-rich glycoprotein | P04196 | 2 | 0.04 | 5 | 7.9 | 26.8 | 1 | 0.02 | 4 | 6.9 | 17.7 | 5-71E-01 |
| Ig alpha-1 chain C region | P01876 | 2 | 0.04 | 13 | 40.8 | 88.0 | 2 | 0.04 | 5 | 16.0 | 41.0 | 3.26E-01 |
| Ig alpha-2 chain C region | P01877 | 4 | 0.15 | 25 | 132.3 | 144.8 | 2 | 0.06 | 11 | 84.2 | 88.9 | 2.33E-01 |
| Ig gamma-1 chain C region | P01857 | 11 | 0.32 | 30 | 787.7 | 358.7 | 11 | 0.32 | 16 | 725.9 | 462.5 | 4.20E-01 |
| Ig gamma-2 chain C region | P01859 | 2 | 0.06 | 21 | 35.2 | 41.8 | 2 | 0.06 | 14 | 65.0 | 94.3 | 3.07E-01 |
| Ig gamma-3 chain C region | P01860 | 3 | 0.10 | 12 | 44.7 | 87.8 | 4 | 0.12 | 8 | 47.2 | 94.0 | 6.65E-01 |
| Ig heavy chain V-III region | P01781 | 3 | 0.27 | 11 | 11.3 | 24.1 | 2 | 0.17 | 7 | 40.3 | 65.3 | 3.01E-01 |
| GAL | | | | | | | | | | | | |
| Ig kappa chain C region | P01834 | 4 | 0.80 | 27 | 113.8 | 116.6 | 4 | 0.80 | 11 | 110.8 | 108.3 | 8.81E-01 |
| Ig kappa chain V-I region EU | P01598 | 2 | 0.80 | 4 | 7.1 | 29.6 | 1 | 0.17 | 3 | 3.1 | 7.4 | 6.58E-D1 |
| Ig lambda-2 chain C regions | P0CG05 | 2 | 0.27 | 25 | 717.5 | 525.6 | 3 | 0.42 | 12 | 452.7 | 305.4 | 7.85E-02 |
| IgGFc-binding protein | Q9Y6R7 | 4 | 0.01 | 20 | 117.2 | 309.7 | 4 | 0.01 | 14 | 181.5 | 268.0 | 1.63E-01 |
| Ig lambda-like polypeptide 5* | B9A064 | 3 | 0.20 | 30 | 358.2 | 422.5 | 2 | 0.13 | 11 | 93.4 | 75.7 | 1.43E-05 |
| Inter-alpha (Globulin) inhibitor H2 | A2RTY6 | 2 | 0.02 | 18 | 18.9 | 23.9 | 2 | 0.02 | 6 | 12.5 | 26.5 | 1.71E-01 |
| Inter-alpha-trypsin inhibitor heavy chain H1 | P19827 | 3 | 0.04 | 28 | 107.9 | 128.4 | 3 | 0.04 | 13 | 92.5 | 79.6 | 9.45E-01 |
| Inter-alpha-trypsin inhibitor heavy chain H4 | Q14624 | 2 | 0.02 | 8 | 4.2 | 9.7 | 1 | 0.01 | 1 | 0.0 | 0.2 | 7.76E-02 |
| Kininogen-1 | P01042 | 5 | 0.09 | 9 | 4.9 | 8.9 | 6 | 0.11 | 7 | 18.4 | 47.8 | 3.49E-01 |
| Leucine-rich alpha-2-glycoprotein | P02750 | 1 | 0.03 | 8 | 26.5 | 86.4 | 2 | 0.06 | 7 | 25.8 | 47.6 | 3.19E-01 |
| Neuroblast differentiation-associated protein AHNAK^{§§} | Q09666 | 2 | 0.01 | 6 | 1.4 | 3.9 | 4 | 0.01 | 9 | 14.0 | 24.8 | 3.34E-03 |
| Obscurin | Q5VST9 | 2 | 0.00 | 21 | 520.3 | 1358.1 | 2 | 0.00 | 16 | 1767.1 | 3857.7 | 1.51E-02 |
| Opticin* | Q9UBM4 | 6 | 0.20 | 23 | 82.8 | 207.2 | 5 | 0.16 | 16 | 163.7 | 95.1 | 7.07E-05 |
| Osteopontin | P10451 | 5 | 0.28 | 25 | 78.7 | 140.7 | 4 | 0.18 | 13 | 53.1 | 80.0 | 7.37E-01 |
| Pigment epithelium-derivedfactor | P36955 | 12 | 0.31 | 30 | 185.7 | 75.7 | 15 | 0.36 | 15 | 161.2 | 134.1 | 1.59E-01 |
| Plasminogen | P00747 | 2 | 0.02 | 9 | 3.9 | 7.3 | 2 | 0.02 | 7 | 12.8 | 18.6 | 1.62E-01 |
| Prostaglandin-H2 D-isomerase | P41222 | 4 | 0.21 | 27 | 2589.7 | 2630.4 | 4 | 0.21 | 13 | 4122.9 | 6744.6 | 1.00E+00 |
| Protein Jade-2 | Q9NQC1 | 1 | 0.01 | 1 | 0.1 | 0.6 | 2 | 0.03 | 2 | 0.2 | 0.7 | 2.58E-01 |
| Protein S100-A9 | P06702 | 1 | 0.06 | 3 | 0.2 | 0.6 | 2 | 0.18 | 5 | 44.6 | 155.8 | 7.79E-02 |
| Prothrombin | P00734 | 2 | 0.04 | 8 | 14.8 | 32.7 | 2 | 0.04 | 3 | 25.6 | 88.9 | 5.17E-01 |
| Retinol-binding protein 3 | P10745 | 19 | 0.22 | 30 | 254.7 | 514.0 | 15 | 0.17 | 16 | 605.1 | 1113.3 | 6.50E-02 |
| RNA-directed DNA polymerase (reverse transcriptase), related domain containing protein^{§§} | - | 2 | 0.09 | 25 | 140.4 | 140.2 | 2 | 0.09 | 12 | 56.7 | 48.5 | 1.51E-02 |
| Serine/cysteine proteinase inhibitor clade G member 1 splice variant 2^{§§} | Q5UGI6 | 5 | 0.19 | 19 | 20.9 | 32.4 | 6 | 0.20 | 12 | 18.0 | 30.6 | 9.63E-01 |
| Serotransferrin | P02787 | 38 | 0.49 | 30 | 264.5 | 82.0 | 33 | 0.45 | 16 | 323.7 | 181.1 | 6.61E-01 |
| SERPINA3 Alpha-1-antichymotrypsin | P01011 | 14 | 0.31 | 30 | 44.9 | 26.8 | 13 | 0.31 | 16 | 43.1 | 30.3 | 8.54E-01 |
| Serum albumin | P02768 | 40 | 0.61 | 30 | 2109.7 | 657.6 | 42 | 0.65 | 16 | 2910.3 | 1163.1 | 2.11E-02 |
| Titin | Q8WZ42 | 3 | 0.00 | 16 | 6.1 | 8.0 | 4 | 0.00 | 6 | 3.5 | 6.1 | 2.96E-01 |
| Translational activator GCN1^{§§} | Q92616 | 2 | 0.01 | 2 | 184.9 | 340.9 | 2 | 0.01 | 1 | 62.8 | 122.1 | 5.01E-01 |
| Transthyretin | P02766 | 7 | 0.69 | 27 | 276.6 | 247.7 | 7 | 0.50 | 14 | 517.6 | 479.2 | 1.52E-01 |
| Vitamin D-binding protein | P02774 | B | 0.14 | 22 | 22.1 | 27.6 | 8 | 0.15 | 11 | 25.3 | 32.1 | 9.07E-01 |
| Vitronectin* | P04004 | 4 | 0.10 | 25 | 89.6 | 136.0 | 3 | 0.08 | 8 | 8.8 | 11.0 | 2.74E-04 |
| Zinc-alpha-2-glycoprotein | P25311 | 3 | 0.14 | 19 | 37.7 | 55.8 | 3 | 0.14 | 7 | 12.5 | 24.5 | 9.43E-02 |
| | | | | | | | | | | | | |
| ‡ | Listed in the universal protein resource (UniProt), a central repository of protein data. | | | | | | | | | | | |
| ## | Number of peptides observed by CE-MS analysis and sequenced by LC-MS/MS for each protein. | | | | | | | | | | | |
| † | Percentage of peptide coverage of the protein sequence. | | | | | | | | | | | |
| # | Number of samples with a signal intensity >0 | | | | | | | | | | | |
| § | P-Value was analyzed by using the Mann-Whitney test A *P* of α<5.00E-02 was considered statistically significant. Significant proteins are highlighted in light grey. | | | | | | | | | | | |
| * | Proteins which remained significant after performing multiple hypotheses testing correction, analyzed by using the Benjamini-Hochberg test for false discovery rate (see Table 2). A adjusted *P*-Value of α<5.00E-02 was considered statistically significant | | | | | | | | | | | |
| §§ | Proteins being not described in previous studies of human vitreous humor proteome [12, 38-40]. | | | | | | | | | | | |

### 2.4.3 Selection of Potential Biomarker Candidates

In the discovery study, the comparison of protein data between RVO (irrespective of subgroup) and control samples resulted in 17 significant proteins (*P*=1.43E-05 to 4.48E-02; **Table 2).** After using correction for multiple testing, five proteins remained significant **(Table 5, see *;** **Figure 5****):** Clusterin, Complement C3, Ig lambda-like polypeptide 5 (IGLL5), Opticin and Vitronectin.

### 2.4.4 Gene Ontology And Pathway Analysis Of Potential Biomarker Candidates

The 17 proteins that were detected significantly different when comparing RVO and control samples **(Table 5)** were classified according to the related Gene Ontology (GO) terms using WebGestalt (http://bioinfo.vanderbilt.edu/webgestalt/).

**Table 5. Significant proteins in vitreous humor detected by capillary electrophoresis coupled to mass spectrometer (CE-MS) and identified by tandem mass spectrometry (LC-MS/MS) when comparing protein signal intensity of retinal vein occlusion (RVO)-samples compared to control-samples.**

| **Protein** | **UniProt^{‡}** | **RVO (n=30)** | | | | | **Control (n=16)** | | | | | ***P*-Value^{§}** | **Benjamini-Hochberg (adjusted *P*-Value)** |
|---|---|---|---|---|---|---|---|---|---|---|---|---|---|
| | | Protein identification | | Statistical analysis | | | Protein identification | | Statistical analysis | | | | |
| | | Peptides^{##} | Coverage^{†} | N^{#} | Mean intensity | SD | Peptide^{##} | Coverage^{†} | N^{#} | Mean intensity | SD | | |
| Upregulation | | | | | | | | | | | | | |
| Ig lambda-like polypeptide 5* | B9A064 | 3 | 0.20 | 30 | 358.2 | 422.5 | 2 | 0.13 | 11 | 93.4 | 75.7 | 1.43E-05 | 1.37E-03 |
| Vitronectin* | P04004 | 4 | 0.10 | 25 | 89.6 | 136.0 | 3 | 0.08 | 8 | 8.8 | 11.0 | 2.74E-04 | 8.77E-03 |
| Clusterin* | P10909 | 9 | 0.26 | 30 | 524.1 | 194.2 | 10 | 0.28 | 15 | 282.3 | 174.6 | 4.97E-04 | 1.19E-02 |
| Complement C3* | P01024 | 43 | 0.29 | 30 | 582.3 | 134.3 | 41 | 0.27 | 16 | 390.2 | 194.5 | 1.85E-03 | 3.55E-02 |
| Collagen alpha-2(XI) chain | P13942 | 3 | 0.06 | 30 | 740.4 | 678.7 | 5 | 0.09 | 13 | 377.1 | 528.5 | 8.55E-03 | 1.10E-01 |
| RNA-directed DNA polymerase (reverse transcriptase). related domain containing protein | - | 2 | 0.09 | 25 | 140.4 | 140.2 | 2 | 0.09 | 12 | 56.7 | 48.5 | 1.516-02 | 1.31E-01 |
| Collagen alpha-1(VII) chain | Q02388 | 3 | 0.02 | 5 | 29.3 | 101.6 | 2 | 0.01 | 8 | 25.1 | 57.2 | 2.946-02 | 1.88E-01 |
| Collagen alpha-2(I) chain | P08123 | 4 | 0.07 | 18 | 15.1 | 15.8 | 4 | 0.07 | 6 | 5.1 | 8.1 | 4.326-02 | 2.53E-01 |
| Collagen alpha-1(III) chain | P02461 | 3 | 0.04 | 23 | 93.4 | 91.6 | 3 | 0.04 | 10 | 37.9 | 41.8 | 4.486-02 | 2.532-01 |
| | | | | | | | | | | | | | |
| Downregulation | | | | | | | | | | | | | |
| Opticin* | Q9UBM4 | 6 | 0.20 | 23 | 82.8 | 207.2 | 5 | 0.16 | 16 | 163.7 | 95.1 | 7.07E-05 | 3.39E-03 |
| Neuroblast differentiation-associated protein AHNAK | 009666 | 2 | 0.01 | 6 | 1.4 | 3.9 | 4 | 0.01 | 9 | 14.0 | 24.8 | 3.34E-03 | 5.34E-02 |
| Alpha-crystallin chain | P02511 | 2 | 0.11 | 3 | 1.9 | 7.4 | 5 | 0.30 | 7 | 30.1 | 75.5 | 9.20E-03 | 1.10E-01 |
| Complement factor B | P00751 | 2 | 0.03 | 13 | 18.1 | 60.4 | 4 | 0.06 | 11 | 44.9 | 61.4 | 1.23E-02 | 1.31E-01 |
| Obscurin | Q5VST9 | 2 | 0.00 | 21 | 520.3 | 1358.1 | 2 | 0.00 | 16 | 1767.1 | 3857.7 | 1.516-02 | 1.31E-01 |
| Apolipoprotein A-II | P02652 | 2 | 0.29 | 10 | 8.1 | 14.4 | 4 | 0.41 | 10 | 50.1 | 67.6 | 1.78E-02 | 1.41E-01 |
| Complement C4-A | P0COL4 | 2 | 0.02 | 4 | 2.0 | 6.9 | 4 | 0.03 | 7 | 29.3 | 78.3 | 1.91E-02 | 1.41E-01 |
| Serum albumin | P02768 | 40 | 0.61 | 30 | 2109.7 | 657.6 | 42 | 0.65 | 16 | 2910.3 | 1163.1 | 2.11E-02 | 1.45E-01 |
| | | | | | | | | | | | | | |
| ‡ | Listed in the universal protein resource (UniProt). a central repository of protein data. | | | | | | | | | | | | |
| ## | Number of peptides observes by CE-MS analysis and sequenced by LC-MS/MS for each protein. | | | | | | | | | | | | |
| † | Percentage of peptide coverage of the protein sequence. | | | | | | | | | | | | |
| # | Number of samples with a signal intensity >0 | | | | | | | | | | | | |
| § | using *P*-Value was analyzed by the Mann-Whitney test. A *P* of α<5.00E-02 was considered statistically significant. | | | | | | | | | | | | |
| * | Proteins which remained significant after performing multiple hypotheses testing correction. analyzed by using the Benjamini-Hochberg test for false discovery rate. A adjusted *P*-Value of α<5.00E-02 was considered statistically significant. | | | | | | | | | | | | |

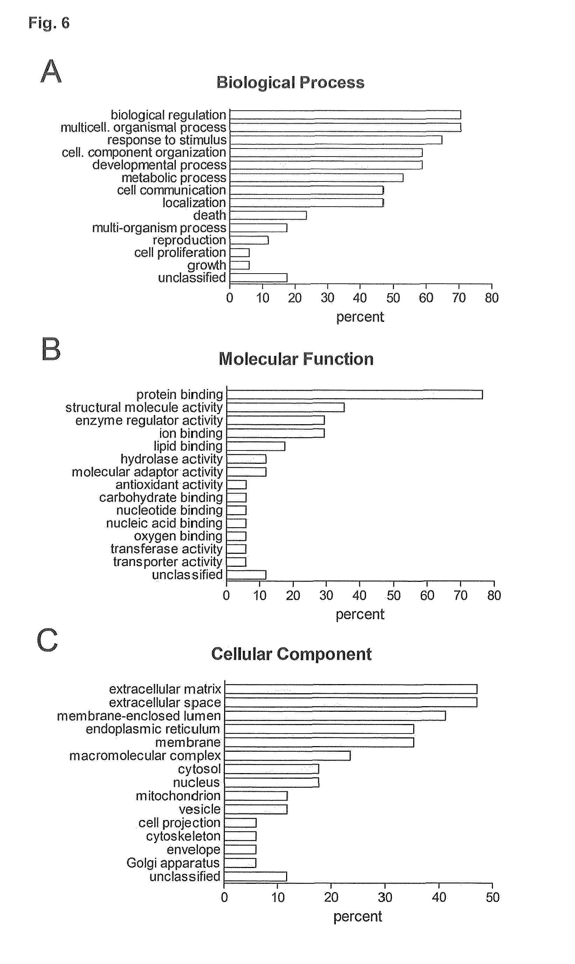

Major biological processes of these proteins were biological regulation, multicellular organismal process and response to stimulus **(****Figure 6A****).** Major molecular functions of the VH proteins enriched among RVO patients were assigned as protein binding, structural molecule activity, enzyme regulator activity and ion binding **(****Figure 6B****).** Most of the VH proteins were categorized as extracellular proteins according to cellular component terms of the GO **(****Figure 6C****).**

To reveal canonical pathways that are potentially involved in the pathology of RVO, we used WebGestalt (http://bioinfo.vanderbilt.edu/webgestalt/) allowing access to Wikipathways and Pathway Commons. Primary Wikipathways that were associated with VH proteins being significantly up- or downregulated (n=17; **Table 5)** were: inflammatory response pathway, focal adhesion, complement activation (classical pathway), complement and coagulation cascades.

### 2.4.5 Subgroup Analysis

Signal intensity of the proteins of each subgroup (CRVO, H-CRVO, BRVO) and the controls was compared by using the closed testing procedure. In step 3 ten proteins were expressed significantly different in each, CRVO, H-CRVO and BRVO samples *versus* controls **(Table 6).**

**Table 6. Significant proteins in vitreous humor detected by capillary electrophoresis coupled to mass spectrometer (CE-MS) and identified by tandem mass spectrometry (LC-MS/MS) when comparing protein signal intensity of subgroups of retinal vein occlusion (RVO)-samples (central RVO (CRVO), hemi-central RVO (H-CRVO), branch RVO (BRVO)) compared to control-samples.**

| **Protein** | **UniPort^{‡}** | **Control (n=16)** | | | **CRVO (n=14)** | | | ***P*-Value^{§}** Control vs. CRVO | **H**-**CRVO (n=6)** | | | ***P*-Value^{§}** Control vs. H-CRVO | **BRVO (n=10)** | | | ***P*-Value^{§}** Control vs. BRVO |
|---|---|---|---|---|---|---|---|---|---|---|---|---|---|---|---|---|
| | | N^{#} | Signal intensity | | N^{#} | Signal intensity | | | N^{#} | Signal intensity | | | N^{#} | Signal intensity | | |
| | | | Mean | SD | | Mean | SD | | | Mean | SD | | | Mean | SD | |
| Clusterin*^{; †; ††; †††; ††††} | P10909 | 15 | 282.3 | 174.6 | 14 | 487.5 | 187.6 | 1.12E-02 | 6 | 570.4 | 248.9 | 9.87E-03 | 10 | 547.6 | 179.5 | 3.75E-03 |
| Collagen alpha-1(V) chain^{†; †††} | P20908 | 13 | 64.7 | 142.9 | 8 | 14.3 | 24.0 | 1.09E-01 | 5 | 34.8 | 23.2 | 3.55E-01 | 9 | 64.7 | 39.9 | 2.85E-02 |
| Collagen alpha-2(X1) chain^{†; ††; †††} | P13942 | 13 | 377.1 | 528.5 | 14 | 1097.0 | 856.5 | 9.92E-03 | 6 | 371.7 | 40.9 | 1.84E-01 | 10 | 462.3 | 222.2 | 7.29E-2 |
| Compliment C3*^{; ††; †††; ††††} | P01024 | 16 | 390.2 | 194.5 | 14 | 569.1 | 167.6 | 1.99E-02 | 6 | 603.5 | 84.2 | 1.83E-02 | 10 | 588.1 | 113.9 | 1.32E-02 |
| Complement C4-A^{†; ††; †††} | P0C0L4 | 7 | 29.3 | 78.3 | 0 | 0.0 | 0.0 | 6.01E-03 | 2 | 7.5 | 14.6 | 6.79E-01 | 2 | 1.5 | 3.3 | 1.72E-01 |
| Complement factor B^{†; ††;} | P00751 | 11 | 44.9 | 61.4 | 8 | 13.3 | 21.2 | 9.64E-02 | 1 | 1.2 | 3.0 | 1.82E-02 | 4 | 35.1 | 102.7 | 7.05E-02 |
| Fibrinogen alpha chain^{†; ††; ††††} | P02671 | 6 | 12.0 | 31.1 | 6 | 6.3 | 8.7 | 7.78E-01 | 6 | 18.7 | 9.9 | 2.02E-02 | 4 | 6.8 | 12.7 | 1.00E+00 |
| Haptoglobin^{†; †††; ††††} | P00738 | 10 | 30.6 | 51.3 | 9 | 14.9 | 23.8 | 7.33E-01 | 5 | 24.3 | 21.1 | 5.00E-01 | 10 | 113.0 | 140.1 | 1.47E-02 |
| Ig lambda-2 chain C regions^{†; ††† ††††} | P0CG05 | 12 | 452.7 | 305.4 | 10 | 484.6 | 494.0 | 9.67E-01 | 5 | 684.3 | 465.2 | 2.36E-01 | 10 | 1063.4 | 446.5 | 1.84E-03 |
| IgGFc-binding protein^{†; ††;} | Q9Y6R7 | 14 | 181.5 | 268.0 | 9 | 170.1 | 443.2 | 1.22E-01 | 2 | 11.3 | 17.7 | 2.10E-02 | 9 | 106.6 | 115.0 | 6.73E-01 |
| Ig lambda-like polypeptide 5*^{; ††; †††; ††††} | B9A064 | 11 | 93.4 | 75.7 | 14 | 215.4 | 118.0 | 2.05E-03 | 6 | 561.9 | 619.6 | 1.11E-03 | 10 | 435.9 | 525.0 | 2.14E-04 |
| Neuroblast differentiation-associated protein AHNAK*^{; †; †††; ††††} | Q09666 | 9 | 14.0 | 24.8 | 3 | 2.1 | 5.1 | 2.91E-02 | 3 | 2.4 | 3.5 | 2.62E-01 | 0 | 0.0 | 0.0 | 5.22E-03 |
| Obscurin*^{; †; ††; ††††} | Q5VST9 | 16 | 1767.1 | 3857.7 | 8 | 775.6 | 1967.0 | 3.00E-02 | 4 | 136.1 | 161.8 | 1.50E-02 | 9 | 393.5 | 349.5 | 2.92E-01 |
| Opticin*^{; †; ††; †††; ††††} | Q9UBM 4 | 16 | 163.7 | 95.1 | 9 | 111.6 | 294.7 | 7.39E-04 | 6 | 62.6 | 61.7 | 1.83E-02 | 8 | 54.6 | 91.0 | 1.87E-03 |
| Pigment epithelium-derived factor^{†; †††; ††††} | P36955 | 15 | 161.2 | 134.1 | 14 | 145.8 | 56.7 | 9.01E-01 | 6 | 177.5 | 55.4 | 3.38E-01 | 10 | 246.5 | 74.1 | 1.77E-02 |
| RNA-directed DNA polymerase (reverse transcriptase). related | - | 12 | 56.7 | 48.5 | 11 | 146.6 | 160.7 | 5.43E-02 | 6 | 202.0 | 159.2 | 3.94E-03 | 8 | 94.8 | 83.7 | 2.89E-01 |
| domain containing protein^{†; ††; ††††} | | | | | | | | | | | | | | | | |
| Serum albumin^{†; ††; †††} | P02768 | 16 | 2910.3 | 1163.1 | 14 | 1759.2 | 603.5 | 6.08E.03 | 6 | 2385.6 | 325.2 | 2.69E-01 | 10 | 2434.6 | 670.3 | 3.17E-01 |
| Vitronectin*^{; †; ††; †††; ††††} | P04004 | 8 | 8.8 | 811.0 | 10 | 71.9 | 100.9 | 1.81E-02 | 6 | 38.3 | 16.0 | 1.51E-03 | 9 | 145.1 | 197.6 | 1.50E-03 |

| | | | | | | | | | | | | | | | | |
|---|---|---|---|---|---|---|---|---|---|---|---|---|---|---|---|---|
| ‡ Listed in the universal protein resource (UniProt). a central repository of protein data. # Number of samples with a signal intensity >0 § *P*-Value was analyzed by using the Mann-Whitney test A P of α<5.00E-02 was considered statistically significant. Significant results are highlighted in dark grey. † Closed testing procedure was used to verify the results. Kruskal-Wallis Test was used for analysis. A P of α<5.00E-02 was considered statistically significant. Proteins are marked when being significant in listed steps: Step 1: † Control vs. CRVO vs. H-CRVO vs. BRVO Step 2: †† Control vs. CRVO vs. H-RVO ††† Control vs. CRVO vs. BRVO †††† Control vs. H-CRVO BRVO Step 3: For detailed information of step 3 see § Proteins which remained significant after performing multiple hypotheses testing correction, analyzed by using the Benjamini-Hochberg test for false discovery rate. Significant proteins in vitreous humor detected by CE-MS and identified by LC-MS/MS when comparing protein signal intensity of RVO-samples (n=30) compared to control-samples (n=16: see Tab. 2). | | | | | | | | | | | | | | | | |

Five proteins remained significant in all comparisons of step 1 to step 3 **(Table S5):** Clusterin, Complement C3, IGLL5, Opticin and Vitronectin.

**Table S5. Proteins in vitreous humor detected by capillary electrophoresis coupled to mass spectrometer (CE-MS) and identified by tandem mass spectrometry (LC-MS/MS) when comparing protein signal intensity of fresh/old retinal vein occlusion (RVO) compared to control-samples.**

| **Protein** | **UniPort^{‡}** | **Control (n=16)** | | | **RVO-fresh (n=22)** | | | ***P*-Value^{§}** Control vs. RVO-fresh | **RVO-old (n=8)** | | | ***P*-Value^{§}** Control vs. RVO-old | ***P*-Value^{§}** RVO-fresh vs. RVO-old |
|---|---|---|---|---|---|---|---|---|---|---|---|---|---|
| | | N^{#} | Signal intensity | | N^{#} | Signal intensity | | | N^{#} | Signal intensity | | | |
| | | | Mean | SD | | Mean | SD | | | Mean | SD | | |
| Afamin | P43652 | 12 | 48.9 | 53.3 | 11 | 42.3 | 123.1 | 8.20E-02 | 8 | 13.3 | 4.6 | 2.20E-01 | 5.01E-01 |
| Alpha-1-acid glycoprotein 1 | P02763 | 14 | 75.8 | 50.2 | 19 | 114.2 | 113.8 | 3.91E-01 | 8 | 227.1 | 139.7 | 2.69E-03 | 2.43E-02 |
| Alpha-1-acid glycoprotein 2 | P19652 | 12 | 68.6 | 63.6 | 18 | 82.8 | 68.2 | 5.93E-01 | 8 | 97.7 | 75.4 | 3.90E-01 | 7.07E-01 |
| Alpha-1-anititrypsin | PD1009 | 16 | 272.4 | 101.11 | 22 | 283.8 | 166.1 | 5.95E-01 | 8 | 298.2 | 73.7 | 6.68E-01 | 3.99E-01 |
| Alpha-1B-glycoprotein | P04217 | 4 | 5.7 | 14.7 | 5 | 12.0 | 31.4 | 9.37E-01 | 1 | 2.3 | 6.4 | 5.46E-01 | 4.81E-01 |
| Alpha-2-HS-glycoprotein | P02765 | 8 | 10.3 | 16.5 | 6 | 5.1 | 11.1 | 1.51E-01 | 5 | 7.6 | 7.3 | 8.98E-01 | 1.15E-01 |
| Alpha-2-macroglobulin | P01023 | 15 | 23.6 | 16.2 | 22 | 124.2 | 342.4 | 4.25E-01 | 8 | 24.7 | 13.1 | 5.40E-01 | 8.51E-01 |
| Alpha-crystallin B chain | P02511 | 7 | 30.1 | 75.5 | 3 | 2.6 | 8.6 | 4.12E-02 | 0 | 0.0 | 0.0 | 3.28E-02 | 2.80E-01 |
| Amyloid-like protein 2 | Q06481 | 9 | 5.6 | 9.4 | 9 | 10.9 | 31.6 | 6.43E-01 | 5 | 4.2 | 4.7 | 8.74E-01 | 5.58E-01 |
| Angiotensinogen | P01019 | 8 | 49.8 | 83.5 | 8 | 18.0 | 38.1 | 1.99E-01 | 2 | 6.9 | 15.5 | 1.94E-01 | 5.02E-01 |
| Antithrombin-III | P01008 | 14 | 50.2 | 31.7 | 20 | 69.1 | 36.8 | 1.47E-01 | 8 | 76.6 | 20.7 | 3.73E.02 | 6.06E-01 |
| Apolipoprotein A-I | P02647 | 15 | 142.0 | 87.6 | 22 | 174.2 | 82.1 | 3.15E-01 | 8 | 212.7 | 48.4 | 3.73E.02 | 2.60E-01 |
| Apolipoprotein A-II | P02652 | 10 | 50.1 | 67.6 | 5 | 3.9 | 8.2 | 6.00E-03 | 5 | 19.7 | 21.2 | 4.89E-01 | 1.75E-02 |
| Apolipoprotein A-IV | P06727 | 16 | 147.4 | 352.9 | 22 | 45.0 | 39.7 | 5.54E-01 | 8 | 52.5 | 21.9 | 5.01E-01 | 3.25E-01 |
| Apolipoprotein E | P02649 | 15 | 112.1 | 116.1 | 18 | 131.0 | 158.1 | 7.67E-01 | 8 | 101.1 | 121.8 | 6.24E-01 | 7.78E-01 |
| Beta-2-glycoprotein 1 | P02749 | 4 | 4.6 | 12.8 | 2 | 0.3 | 1.1 | 1.36E-01 | 2 | 0.8 | 1.7 | 7.47E-01 | 2.67E-01 |
| Beta-crystallin 62 | P43320 | 5 | 12.0 | 39.7 | 5 | 10.5 | 23.7 | 7.17E-01 | 6 | 39.8 | 52.5 | 6.16E-02 | 1.25E-02 |
| Calmodulin-regulated spectrin-associated protein 3 | Q9P1Y5 | 3 | 1.6 | 4.9 | 2 | 58.2 | 192.2 | 4.81E-01 | 0 | 0.0 | 0.0 | 2.01E-01 | 3.86E-01 |
| Cartilage glycoprotein-39 | Q9NY41 | 7 | 6.9 | 12.6 | 7 | 2.5 | 5.0 | 2.97E-01 | 5 | 4.5 | 4.4 | 5.78E-01 | 9.53E-02 |
| Cathepsin D | P07339 | 15 | 553.9 | 1198.1 | 21 | 294.0 | 537.6 | 8.94E-01 | 8 | 99.2 | 74.6 | 5.40E-01 | 4.82E-01 |
| Ceruloplasmin | P00450 | 15 | 157.1 | 187.6 | 22 | 229.3 | 203.9 | 8.64E-02 | 8 | 147.0 | 68.4 | 3.91E-01 | 4.82E-01 |
| Clusterin* | P10909 | 15 | 282.3 | 174.6 | 22 | 551.2 | 209.2 | 6.04E-04 | 8 | 449.6 | 127.8 | 3.21E-02 | 2.41E-01 |
| Collagen alpha-1(I) chain | P02452 | 4 | 5.1 | 11.3 | 10 | 6.2 | 8.3 | 3.05E-01 | 4 | 11.8 | 14.1 | 2.15E-01 | 4.15E-01 |
| Collagen alpha-1(II) chain | P02458 | 15 | 371.1 | 1026.1 | 22 | 103.3 | 147.7 | 2.49E-01 | 8 | 74.5 | 82.6 | 7.13E-01 | 3.48E-01 |
| Collagen alpha-1(III) chain | P02461 | 10 | 37.9 | 41.8 | 16 | 87.8 | 93.8 | 1.18E-01 | 7 | 108.6 | 89.4 | 3.50E-02 | 3.22E-01 |
| Collagen alpha-1(V) chain | P20908 | 13 | 64.7 | 142.9 | 16 | 39.6 | 36.0 | 3.72E-01 | 6 | 23.1 | 38.7 | 3.89E-01 | 2.37E-01 |
| Collagen alpha-1(VII) chain | Q02388 | 8 | 25.1 | 57.2 | 2 | 18.2 | 84.8 | 6.00E-03 | 3 | 59.7 | 140.4 | 8.41E-01 | 6.04E-02 |
| Collagen alpha-1(IX) chain | P20849 | 12 | 73.1 | 187.1 | 14 | 21.5 | 36.9 | 9.76E-01 | 7 | 12.0 | 12.2 | 5.80E-01 | 9.62E-01 |
| Collagen alpha-1(XI) chain | P12107 | 8 | 145.8 | 444.7 | 9 | 116.7 | 368.4 | 7.95E-01 | 5 | 9.8 | 17.8 | 5.62E-01 | 4.60E-01 |
| Collagen alpha-1(XII) chain | Q99715 | 5 | 6.1 | 14.5 | 9 | 14.3 | 21.4 | 3.47E-01 | 5 | 10.8 | 11.6 | 1.42E-01 | 7.41E-01 |
| Collagen alpha-1(XXII) chain | Q8NFW1 | 9 | 14.0 | 22.0 | 11 | 22.5 | 35.2 | 7.90E-01 | 7 | 28.3 | 38.9 | 2.01E-01 | 2.76E-01 |
| Collagen alpha-1(XXIV) chain | Q17RW2 | 3 | 6.6 | 20.7 | 1 | 2.6 | 12.2 | 1.74E-01 | 0 | 0.0 | 0.0 | 2.01E-01 | 5.46E-01 |
| Collagen alpha-1(XXVII) | Q8IZC6 | 2 | 257.3 | 854.5 | 3 | 1.4 | 4.4 | 9.60E-01 | 1 | 0.5 | 1.4 | 9.15E-01 | 9.05E-01 |
| chain | | | | | | | | | | | | | |
| Collagen alpha-1(XXVIII) chain | Q2UY09 | 1 | 16.9 | 67.5 | 1 | 2.8 | 12.9 | 7.89E-01 | 0 | 0.0 | 0.0 | 4.80E-01 | 5.46E-01 |
| Collagen alpha-2(I) chain | P08123 | 6 | 5.1 | 8.1 | 11 | 12.0 | 14.6 | 2.23E-01 | 7 | 23.4 | 16.7 | 3.76E-03 | 9.63E-02 |
| Collagen alpha-2(IX) chain | Q14055 | 5 | 134.1 | 443.6 | 4 | 22.9 | 102.2 | 2.84E-01 | 2 | 1.5 | 3.4 | 5.94E-01 | 7.37E-01 |
| Collagen alpha-2(XI) chain | P13942 | 13 | 377.1 | 528.5 | 22 | 815.0 | 681.6 | 7.12E-03 | 8 | 535.3 | 670.1 | 1.59E-01 | 8.27E-02 |
| Collagen alpha-3(IX) chain | Q14050 | 11 | 155.8 | 156.3 | 13 | 71.5 | 116.4 | 1.33E-01 | 5 | 45.7 | 62.4 | 1.43E-01 | 9.81E-01 |
| Collagen alpha-4(IV) chain | P53420 | 2 | 2.0 | 5.6 | 1 | 1.8 | 8.3 | 4.11E-01 | 0 | 0.0 | 0.0 | 3.07E-01 | 5.46E-01 |
| Collagen alpha-5(IV) chain | P29400 | 16 | 264.0 | 353.1 | 18 | 251.6 | 272.7 | 9.06E-01 | 6 | 249.9 | 288.3 | 7.59E-01 | 8.51E-01 |
| Complement C3* | P01024 | 16 | 390.2 | 194.5 | 22 | 594.1 | 132.0 | 1.91E-03 | 8 | 550.0 | 144.3 | 6.62E-02 | 4.25E-01 |
| Complement C4-A | P0C0L4 | 7 | 29.3 | 78.3 | 3 | 1.1 | 2.8 | 2.82E-02 | 1 | 4.6 | 12.9 | 1.77E-01 | 9.68E-01 |
| Complement component 4B preproprotein | P0C0L5 | 13 | 18.2 | 23.0 | 18 | 16.4 | 17.6 | 8.36E-01 | 8 | 22.5 | 15.3 | 2.44E-01 | 2.05E-01 |
| Complement component C9 | P02748 | 13 | 245.2 | 194.7 | 21 | 296.3 | 182.9 | 6.25E-01 | 7 | 236.5 | 157.7 | 9.76E-01 | 3.60E-01 |
| Complement factor B | P00751 | 11 | 44.9 | 61.4 | 7 | 19.6 | 69.6 | 6.32E-03 | 6 | 14.1 | 24.0 | 2.15E-01 | 1.47E-01 |
| Cystatin-C | P01034 | 12 | 39.9 | 38.3 | 17 | 52.4 | 105.6 | 4.05E-01 | 8 | 37.3 | 23.9 | 8.06E-01 | 1.10E-01 |
| Dermcidin | P81605 | 8 | 79.1 | 178.6 | 7 | 76.9 | 179.9 | 4.21E-01 | 3 | 34.9 | 83.4 | 5.93E-01 | 8.89E-01 |
| Dickkopf-related protein 3 | Q9UBP4 | 14 | 129.9 | 142.7 | 19 | 93.0 | 111.3 | 3.00E-01 | 8 | 91.9 | 80.3 | 6.24E-01 | 7.43E-01 |
| Fibrinogen alpha chain | P02671 | 6 | 12.0 | 31.1 | 9 | 6.7 | 10.2 | 8.93E-01 | 7 | 15.2 | 12.1 | 3.93E-02 | 4.51E-02 |
| Fibrinogen beta chain | P02675 | 7 | 6.9 | 14.1 | 11 | 19.3 | 29.9 | 2.99E-01 | 7 | 49.4 | 51.3 | 5.70E-03 | 5.56E-02 |
| Fizzy-related protein homolog | Q9UM11 | 6 | 2.7 | 4.4 | 6 | 4.7 | 10.8 | 7.47E-01 | 6 | 8.7 | 6.4 | 2.20E-02 | 5.01E-02 |
| Gelsolin | P06396 | 12 | 29.5 | 66.1 | 11 | 26.0 | 49.9 | 5.62E-01 | 6 | 26.6 | 22.3 | 3.55E-01 | 2.61E-01 |
| Glutathione peroxidase 3 | P22352 | 12 | 78.7 | 174.6 | 16 | 86.5 | 87.0 | 3.10E-01 | 8 | 93.6 | 79.0 | 9.75E-02 | 4.80E-01 |
| Haptoglobin | P00738 | 10 | 30.6 | 51.3 | 16 | 39.4 | 94.5 | 6.31E-01 | 8 | 77.1 | 85.8 | 5.58E-02 | 9.01E-02 |
| Hemoglobin subunit beta | P68871 | 7 | 38.6 | 136.9 | 9 | 117.6 | 454.9 | 5.87E-01 | 5 | 32.2 | 38.0 | 2.02E-01 | 4.01E-01 |
| Hemopexin | P02790 | 15 | 159.3 | 92.7 | 22 | 127.4 | 59.4 | 1.47E-01 | 8 | 151.2 | 40.8 | 5.40E-01 | 2.05E-01 |
| Heparin cofactor 2 | P05546 | 4 | 1.3 | 3.0 | 2 | 1.3 | 5.0 | 2.26E-01 | 2 | 1.3 | 2.7 | 1.00E+00 | 3.02E-01 |
| Histidine-rich glycoprotein | P04196 | 4 | 6.9 | 17.7 | 4 | 10.6 | 31.0 | 7.40E-01 | 1 | 0.7 | 2.0 | 4.38E-01 | 6.13E-01 |
| Ig alpha-1 chain C region | P01876 | 5 | 16.0 | 41.0 | 10 | 53.6 | 100.2 | 2.41E-01 | 3 | 5.6 | 8.5 | 8.55E-01 | 3.78E-01 |
| Ig alpha-2 chain C region | P01877 | 11 | 84.2 | 88.9 | 17 | 143.7 | 165.4 | 3.03E-01 | 8 | 101.2 | 57.7 | 2.96E-01 | 8.88E-01 |
| Ig gamma-1 chain C region | P01857 | 16 | 725.9 | 462.5 | 22 | 785.6 | 409.9 | 5.15E-01 | 8 | 793.7 | 170.6 | 4.26E-01 | 1.00E+00 |
| Ig gamma-2 chain C region | P01859 | 14 | 65.0 | 94.3 | 14 | 39.3 | 47.1 | 3.71E-01 | 7 | 23.9 | 19.4 | 3.91E-01 | 9.62E-01 |
| Ig gamma-3 chain C region | P01860 | 8 | 47.2 | 94.0 | 8 | 40.0 | 86.1 | 5.10E-01 | 4 | 57.6 | 97.3 | 8.44E-01 | 4.59E-01 |
| Ig heavy chain V-III region GAL | P01781 | 7 | 40.3 | 65.3 | 5 | 6.2 | 18.8 | 8.85E-02 | 6 | 25.5 | 32.2 | 6.06E-01 | 1.07E-02 |
| Ig kappa chain C region | P01834 | 11 | 110.8 | 108.3 | 19 | 119.3 | 117.5 | 9.17E-01 | 8 | 98.7 | 120.6 | 8.54E-01 | 4.82E-01 |
| Ig kappa chain V-I region EU | P01598 | 3 | 3.1 | 7.4 | 3 | 8.5 | 34.3 | 6.92E-01 | 1 | 3.2 | 9.1 | 7.41E-01 | 9.68E-01 |
| Ig lambda-2 chain C regions | P0CG05 | 12 | 452.7 | 305.4 | 18 | 765.7 | 584.2 | 9.04E-02 | 7 | 584.8 | 305.6 | 2.43E-01 | 4.52E-01 |
| IgGFc-binding protein | Q9Y6R7 | 14 | 181.5 | 268.0 | 15 | 151.4 | 357.3 | 3.72E-01 | 5 | 23.1 | 23.0 | 6.50E-02 | 2.42E-01 |
| Ig lambda-like polypeptide 5* | B9A064 | 11 | 93.4 | 75.7 | 22 | 336.4 | 372.9 | 1.52E-04 | 8 | 418.1 | 562.9 | 2.24E-04 | 9.25E-01 |
| Inter-alpha (Globulin) inhibitor H2 | A2RTY6 | 6 | 12.5 | 26.5 | 12 | 16.8 | 22.3 | 3.06E-01 | 6 | 24.6 | 28.6 | 1.16E-01 | 3.32E-01 |
| Inter-alpha-trypsin inhibitor heavy chain H1 | P19827 | 13 | 92.5 | 79.6 | 21 | 110.0 | 138.2 | 9.41E-01 | 7 | 102.2 | 104.6 | 9.76E-01 | 9.07E-01 |
| Inter-alpha-trypsin inhibitor heavy chain H4 | Q14624 | 1 | 0.0 | 0.2 | 7 | 3.7 | 6.8 | 4.43E-02 | 1 | 5.6 | 15.7 | 5.66E-01 | 4.16E-01 |
| Kininogen-1 | P01042 | 7 | 18.4 | 47.8 | 7 | 4.9 | 9.1 | 4.22E-01 | 2 | 4.8 | 9.1 | 4.39E-01 | 8.17E-01 |
| Leucine-rich alpha-2-glycoprotein | P02750 | 7 | 25.8 | 47.6 | 5 | 30.4 | 100.2 | 2.59E-01 | 3 | 15.6 | 25.8 | 7.58E-01 | 5.67E-01 |
| Neuroblast differentiation-associated protein AHNAK | Q09666 | 9 | 14.0 | 24.8 | 3 | 1.1 | 4.1 | 3.10E-03 | 3 | 2.3 | 3.3 | 1.24E-01 | 1.49E-01 |
| Obscurin | Q5VST9 | 16 | 1767.1 | 3857.7 | 14 | 607.0 | 1582.5 | 2.05E-02 | 7 | 281.9 | 199.5 | 9.82E-02 | 6.69E-01 |
| Opticin* | Q9UBM4 | 16 | 163.7 | 95.1 | 16 | 92.3 | 240.1 | 1.50E-04 | 7 | 56.6 | 61.4 | 7.05E-03 | 6.04E-01 |
| Osteopontin | P10451 | 13 | 53.1 | 80.0 | 18 | 91.3 | 158.7 | 5.73E-01 | 7 | 44.0 | 68.2 | 7.82E-01 | 3.98E-01 |
| Pigment epithelium-derived factor | P36955 | 15 | 161.2 | 134.1 | 22 | 185.9 | 82.5 | 2.25E-01 | 8 | 185.3 | 57.6 | 2.21E-01 | 7.78E-01 |
| Plasminogen | P00747 | 7 | 12.8 | 18.6 | 6 | 3.4 | 7.3 | 1.52E-01 | 3 | 5.2 | 7.7 | 4.73E-01 | 4.52E-01 |
| Prostaglandin-H2 D-isomerase | P41222 | 16 | 4122.9 | 6744.6 | 22 | 2990.7 | 2929.5 | 5.74E-01 | 7 | 1487.0 | 1012.4 | 2.45E-01 | 1.11E-01 |
| Protein Jade-2 | Q9NQC1 | 2 | 0.2 | 0.7 | 0 | 0.0 | 0.0 | 9.28E-02 | 1 | 0.4 | 1.1 | 9.15E-01 | 9.73E-02 |
| Protein S100-A9 | P06702 | 5 | 44.6 | 155.8 | 2 | 0.2 | 0.7 | 9.66E-02 | 1 | 0.1 | 0.4 | 3.14E-01 | 8.57E-01 |
| Prothrombin | P00734 | 3 | 25.6 | 88.9 | 4 | 6.5 | 14.1 | 1.00E+00 | 4 | 37.7 | 54.8 | 1.09E-01 | 5.39E-02 |
| Retinol-binding protein 3 | P10745 | 16 | 605.1 | 1113.3 | 22 | 278.7 | 600.8 | 4.44E-02 | 8 | 188.8 | 69.6 | 4.62E-01 | 3.25E-01 |
| RNA-directed DNA polymerase (reverse transcriptase). related | | 12 | 56.7 | 48.5 | 18 | 155.9 | 157.1 | 1.90E-02 | 7 | 98.0 | 67.6 | 1.10E-01 | 4.52E-01 |
| domain containing protein Serine/cysteine proteinase inhibitor clade G member 1 splice variant 2 | Q5UGI6 | 12 | 18.0 | 30.6 | 11 | 13.6 | 19.7 | 3.76E-01 | 8 | 41.2 | 50.5 | 5.71E-02 | 3.05E-02 |
| Serotransferrin | P02787 | 16 | 323.7 | 181.1 | 22 | 264.5 | 88.7 | 6.36E-01 | 8 | 264.4 | 65.0 | 8.54E-01 | 8.51E-01 |
| SERPINA3 Alpha-1-antichymotrypsin | P01011 | 16 | 43.1 | 30.3 | 22 | 42.9 | 27.7 | 8.59E-01 | 8 | 50.3 | 25.0 | 3.91E-01 | 4.53E-01 |
| Serum albumin | P02768 | 16 | 2910.3 | 1163.1 | 22 | 2161.7 | 704.0 | 4.44E-02 | 8 | 1966.6 | 522.2 | 5.00E-02 | 3.73E-01 |
| Titin | O8WZ42 | 6 | 3.5 | 6.1 | 9 | 5.8 | 8.6 | 5.92E-01 | 7 | 6.8 | 6.3 | 9.41E-02 | 2.26E-01 |
| Translational activator GCN1 | Q92616 | 8 | 62.8 | 122.1 | 9 | 185.1 | 385.0 | 8.46E-01 | 5 | 184.2 | 191.9 | 1.76E-01 | 3.46E-01 |
| Transthyretin | P02766 | 14 | 517.6 | 479.2 | 19 | 242.4 | 252.7 | 1.04E-01 | 8 | 370.9 | 220.8 | 6.68E-01 | 1.11E-01 |
| Vitamin D-binding protein | P02774 | 11 | 25.3 | 32.1 | 14 | 14.6 | 24.3 | 5.07E-01 | 8 | 42.5 | 27.0 | 9.68E-02 | 4.50E-03 |
| Vitronectin* | P04004 | 8 | 8.8 | 11.0 | 17 | 85.1 | 147.7 | 3.11E-03 | 8 | 93.7 | 105.4 | 2.96E-04 | 3.01E-01 |
| Zinc-alpha-2-glycoprotein | P25311 | 7 | 12.5 | 24.5 | 12 | 36.8 | 62.6 | 2.97E-01 | 7 | 40.2 | 33.9 | 2.03E-02 | 1.36E-01 |

| | | | | | | | | | | | | | |
|---|---|---|---|---|---|---|---|---|---|---|---|---|---|
| ‡ Listed in the universal protein resource (UniProt), a central repository of protein data. # Number of samples with a signal intensity >0 § *P*-Value was analyzed by using the Mann-Whitney test A *P* of α<5.00E-02 was considered statistically significant. Significant results are highlighted in dark grey. * Proteins which remained significant after performing multiple hypotheses testing correction, analyzed by using the Benjamini-Hochberg test for false discovery rate. Significant proteins in vitreous humor detected by CE-MS and identified by LC-MS/MS when comparing protein signal intensity of RVO-samples (n=30) compared to control-samples (n=16: see Tab. 2). | | | | | | | | | | | | | |

### 2.4.6 Influence past Time Period since Suffering RVO On Biomarker Discovery

When analyzing the influence of past time period since suffering RVO, Clusterin, Ig lambda-like polypeptide 5 (IGLL5), Opticin and Vitronectin remained significantly different between control samples (n=16) *versus* fresh (n=22) or old RVO-samples (n=8) **(Table S5).** Except for Apolipoprotein A-II (*P*=1.75E-02) no difference was detected of the potential biomarkers when comparing signal intensity of the proteins between the fresh *versus* old RVO-samples (*P*=8.27E-02 to 9.68E-01).

### 2.6.7 Verification of Selected Potential Biomarker Candidates

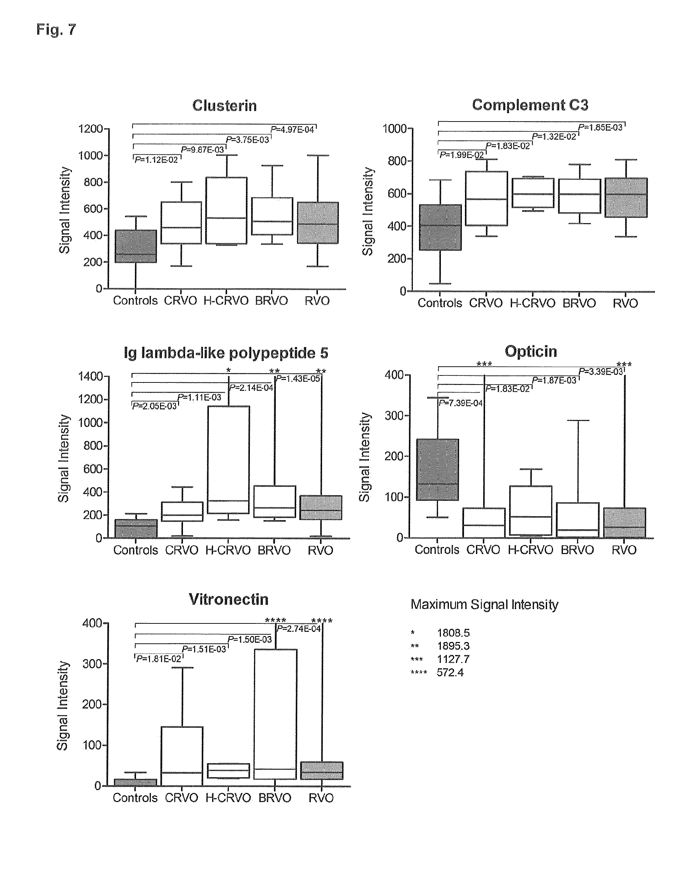

**Figure 7** shows the comparison of signal intensity of Clusterin, Complement C3, IGLL5, Opticin and Vitronectin in the control group (n=16) compared to the 3 subgroups in the discovery cohort (14 CRVO, 6 H-CRVO, 10 BRVO) and compared to the total discovery cohort (30 RVO).

All these proteins remained significant after using correction for multiple testing when comparing RVO versus controls **(Table** 5) and were listed significant in each comparison of the subgroup analysis **(Table 6).** Therefore, these proteins were considered for verification of potential biomarker candidates **(****Figure 5****).**

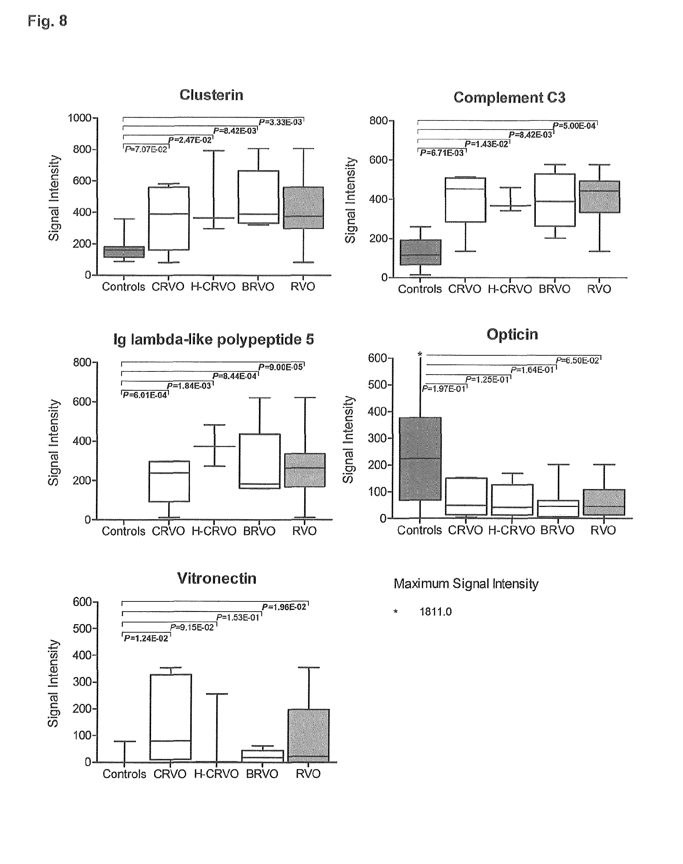

To determine validity of potential biomarker candidates in RVO, we performed receiver operating characteristic (ROC) analysis **(****Figure 8****)** using randomly selected, independent samples.

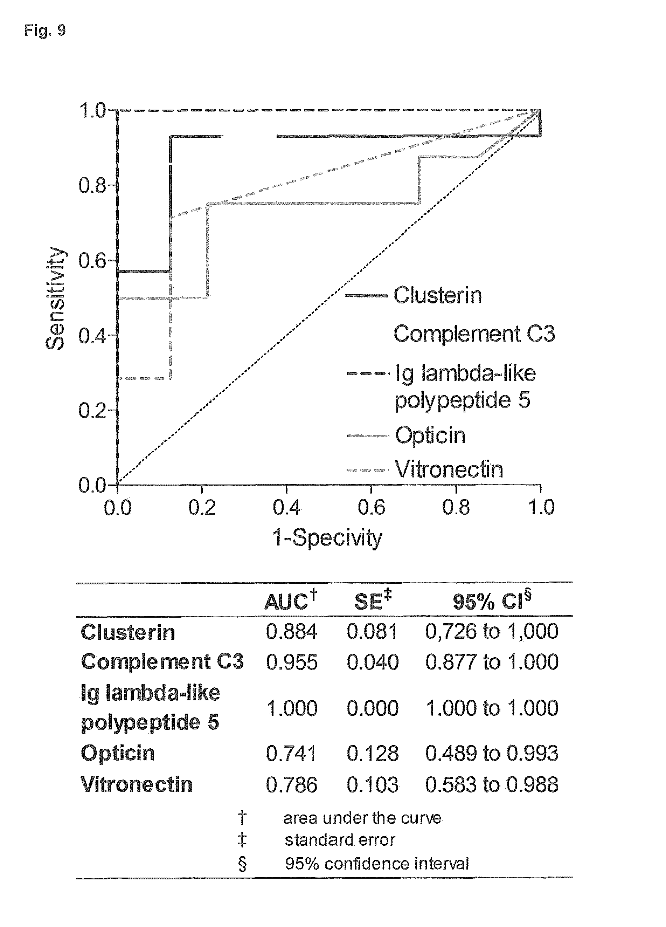

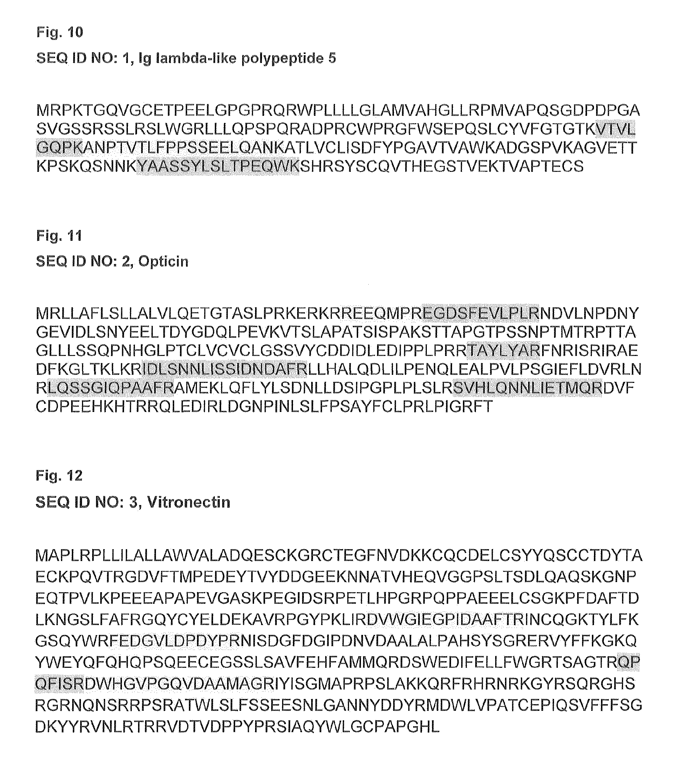

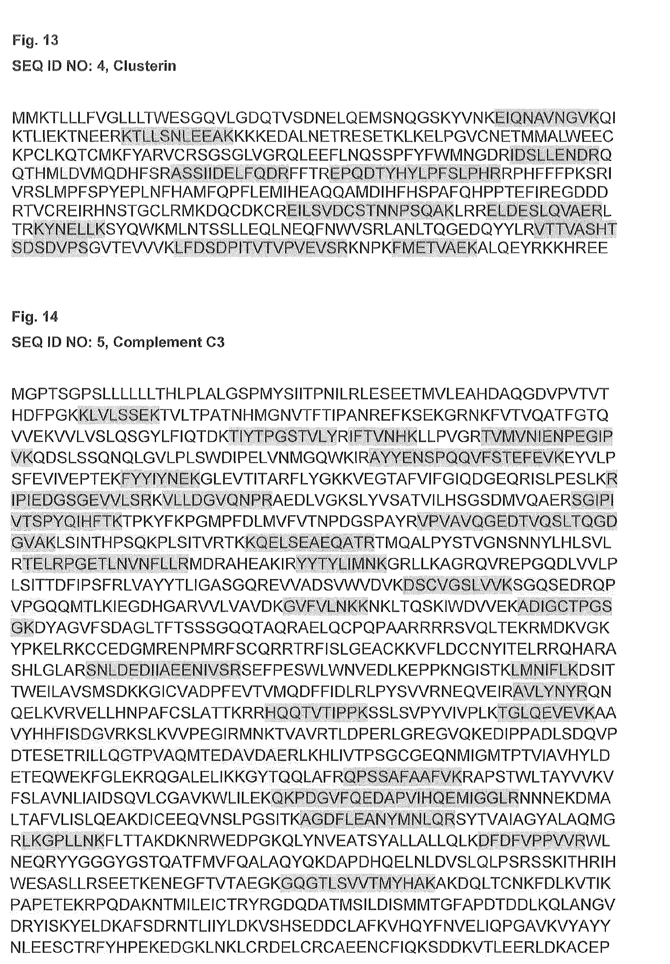

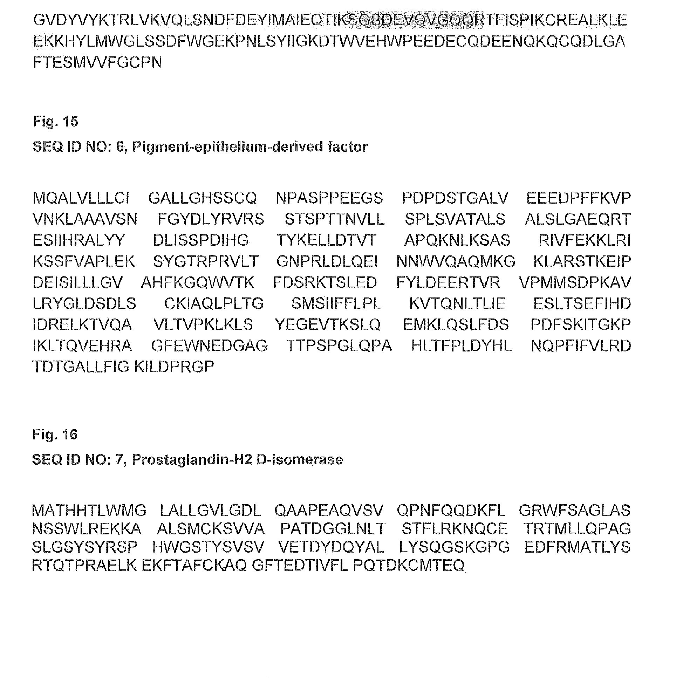

For analyses we used data of 8 controls and 14 RVO patients that were not included in analyses of biomarker definitions. AUCs were: Clusterin 0.884 (P=3.34E-03), Complement C3 0.955 (*P*=5.00E-04), IGLL5 1.000 (P=1.32E-04), Opticin 0.741 (P=6.54E-02), Vitronectin 0.786 (P=2.90E-02). **Figure 9** shows the comparison of signal intensity of the 5 proteins in the control group (n=8) compared to the 3 subgroups in the discovery cohort (6 CRVO, 3 H-CRVO, 5 BRVO) and compared to the total discovery cohort (14 RVO). Validation of the ROC analysis is listed in **Table 7.**

**Table 7. Validation of receiver operation characteristic (ROC) analysis. Means ± standard deviation of 50 analyses are listed. For each analysis 8 randomly selected samples of the 24 controls and 14 randomly selected samples of the 44 RVO-samples were used.**

| | **AUC^{†}** | **SE^{‡}** | **95% CI^{§}** |
|---|---|---|---|
| **Clusterin** | 0.870 ± 0.074 | 0.078 ± 0.028 | 0.717 ± 0.128 to 0.996 ±0.010 |
| **Complement C3** | 0.815 ± 0.086 | 0.097 ± 0.028 | 0.624 ± 0.138 to 0.985 ± 0.028 |
| **Ig lambda-like polypeptide 5** | 0.932 ± 0.037 | 0.052 ± 0.017 | 0.830 ± 0.070 to 1.000 ± 0.002 |
| **Opticin** | 0.862 ± 0.070 | 0.080 ± 0.026 | 0.705 ± 0.120 to 0.995 ± 0.012 |
| **Vitronectin** | 0.825 ± 0.064 | 0.089 ± 0.017 | 0.651 ± 0.096 to 0.987 ± 0.025 |
| | † area under the curve | | |
| | ‡ standard error | | |
| | § 95% confidence interval | | |

### 2.7 Discussion

The aim of this study was to analyze the protein profile of undiluted human vitreous of untreated patients with RVO and to identify potential biomarkers that are associated with the pathophysiology of the disease. CE-MS and LC-MS/MS were used for sample analysis in this study. CE-MS is a powerful and very reproducible technology platform with known performance characteristics and therefore is used as one of the most advanced techniques for the discovery of new protein biomarkers of clinical significance. Furthermore, CE-MS allows the characterization of highly complex samples in a consistent and reproducible way and has a high reproducibility allowing the comparison of the protein content of samples over time. LC-MS/MS was used in this study to provide sequence information (i.e. identify) of the CE-MS detected peptides.

### 2.7.1 Potential Biomarker Candidates

Comparing the proteome of 30 RVO patients and 16 controls, we could define 17 proteins that were significantly up- or downregulated in RVO. Thrombosis and thrombolysis are involved in RVO as well as inflammatory processes due to hypoxic induced cell death. This could be confirmed in our study as inflammatory response pathway, complement activation (classical pathway) as well as complement and coagulation cascades were identified as primary pathways of the 17 significant up- or downregulated proteins in VH of RVO patients compared to controls.

We believe, that five of the 17 significant proteins, Clusterin, Complement C3, IGLL5, Opticin and Vitronectin, are of special interest as they remained significant after multiple testing, analysis of fresh *versus* old RVO, and subgroup analysis (CRVO, H-CRVO, BRVO versus controls).

Clusterin is a glycoprotein with a nearly ubiquitous tissue distribution and an apparent involvement in a number of biological processes, including *inter alia* lipid transport, membrane recycling, cell adhesion, programmed cell death, and complement cascade. Interestingly, increased blood levels of Clusterin are associated with atrophy of the entorhinal cortex in Alzheimer's disease for which this protein seems to be a marker of disease severity. Therefore, upregulated Clusterin levels may indicate biochemical signs of a neurodegenerative disease. Our findings indicate for the first time that Clusterin in VH is associated with retinal disease like RVO.

Complement C3 plays a central role in the activation of complement system. Among other things, it is involved in the adaptive immune response to select the appropriate antigens for a humoral response, promotes phagocytosis and supports local inflammatory responses against pathogens. Complement C3 might thus be involved in the well-known phagocytic and inflammatory process subsequent to the onset of RVO.

Similar to Complement C3, IGLL5 demonstrated upregulation in RVO. IGLL5 could also be involved in immune processes being associated with cell death due to RVO although IGLL5 seems not to be expressed in pre-B-cells (http:// uniprot.org). In general little is known about IGLL5 in the literature and further research is needed to explain the association of high IGLL5 levels in VH of patients with RVO.

Opticin is a protein produced by the non-pigmented ciliary epithelium. It is present in significant quantities in the vitreous of the eye and also localizes at the cornea, the iris, the ciliary body, the optic nerve, the choroid and the retina. It can noncovalently bind collagen fibrils and regulate fibril morphology, spacing, and organization. Furthermore, Opticin was found to be located, besides various ocular tissues, especially in basal and cortical vitreous and adjacent basement membranes, like the internal limiting membrane, suggesting a possible role in vitreoretinal adhesion. Therefore, decreased Opticin levels may be correlated with macular edema due to RVO.

Vitronectin is an abundant glycoprotein which promotes cell adhesion and spreading, inhibits the membrane damaging effect of the terminal cytolytic complement pathway, and binds to several serpins. By its localization in the extracellular matrix and its binding to plasminogen activation inhibitor-1, vitronectin can potentially regulate the proteolytic degradation of this matrix. In addition, vitronectin binds to complement, to heparin and to thrombin-antithrombin III complexes, showing its participation in the immune response and in the regulation of clot formation. Therefore, upregulation of Vitronectin in RVO potentially plays a role in the disease.

### 2.7.2 Advantages of the Study

To our knowledge, this is the first proteomics study using a high number (n=30 and 16 idiopathic vitreous floaters as controls) of undiluted VH of RVO patients and validation of the 5 most significant proteins in an independent data set of an additional 8 controls and 14 RVO patients. We compared our results with other high throughput studies on VH. We have identified 10 novel VH proteins (see Table S4).

### 2.8 Conclusions

In this retrospective, clinical-experimental study we applied a reproducible proteomics detection method in a high number of undiluted vitreous humor of patients with RVO. Our findings were thoroughly statistically evaluated and yielded five potential biomarker candidates associated with the pathophysiology of RVO: Clusterin, Complement C3, IGLL5, Opticin and Vitronectin. Future studies are needed to validate our substantial findings, which might be helpful regarding future diagnostic or therapeutic approaches in RVO. These studies should deplete highly abundant VH proteins such as albumin and immunoglobulin to determine more precisely potential biomarker candidates associated with the pathophysiology of RVO.

### Description of the figures

**Figure 1****:**
   Study design
**Figure 2****: Signal intensities of potential biomarker candidates**
   Comparison of signal intensity of proteins being significant in analyses of choroidal neovascularization without bleeding (CNVw/oB), choroidal neovascularization with bleeding (CNVwB), and hemorrhagic choroidal neovascularization (hCNV) versus controls (see Table 2). Intensity serves as assessment for relative abundance and is shown for all nAMD samples (Group A), for CNVw/oB (G1), CNVwB (G2), hCNV (G3) and controls (C). Wilcoxon-Mann-Whitney test as part of closed test principle was used for analysis.
**Figure 3****: Gene Ontology analysis**
   Gene Ontology (GO) analysis of significant proteins in comparison of nAMD and control samples (n=13, see Table 3). Analysis was performed using WebGestalt (WEB-based GEne SeT AnaLysis Toolkit; http://bioinfo.vanderbilt.edu/webgestalt/).
**Figure 4****. Validation of potential biomarker candidates via ROC analysis**
   ROC curves of clusterin, opticin, pigment epithelium-derived factor (PEDF) and prostaglandin-H2 D-iosmerase (PH2D) regarding protein intensity of independent samples not used for proteomic analysis and biomarker definitions. aAUC: Area under the curve, bSE: Standard error, c95% CI: 95% confidence interval for AUC.
**Figure 5****.**
   Study layout
**Figure 6****.**
   Gene Ontology (GO) analysis of significant vitreous humor proteins when comparing retinal vein occlusion (RVO) and control samples (n=19; see Table 5) as determined by WebGestalt (http://bioinfo.vanderbilt.edu).
**Figure 7****.**
   Comparison of signal intensity of proteins remained significant after using correction for multiple testing (Benjamini-Hochberg test) when comparing retinal vein occlusion (RVO) versus controls (see *; Table 5) of samples used for biomarker candidates identification (discovery set). Mann-Whitney test was used for analysis. A P of α<5.00E-02 was considered statistically significant. RVO was subdivided in following subgroups: Central-RVO (CRVO), hemi-central RVO (H-CRVO), branch RVO (BRVO).
**Figure 8****.**
   Receiver operating characteristic (ROC) curves of selected candidate proteins using independent samples of 14 patients suffered a retinal vein occlusion (RVO) and 8 controls. The area under the curve (AUC) was shown for RVO at 95% confidence level (95% CI).
**Figure 9****.**
   Comparison of signal intensity of proteins used for biomarker validation. Mann-Whitney test was used for analysis. A P of α<5.00E-02 was considered statistically significant. Significant values are written in bold. Retinal vein occlusion (RVO) was subdivided in following subgroups: Central-RVO (CRVO), hemi-central RVO (H-CRVO), branch RVO (BRVO).
**Figure 10****.**
   SEQ ID NO: 1, Ig lambda-like polypeptide 5
**Figure 11****.**
   SEQ ID NO: 2, Opticin
**Figure 12****.**
   SEQ ID NO: 3, Vitronectin
**Figure 13****.**
   SEQ ID NO: 4, Clusterin
**Figure 14****.**
   SEQ ID NO: 5, Complement C3
**Figure 15****.**
   SEQ ID NO: 6, Pigment-epithelium-derived factor
**Figure 16****.**
   SEQ ID NO: 7, Prostaglandin-H2 D-isomerase

## Claims

1. Method for analyzing and/or diagnosing an eye-related disease,
the method comprising determining in vitro the amount of one or more marker(s) selected from the group consisting of the polypeptides Immunoglobulin lambda-like polypeptide 5, Opticin, Vitronectin, Clusterin, Complement C3, pigment-epithelium-derived factor, and prostaglandin-H2 D-isomerase, or a variant thereof, and determining whether or not a respective eye-related disease on the basis of the amount of the marker(s) is present, and wherein the respective variant exhibits % identity of 90% or higher to the amino acid sequence of the respective wildtype polypeptide.

2. Method for analyzing and/or diagnosing an eye-related disease according to claim 1,
wherein the eye-related disease is selected from the group consisting of retino-choroidal diseases, retinal vascular diseases, diabetic retinopathy, retinal detachment, and macular edemas.

3. Method for analyzing and/or diagnosing an eye-related disease according to claim 1,
wherein the eye-related disease is selected from the group consisting of diabetes mellitus, rheumatism, vascular diseases, cardiac diseases, stroke, anemia, and leukemia.

4. Method for analyzing and/or diagnosing according to any of claims 1 to 3, wherein the one or more marker(s) or variant thereof is (are) present in a test sample derived from a test subject.

5. Method according to any of the preceding claims, wherein
Immunoglobulin lambda-like polypeptide 5 is a polypeptide comprising, preferably consisting of, the sequence shown in SEQ ID NO: 1;
Opticin is a polypeptide comprising, preferably consisting of, the sequence shown in SEQ ID NO: 2;
Vitronectin is a polypeptide comprising, preferably consisting of, the sequence shown in SEQ ID NO: 3;
Clusterin is a polypeptide comprising, preferably consisting of, the sequence shown in SEQ ID NO: 4;
Complement C3 is a polypeptide comprising, preferably consisting of, the sequence shown in SEQ ID NO: 5;
Pigment-epithelium-derived factor is a polypeptide comprising, preferably consisting of, the sequence shown in SEQ ID NO: 6; and
Prostaglandin-H2 D-isomerase is a polypeptide comprising, preferably consisting of, the sequence shown in SEQ ID NO: 7.

6. The method according to any of claims 1 to 5, wherein when the amount of the respective marker for analyzing and/or diagnosing the respective disease selected from the group as defined in any of claims 1 to 3 in a test subject is statistically significantly different from that of a normal individual, the test subject is determined to have the respective disease, preferably a P-Value of α<5.00E-02, being analyzed by using the Mann-Whitney test, is considered statistically significant.

7. The method according to any of claims 1 to 6, wherein the test subject is determined to have any of the disease defined in any of claims 1 to 3, if the amount of Immunoglobulin lambda-like polypeptide 5 or a variant thereof is significantly higher; the amount of Opticin or a variant thereof is significantly lower; the amount of Vitronectin or a variant thereof is significantly higher; the amount of Clusterin or a variant thereof is significantly higher; the amount of Complement C3 or a variant thereof is significantly higher; the amount of pigment-epithelium-derived factor or a variant thereof is significantly higher; and/or the amount of prostaglandin-H2 D-isomerase or a variant thereof is significantly higher; respectively compared to the amount of the respective wildtype polypeptide.

8. The method according to any of the preceding claims, wherein the amount of one or more of the marker(s) selected from the group consisting of the polypeptides Immunoglobulin lambda-like polypeptide 5, Opticin, Vitronectin, Clusterin, and Complement C3, or a respective variant thereof, is determined in vitro for determining whether or not the test subject has retinal vascular diseases, preferably RVO.

9. The method according to any of the preceding claims 1 to 7 wherein the amount of one or more of the marker(s) selected from the group consisting of the polypeptides Opticin, Clusterin, pigment-epithelium-derived factor, and prostaglandin-H2 D-isomerase, or a respective variant thereof, is determined in vitro for determining whether or not a test subject has diabetic retinopathy comprising diabetic macular edema, retinal thrombosis including macular edema, further macular edemas comprising inflammatory and postoperative macular edemas, and/or retino-choroidal diseases comprising age-related macular degeneration (AMD), in particular neovascular AMD (nAMD).

10. Use of one or more marker polypeptide(s) selected from the group consisting of the polypeptides Immunoglobulin lambda-like polypeptide 5, Opticin, Vitronectin, Clusterin, Complement C3, pigment-epithelium-derived factor, and prostaglandin-H2 D-isomerase, or a variant thereof, for analyzing and/or diagnosing eye-related diseases, wherein the respective variant exhibits % identity of 90% or higher to the amino acid sequence of the respective wildtype polypeptide.

11. Use of a kit for analyzing, diagnosing, and/or treating eye-related diseases selected from the group consisting of retino-choroidal diseases, retinal vascular diseases, diabetic retinopathy, retinal detachment, and macular edemas, wherein the kit comprises one or more antibodie(s) that is (are) specifically directed against one or more marker(s) selected from the group of polypeptide(s) or variant(s) thereof as defined in claim 1 or 2.

12. A DNA chip for analyzing and/or diagnosing diseases selected from the group consisting of retino-choroidal diseases, retinal vascular diseases, diabetic retinopathy, retinal detachment, and macular edemas, comprising one or more nucleic acid sequences selected from the group of nucleotide sequences that respectively encode the polypeptide(s) as defined in claim 1 or 2.

13. The use of a kit according to claim 11, or the DNA chip according to claim 12, in a method as defined in any of claims 1 to 9.

14. In vitro method for identifying a test compound that is capable of increasing or decreasing the amount of one or more of the polypeptide(s) as defined in claim 1 in a test sample, comprising the steps of
(a) carrying out a method as defined in any of claims 1 to 9 by using a test sample, and based on the result determining whether an eye-related disease is present;
(b) if it is determined that an eye-related disease is present, contacting the test compound with the same or a corresponding test sample;
(c) determining whether the test compound has increased or decreased the amount of the polypeptide(s).
